# EUROPÄISCHE PATENTANMELDUNG

(11) **EP 2 295 422 A2**
(43) Veröffentlichungstag der Anmeldung: **16.03.2011**
(21) Anmeldenummer: 10183906.6
(22) Anmeldetag: 11.03.2005
(51) Int. Cl.: C07D 309/10, C07H 15/04, A61K 31/351

(54) **Glucopyranosylsubstituierte Benzolderivate, diese Verbindungen enthaltende Arzneimittel, deren Verwendung und Verfahren zu ihrer Herstellung**

(30) Priorität: 16.03.2004 DE 102004012676; 18.08.2004 DE 102004040168; 16.12.2004 DE 102004061145; 09.02.2005 EP 05002628
(62) Teilanmeldung aus: 05715979.0
(71) Anmelder: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Himmelsbach, Frank, 88441, MITTELBIBERACH (DE); Eckhardt, Matthias, 88400, BIBERACH (DE); Eickelmann, Peter, 88441, MITTELBIBERACH (DE); Barsoumian, Edward Leon, TOYONAKA Osaka 560-0005 (JP); Thomas, Leo, 88400, BIBERACH (DE)
(74) Vertreter: Hammann, Heinz

(57) **Zusammenfassung**

Glucopyranosyl-substituierte Benzol-Derivate der allgemeinen Formel I 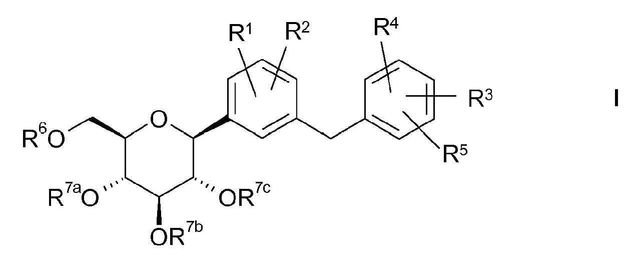 wobei die Reste R¹ bis R⁶ sowie R^{7a}, R^{7b}, R^{7c} gemäß Anspruch 1 definiert sind, einschließlich deren Tautomere, deren Stereoisomere, deren Gemische und deren Salze. Die erfindungsgemäße Verbindungen sind geeignet zur Behandlung von Stoffwechselerkrankungen.

## Beschreibung

Gegenstand der vorliegenden Erfindung sind Glucopyranosyl-substituierte Benzol-Derivate der allgemeinen Formel I 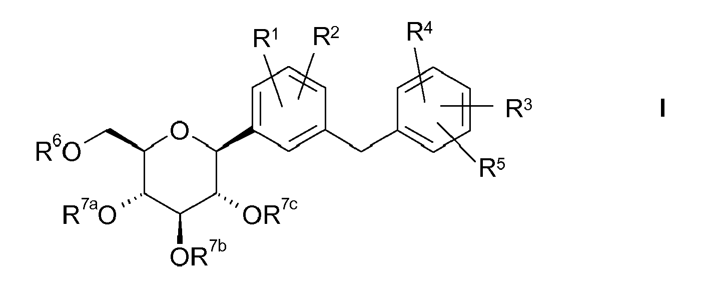 wobei die Reste R¹ bis R⁶ sowie R^{7a}, R^{7b}, R^{7c} nachfolgend definiert sind, einschließlich deren Tautomere, deren Stereoisomere, deren Gemische und deren Salze. Ein weiterer Gegenstand dieser Erfindung betrifft Arzneimittel enthaltend eine erfindungsgemäße Verbindung der Formel I sowie die Verwendung einer erfindungsgemäßen Verbindung zur Herstellung eines Arzneimittels zur Behandlung von Stoffwechselerkrankungen. Darüber hinaus sind Verfahren zur Herstellung eines Arzneimittels sowie einer erfindungsgemäßen Verbindung Gegenstand dieser Erfindung.

In der Literatur werden Verbindungen, die eine Hemmwirkung auf den natriumabhängigen Glucose-Cotransporter SGLT2 besitzen, zur Behandlung von Krankheiten, insbesondere von Diabetes vorgeschlagen.

Aus den internationalen Offenlegungsschriften WO 98/31697, WO 01/27128, WO 02/083066, WO 03/099836, WO 2004/063209, WO 2004/080990, WO 2004/013118, WO 2004/052902, WO 2004/052903 und der US-Anmeldung US 2003/0114390 sind Glucopyranosyl-substituierte Aromaten sowie deren Herstellung und deren mögliche Aktivität als SGLT2-Inhibitoren bekannt.

### Aufgabe der Erfindung

Der vorliegenden Erfindung liegt die Aufgabe zugrunde, neue Pyranosyl-substituierte Benzol-Derivate aufzuzeigen, insbesondere solche, die eine Aktivität bezüglich des natriumabhängigen Glucose-Cotransporters SGLT, insbesondere SGLT2 besitzen. Eine weitere Aufgabe der vorliegenden Erfindung besteht im Aufzeigen von Pyranosylsubstituierten Benzol-Derivate, die *in vitro* und/oder *in vivo* im Vergleich mit bekannten, strukturähnlichen Verbindungen eine erhöhte Hemmwirkung bezüglich des natriumabhängigen Glucose-Cotransporters SGLT2 besitzen und/oder verbesserte pharmakologische oder pharmakokinetische Eigenschaften aufweisen.

Ferner ist es eine Aufgabe der vorliegenden Erfindung, neue Arzneimittel bereit zu stellen, welche zur Prophylaxe und/oder Behandlung von Stoffwechselerkrankungen, insbesondere von Diabetes geeignet sind.

Ebenfalls eine Aufgabe dieser Erfindung ist es, ein Verfahren zur Herstellung der erfindungsgemäßen Verbindungen bereit zu stellen.

Weitere Aufgaben der vorliegenden Erfindung ergeben sich für den Fachmann unmittelbar aus den vorhergehenden und nachfolgenden Ausführungen.

### Gegenstand der Erfindung

Ein erster Gegenstand der vorliegenden Erfindung sind Glucopyranosyl-substituierte Benzol-Derivate der allgemeinen Formel I 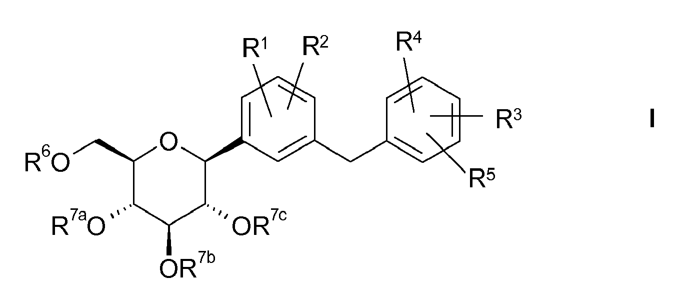 in der
- R¹: ausgewählt ist aus den Bedeutungen der Gruppe A und
falls R³ ausgewählt ist aus den Bedeutungen der Gruppe B, kann R¹ zusätzlich auch ausgewählt sein aus den Bedeutungen Wasserstoff, Fluor, Chlor, Brom, Iod, C₁₋₄- Alkyl, C₂₋₄-Alkenyl-C₁₋₄-alkyl, C₂₋₄-Alkinyl-C₁₋₄-alkyl, C₂₋₄-Alkenyl-C₁₋₄-alkoxy, C₂₋₄-Alkinyl-C₁₋₄-alkoxy, C₃₋₇-Cycloalkyl-C₁₋₄-alkyl, C₅₋₇-Cycloalkenyl-C₁₋₄-alkyl, eine durch 1 bis 3 Fluoratome substituierte Methylgruppe, eine durch 1 bis 5 Fluoratome substituierte Ethylgruppe, C₁₋₄-Alkoxy, eine durch 1 bis 3 Fluoratome substituierte Methoxygruppe, eine durch 1 bis 5 Fluoratome substituierte Ethoxygruppe, eine durch eine Hydroxy- oder C₁₋₃-Alkoxygruppe substituierte C₁₋₄-Alkylgruppe, eine durch eine Hydroxy- oder C₁₋₃-Alkoxygruppe substituierte C₂₋₄-Alkoxygruppe, C₃₋₆-Cycloalkyl-C₁₋₃-alkoxy oder Hydroxy,
wobei in den vorstehend genannten Cycloalkyl- und Cycloalkenyl-Ringen ein oder zwei Methylengruppen unabhängig voneinander durch O oder CO ersetzt sein können, und
- R²: Wasserstoff, Fluor, Chlor, Brom, Hydroxy, C₁₋₄-Alkyl, C₁₋₄-Alkoxy, Cyan oder Nitro, wobei die Alkyl- oder Alkoxygruppe ein oder mehrfach mit Fluor substituiert sein kann, und
- R³: ausgewählt ist aus den Bedeutungen der Gruppe B und
falls R¹ ausgewählt ist aus den Bedeutungen der Gruppe A, kann R³ zusätzlich auch ausgewählt sein aus den Bedeutungen Wasserstoff, Fluor, Chlor, Brom, Iod, C₁₋₆-Alkyl, C₂₋₄-Alkenyl-C₁₋₄-alkyl, C₂₋₄-Alkinyl-C₁₋₄-alkyl, C₂₋₄-Alkenyl-C₁₋₄-alkoxy, C₂₋₄-Alkinyl-C₁₋₄-alkoxy, C₃₋₇-Cycloalkyl, C₅₋₇-Cycloalkenyl, C₃₋₇-Cycloalkyl-C₁₋₄-alkyl, C₅₋₇-Cycloalkenyl-C₁₋₄-alkyl, C₃₋₆-Cycloalkylidenmethyl, Hydroxy, C₁₋₆-Alkoxy, C₃₋₆-Cycloalkyl-C₁₋₃-alkoxy, Aryl, Aryl-C₁₋₃-alkyl, Heteroaryl, Heteroaryl-C₁₋₃-alkyl, Aryloxy, Aryl-C₁₋₃-alkyl-oxy, eine durch 1 bis 3 Fluoratome substituierte Methyl- oder Methoxygruppe, eine durch 1 bis 5 Fluoratome substituierte C₂₋₄-Alkyl- oder C₂₋₄-Alkoxygruppe, eine durch eine Cyangruppe substituierte C₁₋₄-Alkylgruppe, eine durch eine Hydroxy- oder C₁₋₃-Alkyloxygruppe substituierte C₁₋₄-Alkylgruppe, Cyan, Carboxy, C₁₋₃-Alkoxycarbonyl, Aminocarbonyl, (C₁₋₃-Alkylamino)carbonyl, Di-(C₁₋₃-alkyl)aminocarbonyl, Pyrrolidin-1-ylcarbonyl, Piperidin-1-ylcarbonyl, Morpholin-4-ylcarbonyl, Piperazin-1-yl-carbonyl, 4-(C₁₋₃-Alkyl)-piperazin-1-ylcarbonyl, (C₁₋₄-Alkyl)carbonylamino-, C₁₋₄-Alkylsulfonylamino, C₁₋₄-Alkylsulfanyl-, C₁₋₄-Alkylsulfinyl-, C₁₋₄-Alkylsulfonyl-, Arylsulfonylamino, Aryl-C₁₋₃-alkylsulfonylamino oder Arylsulfonyl,
- R⁴, R⁵: unabhängig voneinander Wasserstoff, Fluor, Chlor, Brom, Iod, Cyan, Nitro, C₁-₃-Alkyl, C₁₋₃-Alkoxy, durch 1 bis 3 Fluoratome substituiertes Methyl- oder Methoxy,
- A: C₂₋₆-Alkin-1-yl, C₂₋₆-Alken-1-yl, C₃₋₇-Cycloalkyl, C₅₋₇-Cycloalkenyl, Aryl, Heteroaryl, C₁₋₄-Alkylcarbonyl, Arylcarbonyl, Heteroarylcarbonyl, Aminocarbonyl, C₁₋₄-Alkyl-aminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Arylaminocarbonyl, Heteroarylaminocarbonyl, C₁₋₄-Alkoxycarbonyl, Aryl-C₁₋₃-alkoxycarbonyl, Heteroaryl-C₁₋₃-alkoxycarbonyl, Amino, C₁₋₄-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, C₁₋₄-Alkylcarbonylamino, Arylcarbonylamino, Heteroarylcarbonylamino, C₃₋₇-Cycloalkyl-oxy, C₅₋₇-Cycloalkenyloxy, Aryloxy, Heteroaryloxy, C₁₋₄-Alkylsulfinyl, C₁₋₄-Alkyl-sulfonyl, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Aryl-sulfanyl, Arylsulfinyl, Arylsulfonyl, Heteroarylsulfanyl, Heteroarylsulfinyl, Heteroarylsulfonyl, Cyan oder Nitro bedeutet,
wobei die vorstehend genannten Alkinyl- und Alkenylgruppen ein- oder mehrfach mit Fluor oder Chlor substituiert sein können, und
wobei die vorstehend genannten Alkinyl- und Alkenylgruppen ein- oder zweifach mit gleichen oder verschiedenen Resten L1 substituiert sein können, und
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
- B: Tri-(C₁₋₄-alkyl)silyl-C₁₋₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Arylcarbonylamino, Heteroarylcarbonylamino, Nitro, C₃₋₁₀-Cycloalkyloxy, C₅₋₁₀-Cycloalkenyloxy, C₃₋₁₀-Cycloalkylsulfanyl, C₃₋₁₀-Cycloalkylsulfinyl, C₃₋₁₀-Cycloalkyl-sulfonyl, C₅₋₁₀-Cycloalkenylsulfanyl, C₅₋₁₀-Cycloalkenylsulfinyl, C₅₋₁₀-Cycloalkenyl-sulfonyl, Arylsulfanyl, Arylsulfinyl, Heteroarylsulfanyl oder Heteroarylsulfinyl,
wobei die vorstehend genannten Alkinyl- und Alkenylgruppen ein- oder mehrfach mit Fluor oder Chlor substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
- R^{N}: H, C₁₋₄-Alkyl, C₁₋₄-Alkylcarbonyl oder C₁₋₄-Alkylsulfonyl,
- L1: unabhängig voneinander ausgewählt aus der Gruppe bestehend aus Hydroxy, Cyan-, Nitro-, C₃₋₇-Cycloalkyl, Aryl-, Heteroaryl-, C₁₋₄-Alkylcarbonyl-, Arylcarbonyl-, Heteroarylcarbonyl-, Aminocarbonyl-, C₁₋₄-Alkylaminocarbonyl-, Di-(C₁₋₃-alkyl)-aminocarbonyl-, Pyrrolidin-1-ylcarbonyl-, Piperidin-1-ylcarbonyl-, Morpholin-4-ylcarbonyl, Arylaminocarbonyl-, Heteroarylaminocarbonyl-, C₁₋₄-Alkoxycarbonyl-, Aryl-C₁₋₃-alkoxycarbonyl-, Heteroaryl-C₁₋₃-alkoxycarbonyl-, C₁₋₄-Alkyloxy-, Aryloxy-, Heteroaryloxy-, C₁₋₄-Alkylsulfanyl-, Arylsulfanyl-, Heteroarylsulfanyl-, C₁₋₄-Alkyl-sulfinyl-, Arylsulfinyl-, Heteroarylsulfinyl-, C₁₋₄-Alkylsulfonyl-, Arylsulfonyl- und Heteroarylsulfonyl-; und
- L2: unabhängig voneinander ausgewählt aus der Gruppe bestehend aus Fluor, Chlor, Brom, Iod, C₁₋₃-Alkyl, Difluormethyl, Trifluormethyl, C₁₋₃-Alkoxy, Difluormethoxy, Trifluormethoxy und Cyan; und
- R⁶, R^{7a}, R^{7b}, R^{7c}: unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl besitzen,

wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl-, Isochinolinyl- oder Tetrazolylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L2 substituiert sein können;
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Die erfindungsgemäßen Verbindungen der allgemeinen Formel I und ihre physiologisch verträglichen Salze weisen wertvolle pharmakologische Eigenschaften auf, insbesondere eine Hemmwirkung auf den natriumabhängigen Glucose-Cotransporter SGLT, insbesondere SGLT2. Ferner können erfindunsgemäße Verbindungen eine Hemmwirkung auf den natriumabhängigen Glucose-Cotransporter SGLT1 aufweisen. Verglichen mit einer möglichen Hemmwirkung auf SGLT1 hemmen die erfindungsgemäßen Verbindungen vorzugsweise selektiv SGLT2.

Gegenstand der vorliegenden Erfindung sind auch die physiologisch verträglichen Salze der erfindungsgemäßen Verbindungen mit anorganischen oder organischen Säuren.

Ein weiterer Gegenstand dieser Erfindung sind Arzneimittel, enthaltend mindestens eine erfindungsgemäße Verbindung oder ein erfindungsgemäßes physiologisch verträgliches Salz neben gegebenenfalls einem oder mehreren inerten Trägerstoffen und/oder Verdünnungsmitteln.

Ebenfalls ein Gegenstand dieser Erfindung ist die Verwendung mindestens einer erfindungsgemäßen Verbindung oder eines physiologisch verträglichen Salzes solch einer Verbindung zur Herstellung eines Arzneimittels, das zur Behandlung oder Prophylaxe von Erkrankungen oder Zuständen geeignet ist, die durch Inhibierung des natriumabhängigen Glucose-Cotransporters SGLT, insbesondere SGLT2 beeinflussbar sind.

Ein weiterer Gegenstand dieser Erfindung ist die Verwendung mindestens einer erfindungsgemäßen Verbindung oder eines seiner physiologisch verträglichen Salze zur Herstellung eines Arzneimittels, das zur Behandlung von Stoffwechselerkrankungen geeignet ist

Ein weiterer Gegenstand dieser Erfindung ist die Verwendung mindestens einer erfindungsgemäßen Verbindung oder eines seiner physiologisch verträglichen Salze zur Herstellung eines Arzneimittels zur Inhibition des natriumabhängigen Glucose-Cotransporters SGLT, insbesondere SGLT2.

Ferner ist ein Verfahren zur Herstellung eines erfindungsgemäßen Arzneimittels Gegenstand dieser Erfindung, **dadurch gekennzeichnet, dass** auf nicht-chemischem Wege eine erfindungsgemäße Verbindung oder eines seiner physiologisch verträglichen Salze in einen oder mehrere inerte Trägerstoffe und/oder Verdünnungsmittel eingearbeitet wird.

Gegenstand der vorliegenden Erfindung ist auch ein Verfahren zur Herstellung der erfindungsgemäßen Verbindungen der allgemeinen Formel I, **dadurch gekennzeichnet, dass**
a) zur Herstellung von Verbindungen der allgemeinen Formel I, die wie vor- und nachstehend definiert ist,
   eine Verbindung der allgemeinen Formel II 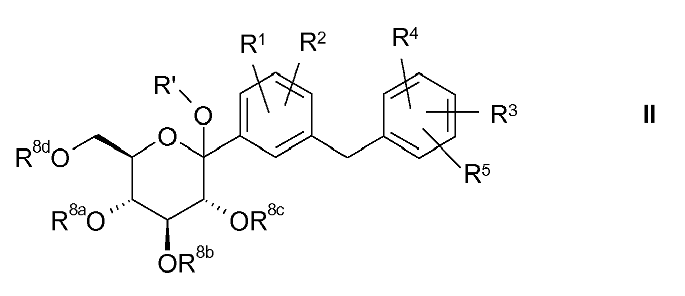 in der
   - R': H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
   - R^{8a}, R^{8b}, R^{8c}, R^{8d}: unabhängig voneinander eine zuvor und nachstehend für die Reste R⁶, R^{7a}, R^{7b}, R^{7c} angegebenen Bedeutungen aufweisen, eine Benzyl-Gruppe oder eine R^{a}R^{b}R^{c}Si-Gruppe oder eine Ketal- oder Acetalgruppe, insbesondere eine Alkyliden- oder Arylalkyliden- Ketal- oder Acetalgruppe bedeuten, wobei jeweils zwei benachbarte Reste R^{8a}, R^{8b}, R^{8c}, R^{8d} eine cyclische Ketal- oder Acetalgruppe oder eine 1,2-Di(C₁₋₃-aikoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring, insbesondere einen 2,3-Dimethyl-2,3-di(C₁₋₃-alkoxy)-1,4-dioxan-Ring, bildet, und wobei Alkyl-, Aryl-und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl, worin die Aryl- oder Alkylgruppen ein- oder mehrfach mit Halogen substituiert sein können;
   wobei unter den bei der Definition der vorstehend genannten Reste erwähnten Arylgruppen Phenyl- oder Naphthylgruppen, vorzugsweise Phenylgruppen zu verstehen sind;
   und in der die Reste R¹ bis R⁵ und R⁶, R^{7a}, R^{7b}, R^{7c} wie vor- und nachstehend definiert sind;
   mit einem Reduktionsmittel in Gegenwart einer Lewis- oder Brønsted-Säure umgesetzt wird, wobei die eventuell vorhandenen Schutzgruppen gleichzeitig oder nachträglich abgespalten werden; oder
b) zur Herstellung von Verbindungen der allgemeinen Formel I, in der R⁶, R^{7a}, R^{7b} und R^{7c} Wasserstoff bedeuten,
   eine Verbindung der allgemeinen Formel III 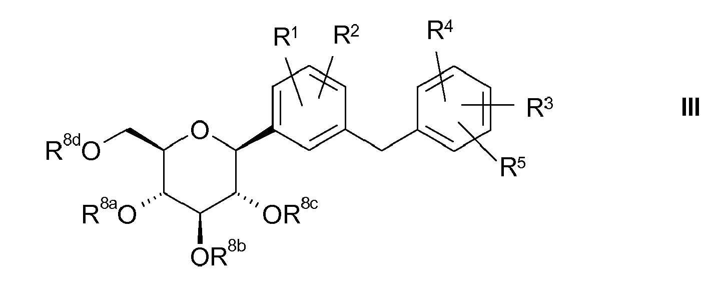 in der R^{8a}, R^{8b}, R^{8c}, R^{8d} sowie R¹ bis R⁵ wie zuvor und nachstehend definiert sind, jedoch mindestens einer der Reste R^{8a}, R^{8b}, R^{8c}, R^{8d} nicht Wasserstoff bedeutet, hydrolysiert wird, und
   gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder
   erforderlichenfalls ein bei den vorstehend beschriebenen Umsetzungen verwendeter Schutzrest wieder abgespalten wird und/oder
   gewünschtenfalls eine so erhaltene Verbindung der allgemeinen Formel I in ihre Stereoisomere aufgetrennt wird und/oder
   gewünschtenfalls eine so erhaltene Verbindung der allgemeinen Formel I in ihre Salze, insbesondere für die pharmazeutische Anwendung in ihre physiologisch verträglichen Salze, überführt wird.

Ein weiterer Gegenstand dieser Erfindung ist ein Verfahren zur Herstellung von Verbindungen der allgemeinen Formel II 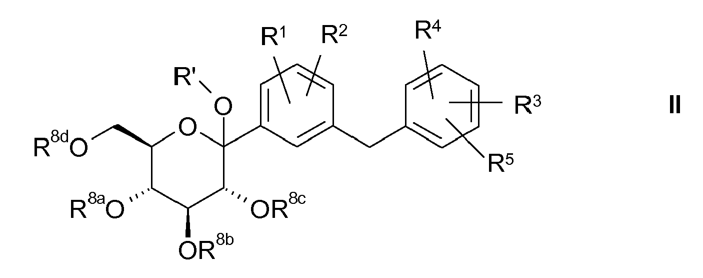 in der
- R': H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
- R^{8a}, R^{8b}, R^{8c}, R^{8d}: unabhängig voneinander eine für die Reste R⁶, R^{7a}, R^{7b}, R^{7c} angegebenen Bedeutungen besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal- oder Acetalgruppe oder mit zwei Sauerstoffatomen des Pyranose-Rings einen substituierten 2,3-Oxydioxan-Ring, insbesondere einen 2,3-Dimethyl-2,3-di(C₁₋₃-alkoxy)-1,4-dioxan-Ring, bilden können, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können;und
- R^{a}, R^{b}, R^{c}: unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵, R⁶, R^{7a}, R^{7b}, R^{7c} die zuvor und nachstehend angegebenen Bedeutungen besitzen,
bei dem eine Organometall-Verbindung (V), die durch Halogen-Metall-Austausch oder durch Insertion eines Metalls in die Kohlenstoff-Halogen-Bindung einer Halogen-Benzylbenzol-Verbindung der allgemeinen Formel IV 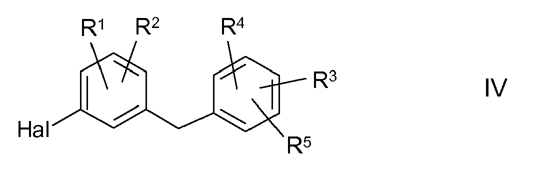 in der Hal Cl, Br und I bedeuten und R¹ bis R⁵ wie zuvor und nachstehend definiert sind, und gegebenenfalls anschließender Ummetallierung erhältlich ist, an ein Gluconolacton der allgemeinen Formel VI 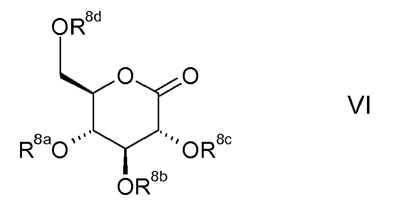 in der R^{8a}, R^{8b}, R^{8c}, R^{8d} wie zuvor und nachstehend definiert sind,
addiert wird, und

anschließend das erhaltene Addukt, vorzugsweise in situ, mit Wasser oder einem Alkohol R'-OH, wobei R' gegebenenfalls substituiertes C₁₋₄-Alkyl bedeutet, in Gegenwart einer Säure, wie beispielsweise Methansulfonsäure, Schwefelsäure, Salzsäure, Essigsäure oder Ammoniumchlorid, umgesetzt wird und optional das in der Umsetzung mit Wasser erhaltene Produkt, in dem R' H bedeutet, in einer nachfolgenden Reaktion mit einem Acylierungsmittel, wie beispielsweise des entsprechenden Säurechlorids oder -anhydrids, in das Produkt der Formel II überführt wird, worin R' (C₁₋₁₈-Alkyl)carbonyl, (C₁₋₁₈-Alkyl)oxycarbonyl, Arylcarbonyl oder Aryl-(C₁₋₃-alkyl)-carbonyl, das wie angegeben substituiert sein kann, bedeutet.

Die aufgezeigten Zwischenprodukte, insbesondere der Formel IV, der Formel II sowie der Formel III, stellen ebenfalls Gegenstände dieser Erfindung dar.

Im folgenden sind Gegenstände der Erfindung aufgelistet und nummeriert:
1. Glucopyranosyl-substituierte Benzol-Derivate der allgemeinen Formel I 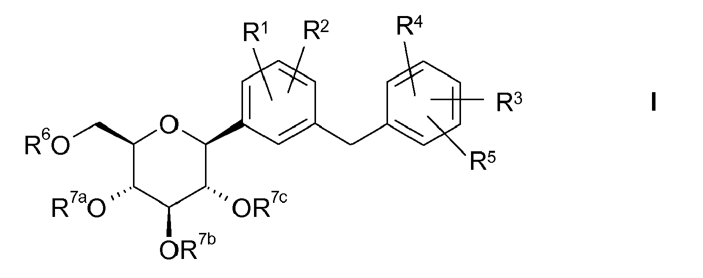 in der
   - R¹: ausgewählt ist aus den Bedeutungen der Gruppe A und
   falls R³ ausgewählt ist aus den Bedeutungen der Gruppe B, kann R¹ zusätzlich auch ausgewählt sein aus den Bedeutungen Wasserstoff, Fluor, Chlor, Brom, Iod, C₁₋₄-Alkyl, C₂₋₄-Alkenyl-C₁₋₄-alkyl, C₂₋₄-Alkinyl-C₁₋₄-alkyl, C₂₋₄-Alkenyl-C₁₋₄-alkoxy, C₂₋₄-Alkinyl-C₁₋₄-alkoxy, C₃₋₇-Cycloalkyl-C₁₋₄-alkyl, C₅₋₇-Cycloalkenyl-C₁₋₄-alkyl, eine durch 1 bis 3 Fluoratome substituierte Methylgruppe, eine durch 1 bis 5 Fluoratome substituierte Ethylgruppe, C₁₋₄-Alkoxy, eine durch 1 bis 3 Fluoratome substituierte Methoxygruppe, eine durch 1 bis 5 Fluoratome substituierte Ethoxygruppe, eine durch eine Hydroxy- oder C₁₋₃-Alkoxygruppe substituierte C₁₋₄-Alkylgruppe, eine durch eine Hydroxy- oder C₁₋₃-Alkoxygruppe substituierte C₂₋₄-Alkoxygruppe, C₃₋₆-Cycloalkyl-C₁-₃-alkoxy oder Hydroxy,
   wobei in den vorstehend genannten Cycloalkyl- und Cycloalkenyl-Ringen ein oder zwei Methylengruppen unabhängig voneinander durch O oder CO ersetzt sein können, und
   - R²: Wasserstoff, Fluor, Chlor, Brom, Hydroxy, C₁₋₄-Alkyl, C₁₋₄-Alkoxy, Cyan oder Nitro, wobei die Alkyl- oder Alkoxygruppe ein oder mehrfach mit Fluor substituiert sein kann, und

   - R³: ausgewählt ist aus den Bedeutungen der Gruppe B und
   falls R¹ ausgewählt ist aus den Bedeutungen der Gruppe A, kann R³ zusätzlich auch ausgewählt sein aus den Bedeutungen Wasserstoff, Fluor, Chlor, Brom, Iod, C₁₋₆-Alkyl, C₂₋₄-Alkenyl-C₁₋₄-alkyl, C₂₋₄-Alkinyl-C₁₋₄-alkyl, C₂₋₄-Alkenyl-C₁₋₄-alkoxy, C₂₋₄-Alkinyl-C₁₋₄-alkoxy, C₃₋₇-Cycloalkyl, C₅₋₇-Cycloalkenyl, C₃₋₇-Cycloalkyl-C₁₋₄-alkyl, C₅₋₇-Cycloalkenyl-C₁₋₄-alkyl, C₃₋₆-Cycloalkyliden-methyl, Hydroxy, C₁₋₆-Alkoxy, C₃₋₆-Cycloalkyl-C₁₋₃-alkoxy, Aryl, Aryl-C₁₋₃-alkyl, Heteroaryl, Heteroaryl-C₁₋₃-alkyl, Aryloxy, Aryl-C₁₋₃-alkyl-oxy, eine durch 1 bis 3 Fluoratome substituierte Methyl- oder Methoxygruppe, eine durch 1 bis 5 Fluoratome substituierte C₂₋₄-Alkyl- oder C₂₋₄-Alkoxygruppe, eine durch eine Cyangruppe substituierte C₁₋₄-Alkylgruppe, eine durch eine Hydroxy- oder C₁₋₃-Alkyloxygruppe substituierte C₁₋₄-Alkylgruppe, Cyan, Carboxy, C₁₋₃-Alkoxy-carbonyl, Aminocarbonyl, (C₁₋₃-Alkylamino)carbonyl, Di-(C₁₋₃-alkyl)amino-carbonyl, Pyrrolidin-1-ylcarbonyl, Piperidin-1-ylcarbonyl, Morpholin-4-ylcarbonyl, Piperazin-1-yl-carbonyl, 4-(C₁₋₃-Alkyl)-piperazin-1-ylcarbonyl, (C₁₋₄-Alkyl)carbonylamino-, C₁₋₄-Alkylsulfonylamino, C₁₋₄-Alkylsulfanyl-, C₁₋₄-Alkylsulfinyl-, C₁₋₄-Alkylsulfonyl-, Arylsulfonylamino, Aryl-C₁₋₃-alkylsulfonyl-amino oder Arylsulfonyl,
   - R⁴, R⁵: unabhängig voneinander Wasserstoff, Fluor, Chlor, Brom, Iod, Cyan, Nitro, C₁₋₃-Alkyl, C₁₋₃-Alkoxy, durch 1 bis 3 Fluoratome substituiertes Methyl- oder Methoxy,
   - A: C₂₋₆-Alkin-1-yl, C₂₋₆-Alken-1-yl, C₃₋₇-Cycloalkyl, C₅₋₇-Cycloalkenyl, Aryl, Heteroaryl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Arylamino-carbonyl, Heteroarylaminocarbonyl, C₁₋₄-Alkoxycarbonyl, Aryl-C₁₋₃-alkoxycarbonyl, Heteroaryl-C₁₋₃-alkoxycarbonyl, Amino, C₁₋₄-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, C₁₋₄-Alkylcarbonylamino, Arylcarbonylamino, Heteroaryl-carbonylamino, C₃₋₇-Cycloalkyloxy, C₅₋₇-Cycloalkenyloxy, Aryloxy, Heteroaryloxy, C₁₋₄-Alkylsulfinyl, C₁₋₄-Alkylsulfonyl, C₃₋₇-Cycloaikylsuifanyl, C₃₋₇-Cycloalkylsuifinyl, C₃₋₇-Cycloalkylsulfonyl, C₅₋₇-Cycloalkenylsulfanyl, C₅₋₇- ₇-Cycloalkenylsulfinyl, C₅₋₇-Cycloalkenylsulfonyl, Arylsulfanyl, Arylsulfinyl, Arylsulfonyl, Heteroarylsulfanyl, Heteroarylsulfinyl, Heteroarylsulfonyl, Cyan oder Nitro bedeutet,
   wobei die vorstehend genannten Alkinyl- und Alkenylgruppen ein- oder mehrfach mit Fluor oder Chlor substituiert sein können, und
   wobei die vorstehend genannten Alkinyl- und Alkenylgruppen ein- oder zweifach mit gleichen oder verschiedenen Resten L1 substituiert sein können, und
   wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
   wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
   - B: Tri-(C₁₋₄-alkyl)silyl-C₁₋₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Arylcarbonylamino, Heteroarylcarbonylamino, Nitro, C₃₋₁₀-Cycloalkyloxy, C₅₋₁₀-Cycloalkenyloxy, C₃₋₁₀-Cycloalkylsulfanyl, C₃₋₁₀Cyclo-alkylsulfinyl, C₃₋₁₀-Cycloalkylsulfonyl, C₅₋₁₀-Cycloalkenylsulfanyl, C₅₋₁₀-Cyclo-alkenylsulfinyl, C₅₋₁₀-Cycloalkenylsulfonyl, Arylsulfanyl, Arylsulfinyl, Heteroarylsulfanyl oder Heteroarylsulfinyl,
   wobei die vorstehend genannten Alkinyl- und Alkenylgruppen ein- oder mehrfach mit Fluor oder Chlor substituiert sein können, und
   wobei die vorstehend genannten Alkinyl- und Alkenylgruppen ein- oder zweifach mit gleichen oder verschiedenen Resten L1 substituiert sein können;
   wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
   wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
   - R^{N}: H, C₁₋₄-Alkyl, C₁₋₄-Alkylcarbonyl oder C₁₋₄-Alkylsulfonyl,
   - L1: unabhängig voneinander ausgewählt aus der Gruppe bestehend aus Hydroxy-, Cyan-, Nitro-, C₃₋₇-Cycloalkyl, Aryl-, Heteroaryl-, C₁₋₄-Alkylcarbonyl-, Arylcarbonyl-, Heteroarylcarbonyl-, Aminocarbonyl-, C₁₋₄-Alkylaminocarbonyl-, Di-(C₁-₃-alkyl)aminocarbonyl-, Pyrrolidin-1-ylcarbonyl-, Piperidin-1-ylcarbonyl-, Morpholin-4-ylcarbonyl, Arylaminocarbonyl-, Heteroarylaminocarbonyl-, C₁₋₄-Alkoxycarbonyl-, Aryl-C₁₋₃-alkoxycarbonyl-, Heteroaryl-C₁₋₃-alkoxycarbonyl-, C₁₋₄-Alkyloxy-, Aryloxy-, Heteroaryloxy-, C₁₋₄-Alkylsulfanyl-, Arylsulfanyl-, Heteroarylsulfanyl-, C₁₋₄-Alkylsulfinyl-, Arylsulfinyl-, Heteroarylsulfinyl-, C₁₋₄-Alkylsulfonyl-, Arylsulfonyl- und Heteroarylsulfonyl-; und
   - L2: unabhängig voneinander ausgewählt aus der Gruppe bestehend aus Fluor, Chlor, Brom, Iod, C₁₋₃-Alkyl, Difluormethyl, Trifluormethyl, C₁₋₃-Alkoxy, Difluormethoxy, Trifluormethoxy und Cyan; und
   - R⁶, R^{7a}, R^{7b}, R^{7c}: unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl besitzen,
   wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
   unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl-, Isochinolinyl- oder Tetrazolylgruppe zu verstehen ist,
   oder eine Pyrrolyl-, Furanyl-, Thienyl- oder Pyridylgruppe zu verstehen ist, in der eine oder zwei Methingruppen durch Stickstoffatome ersetzt sind,
   oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
   wobei die vorstehend erwähnten Heteroarylgruppen unabhängig voneinander ein- oder zweifach mit gleichen oder verschiedenen Resten L2 substituiert sein können;
   wobei, soweit nichts anderes erwähnt wurde, die vorstehend erwähnten Alkylgruppen geradkettig oder verzweigt sein können,
   deren Tautomere, deren Stereoisomere, deren Gemische und deren Salze.
2. Glucopyranosyl-substituierte Benzol-Derivate der allgemeinen Formel 1.2 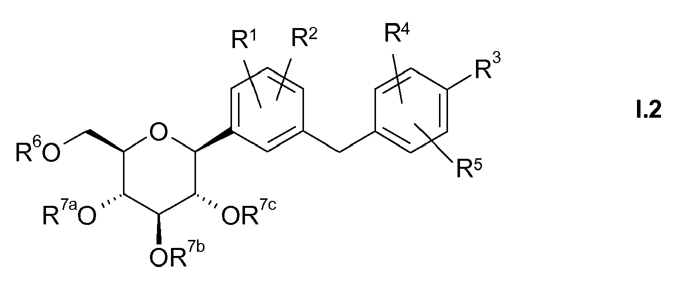 worin die Reste R¹ bis R⁶ und R^{7a}, R^{7b} und R^{7c} wie in Gegenstand 1 definiert sind.
3. Glucopyranosyl-substituierte Benzol-Derivate gemäß Gegenstand 1 oder 2, **dadurch gekennzeichnet, dass** die Gruppe A C₂₋₆-Alkin-1-yl, C₂₋₆-Alken-1-yl, C₃₋₇Cycloalkyl, C₅₋₇-Cycloalkenyl, C₁₋₄-Alkylcarbonyl, Aminocarbonyl, C₁₋₄-Alkylaminocarbonyl, Di-(C₁-₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, C₁₋₄-Alkoxycarbonyl, Amino, C₁₋₄-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, C₁₋₄-Alkylcarbonylamino, C₃₋₇-Cycloalkyloxy, C₅₋₇Cycloalkenyloxy, C₁₋₄-Alkylsulfinyl, C₁₋₄-Alkylsulfonyl, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Cyan oder Nitro bedeutet,
   wobei die vorstehend genannten Alkinyl- und Alkenylgruppen ein- oder mehrfach mit Fluor oder Chlor substituiert sein können, und
   wobei die vorstehend genannten Alkinyl- und Alkenylgruppen ein- oder zweifach mit gleichen oder verschiedenen Resten L1 substituiert sein können, und
   wobei die vorstehend genannten Cycloalkyl- und Cycloalkenyl-Ringe unabhängig voneinander ein- oder zweifach mit Substituenten ausgewählt aus Fluor und C₁-₃-Alkyl substituiert sein können, und
   wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können, und
   L1 und R^{N} wie in Gegenstand 1 definiert sind.
4. Glucopyranosyl-substituierte Benzol-Derivate gemäß einem oder mehreren der Gegenstände 1 bis 3, **dadurch gekennzeichnet, dass** die Gruppe B Tri-(C₁₋₄-alkyl)silyl-C₁₋₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Nitro, C₃₋₇-Cycloalkyloxy, C₅₋₇-Cycloalkenyloxy, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl oder C₅₋₇-Cycloalkenylsulfonyl bedeutet,
   wobei die vorstehend genannten Alkinyl- und Alkenylgruppen ein- oder mehrfach mit Fluor oder Chlor substituiert sein können, und
   wobei die vorstehend genannten Alkinyl- und Alkenylgruppen ein- oder zweifach mit gleichen oder verschiedenen Resten L1 substituiert sein können;
   wobei die vorstehend genannten Cycloalkyl- und Cycloalkenyl-Ringe unabhängig voneinander ein- oder zweifach mit Substituenten ausgewählt aus Fluor und C₁-₃-Alkyl substituiert sein können, und
   wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können, und
   L1 und R^{N} wie in Gegenstand 1 definiert sind.
5. Glucopyranosyl-substituierte Benzol-Derivate gemäß einem oder mehreren der Gegenstände 1 bis 4, **dadurch gekennzeichnet, dass** der Rest R³ ausgewählt ist aus den Bedeutungen der Gruppe B gemäß Gegenstand 1 oder 4.
6. Glucopyranosyl-substituierte Benzol-Derivate gemäß einem oder mehreren der Gegenstände 1 bis 5, **dadurch gekennzeichnet, dass** der Rest R¹ ausgewählt ist aus der Gruppe bestehend aus Wasserstoff, Fluor, Chlor, Brom, Iod, C₁₋₄-Alkyl, C₂₋₆-Alkinyl, C₁₋₄-Alkoxy, C₂₋₄-Alkenyl-C₁₋₄-alkoxy, C₂₋₄-Alkinyl-C₁₋₄-alkoxy, durch 1 bis 3 Fluoratome substituiertes Methyl, durch 1 bis 5 Fluoratome substituiertes Ethyl, durch 1 bis 3 Fluoratome substituiertes Methoxy, durch 1 bis 5 Fluoratome substituiertes Ethoxy, durch eine Hydroxy- oder C₁₋₃-Alkoxygruppe substituiertes C₁₋₄-Alkyl, durch eine Hydroxy- oder C₁₋₃-Alkoxygruppe substituiertes C₂₋₄-Alkoxy, C₂₋₆-Alkenyl, C₃₋₆-Cycloalkyl, C₃₋₆-Cycloalkyl-C₁₋₃-alkyl, C₃₋₇-Cycloalkyloxy, C₃₋₆-Cycloalkyl-C₁₋₃-alkoxy, C₅₋₇-Cycloalkenyloxy, Hydroxy, Amino, Nitro oder Cyan, wobei in den C₅₋₆-Cycloalkylgruppen eine Methylengruppe durch O ersetzt sein kann.
7. Glucopyranosyl-substituierte Benzol-Derivate gemäß einem oder mehreren der Gegenstände 1 bis 5, **dadurch gekennzeichnet, dass** der Rest R¹ ausgewählt ist aus den Bedeutungen der Gruppe A gemäß Gegenstand 1 oder 3.
8. Glucopyranosyl-substituierte Benzol-Derivate gemäß einem oder mehreren der Gegenstände 1 bis 4 und 7, **dadurch gekennzeichnet, dass** der Rest R³ ausgewählt ist aus der Gruppe bestehend aus Wasserstoff, Fluor, Chlor, Brom, Hydroxy, Cyan, C₁₋₆-Alkyl, Trimethylsilylethyl, C₂₋₆-Alkenyl, C₂₋₆-Alkinyl, Difluormethyl, Trifluormethyl, C₃₋₇-Cycloalkyl, C₅₋₇-Cycloalkenyl, C₁₋₆-Alkyloxy, Difluormethoxy, Trifluormethoxy, Pentafluorethoxy, C₃₋₇-Cycloalkyloxy, Tetrahydrofuranyloxy, Tetrahydrofuranonyloxy, C₁₋₆-Alkylsulfanyl, Cyclopropylidenmethyl-, Aryl und Heteroaryl.
9. Glucopyranosyl-substituierte Benzol-Derivate gemäß einem oder mehreren der Gegenstände 1 bis 8, **dadurch gekennzeichnet, dass** der Rest R² Wasserstoff, Fluor, Chlor, Brom, Methyl, Hydroxy, Methoxy, Ethoxy, Trifluormethoxy, Cyan, Nitro oder durch 1 bis 3 Fluoratome substituiertes Methyl bedeutet.
10. Glucopyranosyl-substituierte Benzol-Derivate gemäß einem oder mehreren der Gegenstände 1 bis 9, **dadurch gekennzeichnet, dass** die Reste R⁴ und/oder R⁵ unabhängig voneinander Wasserstoff oder Fluor bedeuten.
11. Glucopyranosyl-substituierte Benzol-Derivate gemäß einem oder mehreren der Gegenstände 1 bis 10, **dadurch gekennzeichnet, dass** der Rest R⁶ Wasserstoff, (C₁₋₈-Alkyl)oxycarbonyl-, C₁₋₈-Alkylcarbonyl- oder Benzoyl, vorzugsweise Wasserstoff bedeutet.
12. Glucopyranosyl-substituierte Benzol-Derivate gemäß einem oder mehreren der Gegenstände 1 bis 11, **dadurch gekennzeichnet, dass** die Reste R^{7a}, R^{7b}, R^{7c} Wasserstoff bedeuten.
13. Physiologisch verträgliche Salze der Verbindungen nach mindestens einem der Gegenstände 1 bis 12 mit anorganischen oder organischen Säuren.
14. Arzneimittel, enthaltend eine Verbindung nach mindestens einem der Gegenstände 1 bis 12 oder ein physiologisch verträgliches Salz gemäß Gegenstand 13 neben gegebenenfalls einem oder mehreren inerten Trägerstoffen und/oder Verdünnungsmitteln.
15. Verwendung mindestens einer Verbindung nach einem oder mehreren der Gegenstände 1 bis 12 oder eines physiologisch verträglichen Salzes gemäß Gegenstand 13 zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind.
16. Verwendung mindestens einer Verbindung nach mindestens einem der Gegenstände 1 bis 12 oder eines physiologisch verträglichen Salzes gemäß Gegenstand 13 zur Herstellung eines Arzneimittels, das zur Behandlung oder Vorbeugung von Stoffwechselerkrankungen geeignet ist.
17. Verwendung nach Gegenstand 16, **dadurch gekennzeichnet, dass** die Stoffwechserkrankung ausgewählt ist aus der Gruppe bestehend aus Diabetes mellitus Typ 1 und Typ 2, diabetische Komplikationen, metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie.
18. Verwendung mindestens einer Verbindung nach mindestens einem der Gegenstände 1 bis 12 oder eines physiologisch verträglichen Salzes gemäß Gegenstand 13 zur Herstellung eines Arzneimittels zur Inhibition des natriumabhängigen Glucose-Cotransporter SGLT2.
19. Verwendung mindestens einer Verbindung nach mindestens einem der Gegenstände 1 bis 12 oder eines physiologisch verträglichen Salzes gemäß Gegenstand 13 zur Herstellung eines Arzneimittels zum Verhindern der Degeneration von pankreatischen beta-Zellen und/oder zum Verbessern und/oder Wiederherstellen der Funktionalität von pankreatischen beta-Zellen.
20. Verwendung mindestens einer Verbindung nach mindestens einem der Gegenstände 1 bis 12 oder eines physiologisch verträglichen Salzes gemäß Gegenstand 13 zur Herstellung von Diuretika und/oder Antihypertensiva.
21. Verfahren zur Herstellung eines Arzneimittels gemäß Gegenstand 14, **dadurch gekennzeichnet, dass** auf nicht-chemischem Wege eine Verbindung nach mindestens einem der Gegenstände 1 bis 12 oder ein physiologisch verträgliches Salz gemäß Gegenstand 13 in einen oder mehrere inerte Trägerstoffe und/oder Verdünnungsmittel eingearbeitet wird.
22. Verfahren zur Herstellung der Verbindungen der allgemeinen Formel I gemäß den Gegenständen 1 bis 12, **dadurch gekennzeichnet, dass** eine Verbindung der allgemeinen Formel II 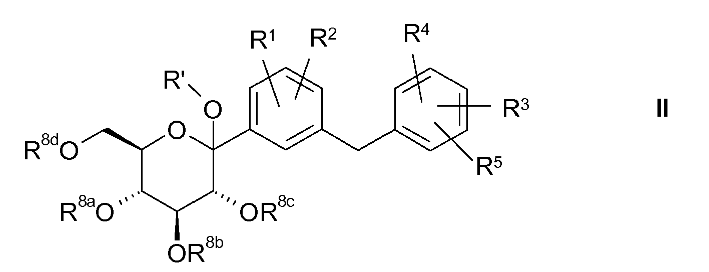 in der
   - R': H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
   - R^{8a}, R^{8b}, R^{8c}, R^{8d}: unabhängig voneinander eine für die Reste R⁶ , R^{7a}, R^{7b}, R^{7c} angegebenen Bedeutungen besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal-oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
   wobei unter den bei der Definition der vorstehend genannten Reste erwähnten Arylgruppen Phenyl- oder Naphthylgruppen, vorzugsweise Phenylgruppen zu verstehen sind;
   und R¹ bis R⁵, R⁶, R^{7a}, R^{7b}, R^{7c} die in den Gegenständen 1 bis 12 angegebenen Bedeutungen besitzen,
   mit einem Reduktionsmittel in Gegenwart einer Lewis- oder Brøsted-Säure umgesetzt wird, wobei die eventuell vorhandenen Schutzgruppen gleichzeitig oder nachträglich abgespalten werden;
   gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder
   erforderlichenfalls ein bei den vorstehend beschriebenen Umsetzungen verwendeter Schutzrest wieder abgespalten wird und/oder
   gewünschtenfalls eine so erhaltene Verbindung der allgemeinen Formel I in ihre Stereoisomere aufgetrennt wird und/oder
   gewünschtenfalls eine so erhaltene Verbindung der allgemeinen Formel I in ihre physiologisch verträglichen Salze überführt wird.
23. Verfahren gemäß Gegenstand 22, **dadurch gekennzeichnet, dass** die Verbindung der allgemeinen Formel II gemäß dem in Gegenstand 24 oder 25 beschriebenen Verfahren erhalten wird.
24. Verfahren zur Herstellung von Verbindungen der allgemeinen Formel II 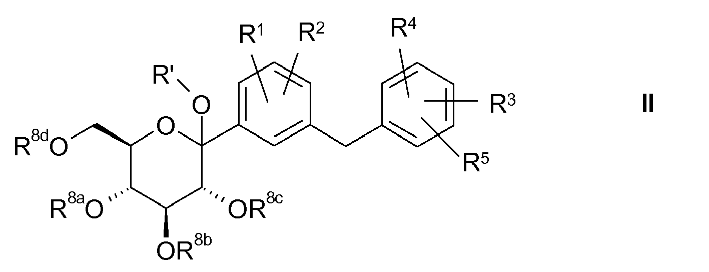 in der
   - R': H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
   - R^{8a}, R^{8b}, R^{8c}, R^{8d}: unabhängig voneinander eine für die Reste R⁶ , R^{7a}, R^{7b}, R^{7c} angegebenen Bedeutungen besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal-oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
   wobei unter den bei der Definition der vorstehend genannten Reste erwähnten Arylgruppen Phenyl- oder Naphthylgruppen, vorzugsweise Phenylgruppen zu verstehen sind;
   und R¹ bis R⁵, R⁶, R^{7a}, R^{7b}, R^{7c} die in den Gegenständen 1 bis 12 angegebenen Bedeutungen besitzen,
   bei dem eine Organometall-Verbindung (V), die durch Halogen-Metall-Austausch oder durch Insertion eines Metalls in die Kohlenstoff-Halogen-Bindung einer Halogen-Benzylbenzol-Verbindung der allgemeinen Formel IV 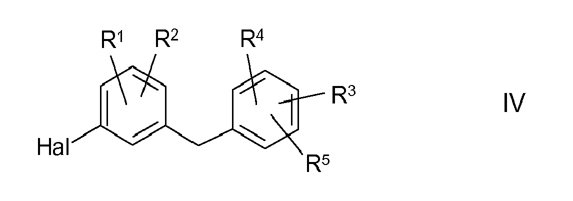 in der Hal CI, Br und I bedeutet und R¹ bis R⁵ wie zuvor definiert sind, und gegebenenfalls anschließender Ummetallierung erhältlich ist, an ein Gluconolacton der allgemeinen Formel VI 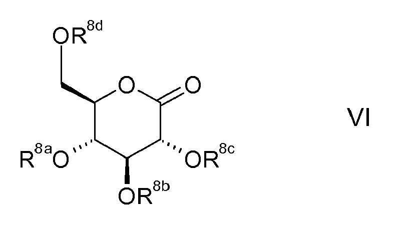 in der R^{8a}, R^{8b}, R^{8c}, R^{8d} wie zuvor definiert sind, addiert wird, und
   anschließend das erhaltene Addukt mit Wasser oder einem Alkohol R'-OH, wobei R' gegebenenfalls substituiertes C₁₋₄-Alkyl bedeutet, in Gegenwart einer Säure umgesetzt wird und optional das in der Umsetzung mit Wasser erhaltene Produkt, in dem R' H bedeutet, in einer nachfolgenden Reaktion mit einem Acylierungsmittel in das Produkt der Formel II überführt wird, worin R' (C₁₋₁₈-Alkyl)carbonyl, (C₁₋₁₈-Alkyl)oxycarbonyl, Arylcarbonyl oder Aryl-(C₁₋₃-alkyl)-carbonyl, das wie angegeben substituiert sein kann, bedeutet.
25. Verfahren gemäß Gegenstand 24, **dadurch gekennzeichnet, dass** die Organometall-Verbindung (V) eine Lithium- oder Magnesium-Verbindung ist.
26. Verfahren zur Herstellung der Verbindungen der allgemeinen Formel I gemäß den Gegenständen 1 bis 12, in der R⁶, R^{7a}, R^{7b} und R^{7c} Wasserstoff bedeuten, **dadurch gekennzeichnet, dass** eine Verbindung der allgemeinen Formel III 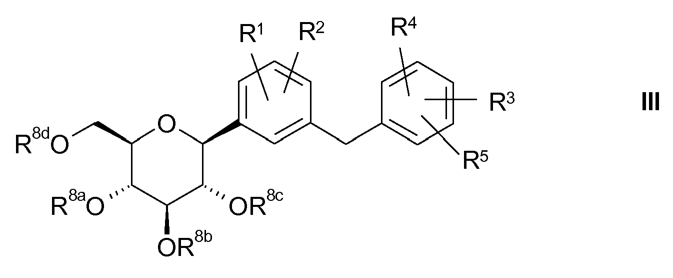 in der
   - R^{8a}, R^{8b}, R^{8c}, R^{8d}: unabhängig voneinander eine für die Reste R⁶ , R^{7a}, R^{7b}, R^{7c} angegebenen Bedeutungen, jedoch mindestens einer der Reste R^{8a}, R^{8b}, R^{8c}, R^{8d} nicht Wasserstoff bedeutet, besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal- oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein-oder mehrfach mit Halogen oder C₁₋₃-Aikoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
   wobei unter den bei der Definition der vorstehend genannten Reste erwähnten Arylgruppen Phenyl- oder Naphthylgruppen, vorzugsweise Phenylgruppen zu verstehen sind;
   und R¹ bis R⁵, R⁶, R^{7a}, R^{7b}, R^{7c} die in den Gegenständen 1 bis 12 angegebenen Bedeutungen besitzen,
   hydrolysiert wird, und
   gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder erforderlichenfalls ein bei den vorstehend beschriebenen Umsetzungen verwendeter Schutzrest wieder abgespalten wird und/oder
   gewünschtenfalls eine so erhaltene Verbindung der allgemeinen Formel I in ihre Stereoisomere aufgetrennt wird und/oder
   eine so erhaltene Verbindung der allgemeinen Formel I in ihre Salze, insbesondere für die pharmazeutische Anwendung in ihre physiologisch verträglichen Salze, überführt wird.
27. Verfahren gemäß Gegenstand 26, **dadurch gekennzeichnet, dass** die Verbindung der Formel III mittels eines Verfahrens gemäß Gegenstand 22 oder 23 erhalten wird.
28. Verbindung der allgemeinen Formel IV 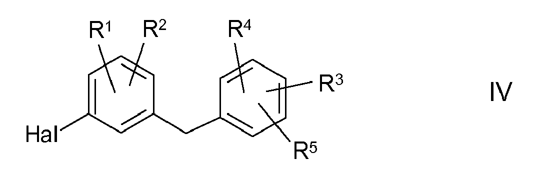 in der Hal Chlor, Brom oder Iod bedeutet und die Reste R¹, R², R³, R⁴ und R⁵ wie in einem oder mehreren der Gegenstände 1 und 3 bis 10 definiert sind.
29. Verbindung der Formel IV gemäß Gegenstand 28 gekennzeichnet durch die Formel 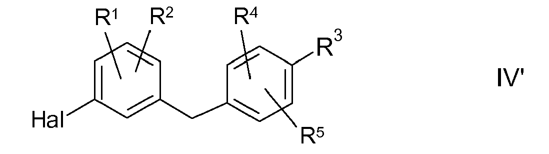 in der Hal Chlor, Brom oder Iod bedeutet und die Reste R¹, R², R⁴ und R⁵ wie in einem oder mehreren der Gegenstände 1, 3, 6, 7, 9, 10 definiert sind und der Rest R³ aus der Gruppe B gemäß Gegenstand 1 oder 4 ausgewählt ist.
30. Verbindung der allgemeinen Formel II 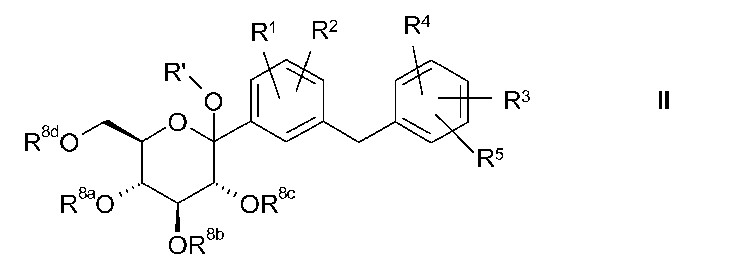 in der
   - R': H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
   - R^{8a}, R^{8b}, R^{8c}, R^{8d}: unabhängig voneinander eine für die Reste R⁶, R^{7a}, R^{7b}, R^{7c} angegebenen Bedeutungen besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal-oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
   - wobei unter gruppen sind;: den bei der Definition der vorstehend genannten Reste erwähnten Aryl-Phenyl- oder Naphthylgruppen, vorzugsweise Phenylgruppen zu verstehen
   - und R¹ bis sind.: R⁵ wie in einem oder mehreren der Gegenstände 1 und 3 bis 10 definiert

### Detailierte Beschreibung der Erfindung

Sofern nicht anders angegeben besitzen die Gruppen, Reste und Substituenten, insbesondere R¹ bis R⁵, A, B, L1, L2, R^{N}, R⁶, R^{7a}, R^{7b}, R^{7c}, R^{8a}, R^{8b}, R^{8c}, R^{8d} die zuvor und nachfolgend angegebenen Bedeutungen.

Kommen Reste, Substituenten oder Gruppen in einer Verbindung mehrfach vor, so können diese eine gleiche oder verschiedene Bedeutungen aufweisen.

Erfindungsgemäß sind Glucopyranosyl-substituierte Benzol-Derivate der allgemeinen Formel I bevorzugt 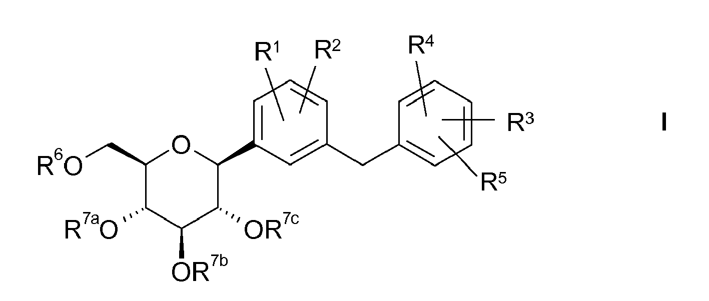 in der
- R¹: ausgewählt ist aus den Bedeutungen der Gruppe A und
falls R³ ausgewählt ist aus den Bedeutungen der Gruppe B, kann R¹ zusätzlich auch ausgewählt sein aus den Bedeutungen Wasserstoff, Fluor, Chlor, Brom, Iod, C₁₋₄-Alkyl, C₂₋₄-Alkenyl-C₁₋₄-alkyl, C₂₋₄-Alkinyl-C₁₋₄-alkyl, C₃₋₇-Cycloalkyl-C₁₋₄-alkyl, C₅₋₇-Cycloalkenyl-C₁₋₄-alkyl, eine durch 1 bis 3 Fluoratome substituierte Methylgruppe, eine durch 1 bis 5 Fluoratome substituierte Ethylgruppe, C₁₋₄-Alkoxy, eine durch 1 bis 3 Fluoratome substituierte Methoxygruppe, eine durch 1 bis 5 Fluoratome substituierte Ethoxygruppe, eine durch eine Hydroxy- oder C₁₋₃-Alkoxygruppe substituierte C₁₋₄-Alkylgruppe, eine durch eine Hydroxy- oder C₁₋₃-Alkoxygruppe substituierte C₂₋₄-Aikoxygruppe, C₃₋₆-Cycloalkyl-C₁₋₃-alkoxy oder Hydroxy,
wobei in den vorstehend genannten Cycloalkyl- und Cycloalkenyl-Ringen ein oder zwei Methylengruppen unabhängig voneinander durch O oder CO ersetzt sein können, und
- R²: Wasserstoff, Fluor, Chlor, Brom, Hydroxy, C₁₋₄-Alkyl, C₁₋₄-Alkoxy, Cyan oder Nitro, wobei die Alkyl- oder Alkoxygruppe ein oder mehrfach mit Fluor substituiert sein kann, und
- R³: ausgewählt ist aus den Bedeutungen der Gruppe B und
falls R¹ ausgewählt ist aus den Bedeutungen der Gruppe A, kann R³ zusätzlich auch ausgewählt sein aus den Bedeutungen Wasserstoff, Fluor, Chlor, Brom, Iod, C₁₋₆-Alkyl, C₂₋₄-Alkenyl-C₁₋₄-alkyl, C₂₋₄-Alkinyl-C₁₋₄-alkyl, C₃₋₇-Cycloalkyl, C₅₋₇-Cyclo-alkenyl, C₃₋₇-Cycloalkyl-C₁₋₄-alkyl, C₅₋₇-Cycloalkenyl-C₁₋₄-alkyl, C₃₋₆-Cycloalkyliden-methyl, Hydroxy, C₁₋₆-Alkoxy, C₃₋₆-Cycloalkyl-C₁₋₃-alkoxy, Aryl, Aryl-C₁₋₃-alkyl, Heteroaryl, Heteroaryl-C₁₋₃-alkyl, Aryloxy, Aryl-C₁₋₃-alkyl-oxy, eine durch 1 bis 3 Fluoratome substituierte Methyl- oder Methoxygruppe, eine durch 1 bis 5 Fluor-atome substituierte C₂₋₄-Alkyl- oder C₂₋₄-Alkoxygruppe, eine durch eine Cyangruppe substituierte C₁₋₄-Alkylgruppe, eine durch eine Hydroxy- oder C₁₋₃-Alkyloxygruppe substituierte C₁₋₄-Alkylgruppe, Cyan, Carboxy, C₁₋₃-Alkoxycarbonyl, Aminocarbonyl, (C₁₋₃-Alkylamino)carbonyl, Di-(C₁₋₃-alkyl)aminocarbonyl, Pyrrolidin-1-ylcarbonyl, Piperidin-1-ylcarbonyl, Morpholin-4-ylcarbonyl, Piperazin-1-yl-carbonyl, 4-(C₁₋₃-Alkyl)-piperazin-1-ylcarbonyl, (C₁₋₄-Alkyl)carbonylamino-, C₁₋₄-Alkylsulfonylamino, C₁₋₄-Alkylsulfanyl-, C₁₋₄-Alkylsulfinyl-, C₁₋₄-Alkylsulfonyl-, Arylsulfonylamino, Aryl-C₁₋₃-alkylsulfonylamino oder Arylsulfonyl,
- R⁴, R⁵: unabhängig voneinander Wasserstoff, Fluor, Chlor, Brom, Iod, Cyan, Nitro, C₁₋₃-Alkyl, C₁₋₃-Alkoxy, durch 1 bis 3 Fluoratome substituiertes Methyl- oder Methoxy,
- A: C₂₋₆-Alkin-1-yl, C₂₋₆-Alken-1-yl, C₃₋₇-Cycloalkyl, C₅₋₇-Cycloalkenyl, Aryl, Heteroaryl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Arylaminocarbonyl, Heteroarylaminocarbonyl, C₁₋₄-Alkoxycarbonyl, Aryl-C₁₋₃-alkoxycarbonyl, Heteroaryl-C₁₋₃-alkoxycarbonyl, Amino, C₁₋₄-Alkylamino, Di-(C₁₋₃-alkyl)amino, Pyrrolidin-1-yl, Pyrrolidin-2-on-1-yl, Piperidin-1-yl, Piperidin-2-on--yl, Morpholin-4-yl, Morpholin-3-on-4-yl, Piperazin-1-yl, 4-(C₁₋₃-Alkyl)piperazin-1-yl, C₁₋₄-Alkylcarbonylamino, Arylcarbonylamino, Heteroarylcarbonylamino, C₃₋₇-Cyclo-alkyloxy, C₅₋₇-Cycloalkenyloxy, Aryloxy, Heteroaryloxy, C₁₋₄-Alkylsulfinyl, C₁₋₄-Alkyl-sulfonyl, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Arylsulfanyl, Arylsulfinyl, Arylsulfonyl, Heteroarylsulfanyl, Heteroarylsulfinyl, Heteroarylsulfonyl, Cyan oder Nitro bedeutet,
wobei die vorstehend genannten Alkinyl- und Alkenylgruppen ein- oder mehrfach mit Fluor oder Chlor substituiert sein können, und
wobei die vorstehend genannten Alkinyl- und Alkenylgruppen ein- oder zweifach mit gleichen oder verschiedenen Resten L1 substituiert sein können, und
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
- B: Tri-(C₁₋₄-alkyl)silyl-C₁₋₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Arylcarbonylamino, Heteroarylcarbonylamino, Nitro, C₃₋₇-Cycloalkyloxy, C₅₋₇-Cycloalkenyloxy, C₃₋₇-Cycloalkylsulfanyl, C₃₋₇-Cycloalkylsulfinyl, C₃₋₇-Cycloalkyl- sulfonyl, C₅₋₇-Cycloalkenylsulfanyl, C₅₋₇-Cycloalkenylsulfinyl, C₅₋₇-Cycloalkenyl-sulfonyl, Arylsulfanyl, Arylsulfinyl, Heteroarylsulfanyl oder Heteroarylsulfinyl,
wobei die vorstehend genannten Alkinyl- und Alkenylgruppen ein- oder mehrfach mit Fluor oder Chlor substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
- R^{N}: H oder C₁₋₄-Alkyl,
- L1: unabhängig voneinander ausgewählt aus der Gruppe bestehend aus Cyan-, Nitro-, Aryl-, Heteroaryl-, C₁₋₄-Alkylcarbonyl-, Arylcarbonyl-, Heteroarylcarbonyl-, Aminocarbonyl-, C₁₋₄-Alkylaminocarbonyl-, Di-(C₁₋₃-alkyl)aminocarbonyl-, Pyrrolidin-1-ylcarbonyl-, Piperidin-1-ylcarbonyl-, Morpholin-4-ylcarbonyl, Arylaminocarbonyl-, Heteroarylaminocarbonyl-, C₁₋₄-Alkoxycarbonyl-, Aryl-C₁₋₃-alkoxycarbonyl-, Heteroaryl-C₁₋₃-alkoxycarbonyl-, C₁₋₄-Alkyloxy-, Aryloxy-, Heteroaryloxy-, C₁₋₄-Alkylsulfanyl-, Arylsulfanyl-, Heteroarylsulfanyl-, C₁₋₄-Alkylsulfinyl-, Arylsulfinyl-, Heteroarylsulfinyl-, C₁₋₄-Alkylsulfonyl-, Arylsulfonyl- und Heteroarylsulfonyl-; und
- L2: unabhängig voneinander ausgewählt aus der Gruppe bestehend aus Fluor, Chlor, Brom, Iod, C₁₋₃-Alkyl, Difluormethyl, Trifluormethyl, C₁₋₃-Alkoxy, Difluormethoxy, Trifluormethoxy und Cyan; und
- R⁶, R^{7a}, R^{7b}, R^{7c}: unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl besitzen,
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl-, Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L2 substituiert sein können;
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Nachfolgend werden bevorzugte Bedeutungen einzelner Gruppen und Substituenten der erfindungsgemäßen Verbindungen angegeben.

Der Rest R³ steht vorzugsweise in meta- oder para Position zur -CH₂-Brücke, so dass Verbindungen gemäß der folgenden Formeln I.1 und I.2, insbesondere der Formel I.2, bevorzugt sind:

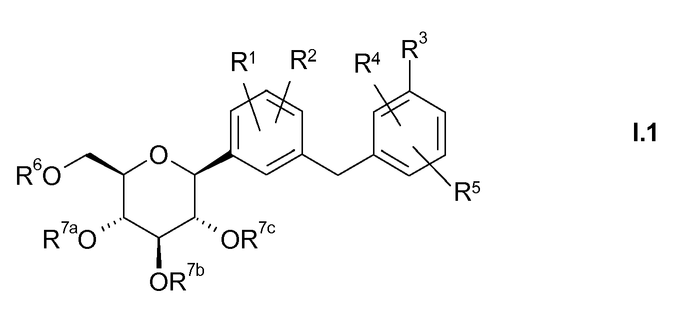

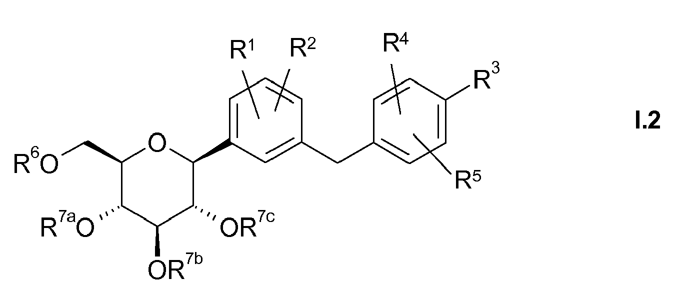

Die in den Gruppen L1, R¹, R³, A und B vorkommende Bezeichnung Aryl bedeutet vorzugsweise Phenyl.

Die in den Gruppen L1, R¹, R³, A und B vorkommende Bezeichnung Heteroaryl bedeutet vorzugsweise Pyridinyl, Pyrimidinyl, Pyridazinyl, Pyrazinyl, Triazinyl, Imidazolyl, Pyrazolyl, Triazolyl, Tetrazolyl, Oxazolyl, Oxadiazolyl, Thiazolyl oder Thiadiazolyl.

Die Gruppe A bedeutet vorzugsweise C₂₋₆-Alkin-1-yl, C₂₋₆-Alken-1-yl, C₃₋₇-Cycloalkyl, C₅₋₇-Cycloalkenyl, C₁₋₄-Alkylcarbonyl, Aminocarbonyl, C₁₋₄-Alkylaminocarbonyl, Di-(C₁₋₃-Alkyl)-aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, C₁₋₄-Alkoxycarbonyl, Amino, C₁₋₄-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, C₁₋₄-Alkylcarbonylamino, C₃₋₇-Cycalkyloxy, C₅₋₇-Cycalkenyloxy, C₁₋₄-Alkylsulfinyl, C₁₋₄-Alkylsulfonyl, C₃₋₇-Cycalkylsulfanyl, C₃₋₇-Cycloalkylsulfinl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Cyan und Nitro,
wobei die vorstehend genannten Alkinyl- und Alkenylgruppen ein- oder mehrfach mit Fluor oder Chlor, vorzugsweise Fluor, substituiert sein können, und
wobei die vorstehend genannten Alkinyl- und Alkenylgruppen ein- oder zweifach mit gleichen oder verschiedenen Resten L1 substituiert sein können, und
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N}, vorzugsweise O oder CO, ganz besonders bevorzugt durch O, ersetzt sein können.

Besonders bevorzugt bedeutet die Gruppe A C₂₋₆-Alkin-1-yl, C₂₋₆-Alken-1-yl, C₃₋₇-Cycloalkyl, C₅₋₇-Cycloalkenyl, C₃₋₇-Cycloalkyloxy, C₅₋₇-Cycloalkenyloxy, C₁₋₄-Alkylsulfinyl, C₁₋₄-Alkylsulfonyl, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Cyan und Nitro,
wobei die vorstehend genannten Alkinyl- und Alkenylgruppen ein- oder mehrfach mit Fluor oder Chlor, vorzugsweise Fluor, substituiert sein können, und
wobei die vorstehend genannten Alkinyl- und Alkenylgruppen ein- oder zweifach mit gleichen oder verschiedenen Resten L1 substituiert sein können, und
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten C₅₋₆-Cycloalkyl-Ringen eine Methylengruppe durch O ersetzt sein kann.

Ganz besonders bevorzugt bedeutet die Gruppe A C₂₋₆-Alkin-1-yl, C₂₋₆-Alken-1-yl, C₃₋₇-Cycloalkyl, C₃₋₇-Cycloalkyloxy, Cyan, wobei in C₅₋₆-Cycloalkyl-Gruppen eine Methyleneinheit durch O ersetzt sein kann.

Beispiele der ganz besonders bevorzugten Bedeutungen der Gruppe A sind Ethinyl, Prop-1-in-1-yl, But-1-in-1-yl, Cyan, Cyclopropyloxy, Cyclobutyloxy, Cyclopentyloxy, Cyclohexyloxy.

Die Gruppe B bedeutet vorzugsweise Tri-(C₁₋₄-alkyl)silyl-C₁₋₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Nitro, C₃₋₇-Cycloalkyloxy, C₅₋₇-Cycloalkenyloxy, C₃₋₇-Cycloalkylsulfanyl, C₃₋₇-Cycloalkylsulfinyl, C₃₋₇-Cycoalkylsulfonyl, C₅₋₇-Cycloalkenylsulfanyl, C₅₋₇-Cycloalkenylsulfinyl, C₅₋₇-Cycloalkenylsulfonyl,
wobei die vorstehend genannten Alkinyl- und Alkenylgruppen ein- oder mehrfach mit Fluor oder Chlor, vorzugsweise Fluor, substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR ^{N} vorzugsweise O, CO, S, SO₂ oder NR^{N}, ganz besonders bevorzugt durch O oder CO ersetzt sein können.

Besonders bevorzugt bedeutet die Gruppe B Tri-(C₁₋₄-alkyl)silyl-C₁₋₆-alkyl, C₂₋₆-Alkin-1-yl, C₂₋₆-Alken-1-yl, Nitro, C₃₋₇-Cycloalkyloxy, C₅₋₇-Cycloalkenyloxy, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl,
wobei die vorstehend genannten Alkinyl- und Alkenylgruppen ein- oder mehrfach mit Fluor oder Chlor, vorzugsweise Fluor, substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N}, vorzugsweise O, CO, S, SO₂ oder NR^{N}, ganz besonders bevorzugt durch O oder CO, ersetzt sein können.

Ganz besonders bevorzugt bedeutet die Gruppe B Tri-(C₁₋₄-alkyl)silyl-C₁₋₆-alkyl, C₂₋₆-Alkin-1-yl, C₂₋₆-Alken-1-yl, C₃₋₇-Cycloalkyloxy, C₅₋₇-Cycloalkenyloxy, C₃₋₇-Cycloalkylsulfanyl, C₅₋₇-Cycloalkenylsulfanyl, wobei die vorstehend genannten Alkinyl- und Alkenylgruppen ein- oder mehrfach mit Fluor oder einfach mit Chlor oder dem Rest L1 substituiert sein können, und
wobei in den Cycloalkyl- und Cycloalkenylgruppen ein oder zwei Methylengruppen unabhängig voneinander durch O, CO, S, SO₂ oder NR^{N}, insbesondere O oder CO, ersetzt sein können.

Beispiele ganz besonders bevorzugter Bedeutungen der Gruppe B sind Trimethylsilylethyl, Ethinyl, 1-Propin-1-yl, 1-Butin-1-yl, tert.-Butylethinyl, 2-Hydroxyprop-2-ylethinyl, 2-Methoxy-prop-2-ylethinyl, 3-Hydroxy-1-propin-1-yl, 3-Methoxy-1-propin-1-yl, Ethenyl, 1-Propenyl, 1-Butenyl, tert.-Butylethenyl, Cyclopropyloxy, Cyclobutyloxy, Cyclopentyloxy, Cyclohexyloxy, Tetrahydrofuranyloxy, Tetrahydrothiophenyloxy, 1,1-Dioxotetrahydrothiophenyloxy, Tetrahydropyranyloxy, Tetrahydrothiopyranyloxy, 1,1-Dioxotetrahydrothiopyranyloxy, Tetrahydrofuranonyloxy, Piperidinyloxy, Piperidinonyloxy, Pyrrolidin-3-yloxy, Pyrrolidinon-3-yloxy, Tetrahydrofuranyl-sulfanyl, Cyclopropylsulfanyl, Cyclobutylsulfanyl, Cyclopentylsulfanyl und Cyclohexylsulfanyl, wobei die -NH-Gruppe in einem Piperidinyl-, Piperidinonyl-, Pyrrolidinyl- oder Pyrrolidinonyl-Ring mit R^{N}, insbesondere C₁₋₃-Alkyl oder Acetyl, substituiert sein kann.

Ganz besonders bevorzugte Bedeutungen sind hierbei Trimethylsilylethyl, Ethinyl, 2-Hydroxyprop-2-ylethinyl, 2-Methoxyprop-2-ylethinyl, 3-Hydroxy-1-propin-1-yl, 3-Methoxy-1-propin-1-yl, Cyclopropyloxy, Cyclobutyloxy, Cyclopentyloxy, Cyclohexyloxy, Tetrahydrofuran-3-yloxy, Tetrahydropyran-4-yloxy, Piperidin-4-yloxy, N-Methylpiperidin-4-yloxy und N-Acetylpiperidin-4-yloxy. Hiervon besonders hervorzuhebende Beispiele sind Ethinyl, Trimethylsilylethyl, Cyclobutyloxy, Cyclopentyloxy, Cyclohexyloxy, Tetrahydrofuran-3-yloxy und Tetrahydropyran-4-yloxy.

Sind in den Resten oder Gruppen A, B, R¹ oder R³ Cycloalkyl- oder Cycloalkenyl-Ringe vorhanden, in denen zwei Methylengruppen durch O, S oder NR^{N} ersetzt sind oder durch S, NR^{N}, CO, SO oder SO₂ ersetzt sind, so sind diese Methylengruppen vorzugsweise nicht unmittelbar miteinander verbunden. Sind jedoch zwei Methylengruppen durch O und CO oder durch NR^{N} und CO ersetzt, so können diese unmittelbar miteinander verbunden sein, so dass eine -O-CO- bzw. -NR^{N}-CO-Gruppe gebildet wird.

Bevorzugte Bedeutungen des Rests L1 sind ausgewählt aus der Gruppe bestehend aus Hydroxy-, Cyan-, C₃₋₆-Cycloalkyl, C₁₋₄-Alkylcarbonyl-, Aminocarbonyl-, C₁₋₄-Alkylaminocarbonyl-, Di-(C₁₋₃-alkyl)aminocarbonyl-, Pyrrolidin-1-ylcarbonyl-, Piperidin-1-ylcarbonyl-, Morpholin-4-ylcarbonyl, C₁₋₄-Alkoxycarbonyl-, C₁₋₄-Alkyloxy-, C₁₋₄-Alkylsulfanyl-, C₁₋₄-Alkylsulfinyl-, und C₁₋₄-Alkylsulfonyl-.

Besonders bevorzugte Bedeutungen des Rests L1 sind ausgewählt aus der Gruppe bestehend aus Hydroxy-, C₁₋₄-Alkyloxy- und C₁₋₄-Alkylsulfanyl-.

Bedeutet L1 Hydroxy, so ist die Hydroxygruppe nicht unmittelbar an ein C-Atom einer Doppel- oder Dreifachbindung geknüpft.

Erfindungsgemäße Verbindungen gemäß einer ersten Ausführungsform dieser Erfindung können beschrieben werden durch die allgemeine Formel I, insbesondere die Formeln I.1 und I.2, besonders bevorzugt die Formel I.2, in der
- R³: ausgewählt ist aus einer der zuvor angeführten Bedeutungen der Gruppe B und
die übrigen Reste und Substituenten wie zuvor und nachstehend definiert sind,
einschließlich deren Tautomere, deren Stereoisomere, deren Gemische und deren Salze.

Gemäß dieser Ausführungsform bevorzugte Bedeutungen des Rests R¹ sind Wasserstoff, Fluor, Chlor, Brom, Iod, C₁₋₄-Alkyl, C₂₋₆-Alkinyl, C₁₋₄-Alkoxy, C₂₋₄-Alkenyl-C₁₋₄-alkoxy, C₂₋₄-Alkinyl-C₁₋₄-alkoxy, durch 1 bis 3 Fluoratome substituiertes Methyl, durch 1 bis 5 Fluoratome substituiertes Ethyl, durch 1 bis 3 Fluoratome substituiertes Methoxy, durch 1 bis 5 Fluoratome substituiertes Ethoxy, durch eine Hydroxy- oder C₁₋₃-Alkoxygruppe substituiertes C₁₋₄-Alkyl, durch eine Hydroxy- oder C₁₋₃-Alkoxygruppe substituiertes C₂₋₄-Alkoxy, C₂₋₆-Alkenyl, C₃₋₆-Cycloalkyl, C₃₋₆-Cycloalkyl-C₁₋₃-alkyl, C₃₋₇-Cycloalkyloxy, C₃₋₆-Cycloalkyl-C₁₋₃-alkoxy, C₅₋₇-Cycloalkenyoxy,Hydroxy, Amino, Nitro oder Cyan, wobei in den C₅₋₆-Cycloalkylgruppen eine Methylengruppe durch O ersetzt sein kann.

Besonders bevorzugte Bedeutungen sind hierbei Wasserstoff, Fluor, Chlor, Brom, Cyan, Methyl, Ethyl, Isopropyl, Difluormethyl, Trifluormethyl, Ethinyl, Prop-1-in-1-yl, But-1-in-1-yl, Hydroxy, Methoxy, Ethoxy, Difluormethoxy, Cyclopropyloxy, Cyclobutyloxy, Cyclopentyloxy, Cyclohexyloxy, insbesondere Methyl und Chlor.

Erfindungsgemäße Verbindungen gemäß einer zweiten Ausführungsform dieser Erfindung können beschrieben werden durch die allgemeine Formel I, insbesondere die Formeln I.1 und I.2, besonders bevorzugt die Formel I.2, in der
- R¹: ausgewählt ist aus den zuvor angeführten Bedeutungen der Gruppe A und die übrigen Reste und Substituenten wie zuvor und nachstehend definiert sind,
einschließlich deren Tautomere, deren Stereoisomere, deren Gemische und deren Salze.

Gemäß dieser zweiten Ausführungsform bevorzugte Bedeutungen des Rests R³ sind Wasserstoff, Fluor, Chlor, Brom, Hydroxy, Cyan, C₁₋₆-Alkyl, Trimethylsilylethyl, C₂₋₆-Alkenyl, C₂₋₆-Alkinyl, Difluormethyl, Trifluormethyl, C₃₋₇-Cycloalkyl, C₅₋₇-Cycloalkenyl, C₁₋₆-Alkyloxy, Difluormethoxy, Trifluormethoxy, Pentafluorethoxy, C₃₋₇-Cycloalkyloxy, Tetrahydrofuranyloxy, Tetrahydrofuranonyloxy, C₁₋₆-Alkylsulfanyl, Cyclopropylidenmethyl-, Aryl oder Heteroaryl.

Gemäß dieser zweiten Ausführungsform besonders bevorzugte Bedeutungen des Rests R³ sind Wasserstoff, Fluor, Chlor, Methyl, Ethyl, Isopropyl, tert.-Butyl, Ethinyl, 1-Propinyl, Trimethylsilylethyl, Difluormethyl, Trifluormethyl, Cyclopropyl, Cyclobutyl, Cyclopentyl, Methoxy, Ethoxy, Isopropoxy, Cyclopentyloxy, Difluormethoxy, Trifluormethoxy, Pentafluorethoxy, Tetrahydrofuran-3-yloxy, Tetrahydrofuran-2-on-3-yloxy, Methylsulfanyl, Ethylsulfanyl, Isopropylsulfanyl, Cyclopropylidenmethyl-, Phenyl-, Fluorphenyl, Pyridinyl, Pyrimidinyl, Pyridazinyl, Pyrazinyl, Imidazolyl, Pyrazolyl, Triazolyl, Tetrazolyl, Oxazolyl, Oxadiazolyl, Thiazolyl oder Thiadiazolyl.

Gemäß dieser zweiten Ausführungsform ganz besonders bevorzugte Bedeutungen des Rests R³ sind Wasserstoff, Fluor, Chlor, Methyl, Ethyl, Isopropyl, tert.-Butyl, Ethinyl, 1-Propinyl, Trimethylsilylethyl, Difluormethyl, Trifluormethyl, Cyclopropyl, Cyclobutyl, Cyclopentyl, Methoxy, Ethoxy, Isopropoxy, Cyclopentyloxy, Difluormethoxy, Trifluormethoxy, Pentafluorethoxy, Tetrahydrofuran-3-yloxy, Tetrahydrofuran-2-on-3-yloxy, Methylsulfanyl, Ethylsulfanyl, Isopropylsulfanyl, Cyclopropylidenmethyl. Beispiele solcher besonders bevorzugter Bedeutungen sind hierbei Methyl, Ethyl, Methoxy, Ethoxy, Trimethylsilylethyl, Ethinyl, Cyclopentyloxy, Tetrahydrofuran-3-yloxy, Tetrahydrofuran-2-on-3-yloxy, insbesondere Trimethylsilylethyl, Ethoxy, Cyclopentyloxy und Tetrahydrofuran-3-yloxy.

Nachfolgend werden Bedeutungen weiterer Reste und Substituenten angegeben, die gemäß der allgemeinen Formel I, der Formeln I.1 und I.2 als auch gemäß der zuvor beschriebenen Ausführungsformen als bevorzugt anzusehen sind:
Bevorzugte Bedeutungen des Rests R² sind Wasserstoff, Fluor, Chlor, Brom, Methyl, Hydroxy, Methoxy, Ethoxy, Trifluormethoxy, Cyan, Nitro und durch 1 bis 3 Fluoratome substituiertes Methyl.

Besonders bevorzugte Bedeutungen des Rests R² sind Wasserstoff, Fluor, Hydroxy, Methoxy, Ethoxy und Methyl, insbesondere Wasserstoff und Methyl.

Bevorzugte Bedeutungen des Rests R⁴ sind Wasserstoff und Fluor, insbesondere Wasserstoff.

Bevorzugte Bedeutungen des Rests R⁵ sind Wasserstoff und Fluor, insbesondere Wasserstoff.

Der Rest R^{N} bedeutet vorzugsweise H, Methyl, Ethyl oder Acetyl.

Der Rest R⁶ bedeutet erfindungsgemäß vorzugsweise Wasserstoff, (C₁₋₈-Alkyl)oxycarbonyl-, C₁₋₈-Alkylcarbonyl- oder Benzoyl, insbesondere Wasserstoff oder (C₁₋₆-Alkyl)oxycarbonyl, C₁₋₆-Alkylcarbonyl, besonders bevorzugt Wasserstoff, Methylcarbonyl, Methoxycarbonyl oder Ethoxycarbonyl, ganz besonders bevorzugt Wasserstoff oder Methoxycarbonyl.

Die Substituenten R^{7a}, R^{7b}, R^{7c} bedeuten unabhängig voneinander vorzugsweise Wasserstoff, (C₁₋₈-Alkyl)oxycarbonyl-, (C₁₋₁₈-Alkyl)carbonyl, Benzoyl, insbesondere Wasserstoff oder (C₁₋₆-Alkyl)oxycarbonyl-, (C₁₋₈-Alkyl)carbonyl, besonders bevorzugt Wasserstoff, Methoxycarbonyl, Ethoxycarbonyl, Methylcarbonyl oder Ethylcarbonyl. Ganz besonders bevorzugt bedeuten R^{7a}, R^{7b} und R^{7c} Wasserstoff.

Die Verbindungen der Formel I, in denen R⁶, R^{7a}, R^{7b} und R^{7c} eine erfindungsgemäße, von Wasserstoff verschiedene Bedeutung aufweisen, beispielsweise C₁₋₈-Alkylcarbonyl, eignen sich bevorzugt als Zwischenprodukte bei der Synthese von Verbindungen der Formel I in denen R^{7a}, R^{7b} und R^{7c} Wasserstoff bedeuten.

Besonders bevorzugte Verbindungen der allgemeinen Formel I sind ausgewählt aus der Gruppe der Formeln I.2a bis I.2d, insbesondere I.2c: 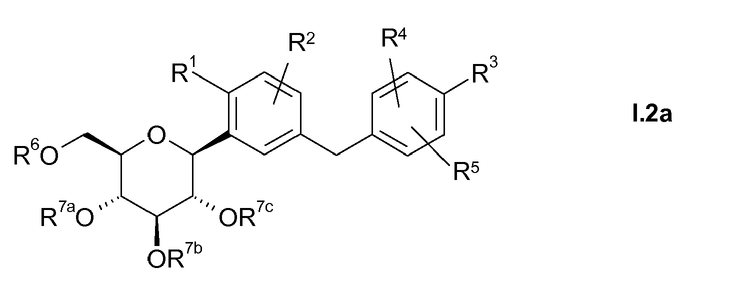 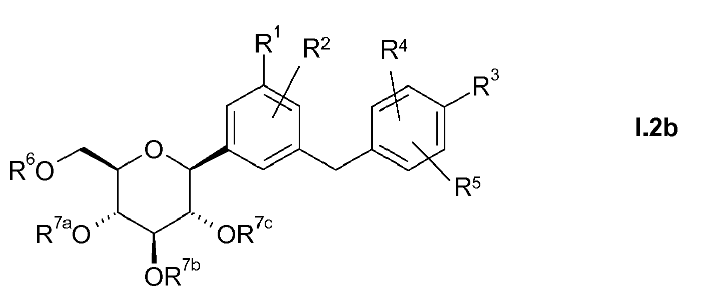 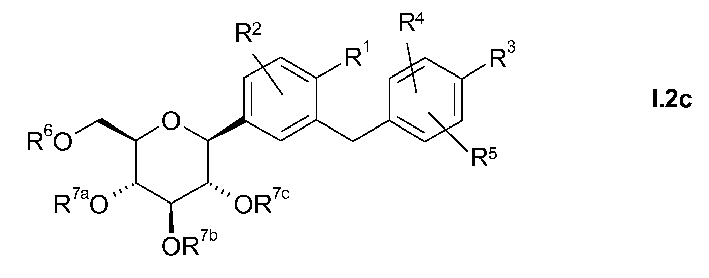 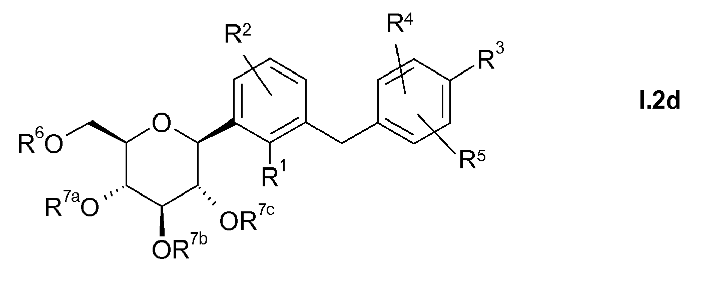 wobei die Reste R¹ bis R⁶ und R^{7a}, R^{7b}, R^{7c} eine der zuvor angegebenen, insbesondere eine der als bevorzugt angegebenen Bedeutungen aufweisen; insbesondere wobei
- R¹: Wasserstoff, Fluor, Chlor, Brom, Iod, C₁₋₄-Alkyl, C₂₋₆-Alkinyl, C₁₋₄-Alkoxy, C₂₋₄-Alkenyl-C₁₋₄-alkoxy, C₂₋₄-Alkinyl-C₁₋₄-alkoxy, durch 1 bis 3 Fluoratome substituiertes Methyl, durch 1 bis 5 Fluoratome substituiertes Ethyl, durch 1 bis 3 Fluoratome substituiertes Methoxy, durch 1 bis 5 Fluoratome substituiertes Ethoxy, durch eine Hydroxy- oder C₁₋₃-Alkoxygruppe substituiertes C₁₋₄-Alkyl, durch eine Hydroxy- oder C₁₋₃-Alkoxygruppe substituiertes C₂₋₄-Alkoxy, C₂₋₆-Alkenyl, C₃₋₆-Cycloalkyl, C₃₋₆-Cycloalkyl-C₁₋₃-alkyl, C₃₋₇-Cycloalkyloxy, C₃₋₆-Cycloalkyl-C₁₋₃-alkoxy, C₅₋₇-Cyclo-alkenyloxy, Hydroxy, Amino, Nitro oder Cyan bedeutet, wobei in den C₅₋₆-Cycloalkylgruppen eine Methylengruppe durch O ersetzt sein kann; besonders bevorzugt Wasserstoff, Fluor, Chlor, Brom, Cyan, Methyl, Ethyl, Isopropyl, Difluormethyl, Trifluormethyl, Ethinyl, Prop-1-in-1-yl, But-1-in-1-yl, Hydroxy, Methoxy, Ethoxy, Difluormethoxy, Cyclopropyloxy, Cyclobutyloxy, Cyclopentyloxy
oder Cyclohexyloxy bedeutet; und
- R²: Wasserstoff, Fluor, Hydroxy, Methoxy, Ethoxy oder Methyl, insbesondere Wasserstoff oder Methyl bedeutet; und
- R³: ausgewählt ist aus der Gruppe B bestehend aus Trimethylsilylethyl, Ethinyl, 1-Propin-1-yl, 1-Butin-1-yl, tert.-Butylethinyl, 2-Hydroxyprop-2-ylethinyl, 2-Methoxy-prop-2-ylethinyl, 3-Hydroxy-1-propin-1-yl, 3-Methoxy-1-propin-1-yl, Ethenyl, 1-Propenyl, 1-Butenyl, tert.-Butylethenyl, Cyclopropyloxy, Cyclobutyloxy, Cyclo-pentyloxy, Cyclohexyloxy, Tetrahydrofuranyloxy, Tetrahydrothiophenyloxy, 1,1-Dioxotetrahydrothiophenyloxy, Tetrahydropyranyloxy, Tetrahydrothiopyranyloxy, 1,1-Dioxotetrahydrothiopyranyloxy, Tetrahydrofuranonyloxy, Piperidinyloxy, Piperidinonyloxy, Pyrrolidin-3-yloxy, Pyrrolidinon-3-yloxy, Tetrahydrofuranyl-sulfanyl, Cyclopropylsulfanyl, Cyclobutylsulfanyl, Cyclopentylsulfanyl und Cyclohexylsulfanyl, wobei die -NH-Gruppe in einem Piperidinyl-, Piperidinonyl-, Pyrrolidinyl- oder Pyrrolidinonyl-Ring mit R^{N}, insbesondere C₁₋₃-Alkyl oder Acetyl, substituiert sein kann; besonders bevorzugt ausgewählt aus Trimethylsilylethyl, Ethinyl, 2-Hydroxyprop-2-ylethinyl, 2-Methoxyprop-2-ylethinyl, 3-Hydroxy-1-propin-1-yl, 3-Methoxy-1-propin-1-yl, Cyclopropyloxy, Cyclobutyloxy, Cylopentyloxy, Cyclohexyloxy, Tetrahydrofuran-3-yloxy, Tetrahydropyran-4-yloxy, Piperidin-4-yloxy, N-Methylpiperidin-4-yloxy und N-Acetylpiperidin-4-yloxy; und
- R⁴: Wasserstoff oder Fluor, insbesondere Wasserstoff bedeutet; und
- R⁵: Wasserstoff oder Fluor, insbesondere Wasserstoff bedeutet; und
- R⁶: Wasserstoff, (C₁₋₆-Alkyl)oxycarbonyl, (C₁₋₆-Alkyl)carbonyl oder Benzoyl, insbesondere Wasserstoff, Methylcarbonyl, Methoxycarbonyl oder Ethoxycarbonyl, ganz besonders bevorzugt Wasserstoff bedeutet; und
- R^{7a}, R^{7b}, R^{7c}: unabhängig voneinander Wasserstoff, (C₁₋₆-Alkyl)oxycarbonyl-, (C₁₋₈-Alkyl)carbonyl oder Benzoyl, insbesondere Wasserstoff, Methoxycarbonyl, Ethoxycarbonyl, Methylcarbonyl oder Ethylcarbonyl, besonders bevorzugt Wasserstoff bedeuten;
einschließlich deren Tautomere, deren Stereoisomere, deren Gemische und deren Salze.

Gemäß einer Variante der zuvor angeführten Ausführungsformen sind diejenigen Verbindungen auch bevorzugt, in denen die Phenylgruppe, die den Substituenten R³ trägt, mindestens einen weiteren, von Wasserstoff verschiedenen Substituenten R⁴ und/oder R⁵ aufweist. Nach dieser Variante sind diejenigen Verbindungen besonders bevorzugt, die einen Substituenten R⁴ in der Bedeutung Fluor aufweisen.

Der Phenylrest, der den Substituenten R³ trägt, ist vorzugsweise maximal einfach fluoriert.

Die im nachfolgenden experimentellen Teil angegebenen Verbindungen der allgemeinen Formel I, sowie deren Derivate, in denen R⁶ eine erfindungsgemäße, von Wasserstoff verschiedene Bedeutung aufweist, insbesondere R⁶ Ethoxycarbonyl oder Methoxycarbonyl bedeutet, einschließlich deren Tautomere, deren Stereoisomere und deren Gemische, sind erfindungsgemäß bevorzugt.

Besonders bevorzugte Verbindungen der allgemeinen Formel I sind ausgewählt aus der Gruppe :
(1) 1-Chlor-2-(4-cyclopentyloxybenzyl)-4-(β-D-glucopyranos-1-yl)-benzol
(2) 1-Chlor-4-(β-D-glucopyranos-1-yl)-2-[4-((R)-tetrahydrofuran-3-yloxy)-benzyl]-benzol
(3) 1-Chlor-4-(β-D-glucopyranos-1-yl)-2-[4-((S)-tetrahydrofuran-3-yloxy)-benzyl]-benzol
(4) 1-Chlor-4-(β-D-glucopyranos-1-yl)-2-[4-(tetrahydrofuran-2-on-3-yloxy)-benzyl]-benzol
(5) 1-Chlor-4-(β-D-glucopyranos-1-yl)-2-(4-cyclobutyloxy-benzyl)-benzol
(6) 1-Chlor-4-(β-D-glucopyranos-1-yl)-2-(4-cyclohexyloxy-benzyl)-benzol
(7) 1-Chlor-4-(β-D-glucopyranos-1-yl)-2-[4-(tetrahydropyran-4-yloxy)-benzyl]-benzol
(8) 1-Chlor-4-(β-D-glucopyranos-1-yl)-2-[4-(1-acetyl-piperidin-4-yloxy)-benzyl]-benzol
(10) 1-(β-D-Glucopyranos-1-yl)-4-methyl-3-[4-(tetrahydrofuran-3-yloxy)-benzyl]-benzol
(11) 1-(β-D-Glucopyranos-1-yl)-4-methyl-3-[4-(2-trimethylsilyl-ethyl)-benzyl]-benzol
(12) 1-Chlor-4-(β-D-glucopyranos-1-yl)-2-(4-ethinyl-benzyl)-benzol
(13) 1-Chlor-4-(β-D-glucopyranos-1-yl)-2-[4-(piperdin-4-yloxy)-benzyl]-benzol
(14) 1-Fluor-4-(β-D-glucopyranos-1-yl)-2-(4-ethinyl-benzyl)-benzol
(15) 1-(β-D-Glucopyranos-1-yl)-3-(4-ethinyl-benzyl)-benzol
(16) 1-Ethinyl-4-(β-D-glucopyranos-1-yl)-2-(4-ethoxy-benzyl)-benzol
(17) 1-Methoxy-4-(β-D-glucopyranos-1-yl)-2-(4-ethinyl-benzyl)-benzol
sowie deren Derivate, in denen R⁶ eine erfindungsgemäße, von Wasserstoff verschiedene Bedeutung aufweist, insbesondere R⁶ Ethoxycarbonyl oder Methoxycarbonyl bedeutet,
einschließlich deren Tautomere, deren Stereoisomere und deren Gemische.

In den erfindungsgemäßen Verfahren weisen die Reste R¹, R², R³, R⁴ und R⁵ vorzugsweise die zuvor als bevorzugt angegebenen Definitionen auf. Ferner bedeutet hierbei R' vorzugsweise H, C₁₋₃-Alkyl oder Benzyl, insbesondere H, Ethyl oder Methyl. Die Reste R^{8a}, R^{8b}, R^{8c} und R^{8d} bedeuten vorzugsweise unabhängig voneinander H, C₁₋₄-Alkylcarbonyl oder Benzyl, insbesondere H, Methylcarbonyl, Ethylcarbonyl oder Benzyl.

Als Zwischenprodukte oder Ausgangsstoffe in der Synthese der erfindungsgemäßen Verbindungen sind auch Verbindungen der allgemeinen Formel IV, insbesondere der allgemeinen Formel IV' 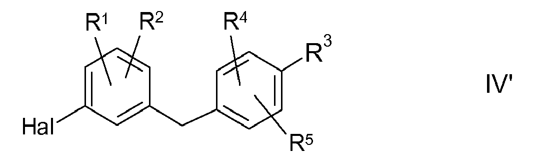 in der Hal Chlor, Brom oder Iod bedeutet und die Reste R¹, R², R⁴ und R⁵ wie zuvor definiert sind und der Rest R³ aus der Gruppe B ausgewählt ist, Gegenstand dieser Erfindung. Besonders bevorzugt weisen hierbei die Reste R¹, R², R³, R⁴ und R⁵ die im Anschluss an die Formeln I.2a bis I.2d angegebenen Definitionen auf. Ganz besonders bevorzugt sind Verbindungen der allgemeinen Formel IV', in der Hal Chlor, Brom oder Iod bedeutet und die Reste R¹ R², R⁴ und R⁵ die im Anschluss an die Formeln I.2a bis I.2d angegebenen Definitionen aufweisen und der Rest R³ Ethinyl oder C₃₋₆-1-Alkin-1-yl bedeutet, wobei die Ethinyl-Gruppe mit dem Rest -SiR₃ substituiert sein kann, wobei die Reste R unabhängig voneinander C₁₋₄-Alkyl, C₁₋₄-Alkoxy oder Aryl bedeuten, und wobei die C₃₋₆-1-Alkin-1-yl-Gruppe mit Hydroxy oder C₁₋₃-Alkoxy, insbesondere Hydroxy oder Methoxy, substituiert sein kann.

Als Zwischenprodukte oder Ausgangsstoffe in der Synthese der erfindungsgemäßen Verbindungen sind auch Verbindungen der allgemeinen Formel II, insbesondere der allgemeinen Formel II' 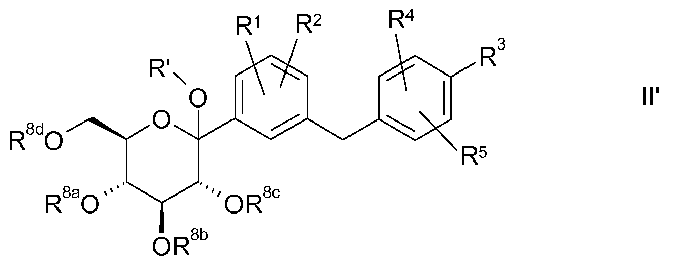 in der R', R^{8a}, R^{8b}, R^{8e}, R^{8d}, R¹, R², R³, R⁴ und R⁵ wie zuvor und nachstehend definiert sind; insbesondere in der R' H, C₁₋₃-Alkyl oder Benzyl, insbesondere H, Ethyl oder Methyl bedeutet; und die Reste R^{8a}, R^{8b}, R^{8e} und R^{$d} unabhängig voneinander H, C₁₋₄-Alkylcarbonyl oder Benzyl, insbesondere H, Methylcarbonyl, Ethylcarbonyl oder Benzyl bedeuten und die Reste R¹, R², R⁴ und R⁵ wie zuvor definiert sind und der Rest R³ aus der Gruppe B ausgewählt ist, Gegenstand dieser Erfindung. Besonders bevorzugt weisen hierbei die Reste R¹, R², R³, R⁴ und R⁵ die im Anschluss an die Formeln I.2a bis I.2d angegebenen Definitionen auf.

Im folgenden werden Begriffe, die zuvor und nachfolgend zur Beschreibung der erfindungsgemäßen Verbindungen verwendet werden, näher definiert.

Die Bezeichnung Halogen bezeichnet ein Atom ausgewählt aus der Gruppe bestehend aus F, Cl, Br und 1, insbesondere F, Cl und Br.

Die Bezeichnung C₁₋ₙ-Alkyl, wobei n einen Wert von 1 bis 18 besitzen kann, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, iso-Hexyl, etc..

Der Begriff C₂₋ₙ-Alkinyl, wobei n einen Wert von 3 bis 6 besitzt, bezeichnet eine verzweigte oder unverzweigte Kohlenwasserstoffgruppe mit 2 bis n C-Atomen und einer C---C-Dreifachbindung. Beispiele solcher Gruppen umfassen Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 1-Hexinyl, 2-Hexinyl, 3-Hexinyl, 4-Hexinyl-, 5-Hexinyl etc.. Sofern nicht anders angegeben sind Alkinyl-Gruppen über das C-Atom in Position 1 mit dem Rest des Moleküls verbunden. Daher sind Bezeichnungen wie 1-Propinyl, 2-Propinyl, 1-Butinyl, etc. gleichbedeutend mit den Bezeichnungen 1-Propin-1-yl, 2-Propin-1-yl, 1-Butin-1-yl, etc.. Dies gilt in anologer Anwendung auch für C₂₋ₙ-Alkenyl-Gruppen.

Der Begriff C₁₋ₙ-Alkoxy oder C₁₋ₙ-Alkyloxy bezeichnet eine C₁₋ₙ-Alkyl-O-Gruppe, worin C₁₋ₙ-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff C₁₋ₙ-Alkylcarbonyl bezeichnet eine C₁₋ₙ-Alkyl-C(=O)-Gruppe, worin C₁₋ₙ-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff C₃₋ₙ-Cycloalkyl bezeichnet eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclodecyl, Bicyclo[3.2.1.]octyl, Spiro[4.5]decyl, Norpinyl, Norbonyl, Norcaryl, Adamantyl, etc.. Vorzugsweise umfasst der Begriff C₃₋₇-Cycloalkyl gesättigte monocyclische Gruppen.

Der Begriff C₅₋ₙ-Cycloalkenyl bezeichnet eine C₅₋ₙ-Cycloalkyl-Gruppe, die wie oben definiert ist und zusätzlich mindestens eine ungesättigte C=C-Doppelbindung hat.

Der Begriff C₃₋ₙ-Cycloalkylcarbonyl bezeichnet eine C₃₋ₙ-Cycloalkyl-C(=O)-Gruppe, worin C₃₋ₙ-Cycloalkyl wie oben definiert ist.

Der Begriff Tri-(C₁₋₄-alkyl)silyl umfasst Silyl-Gruppen, die gleiche oder zwei oder drei verschiedene Alkylgruppen aufweisen.

Der Begriff Di-(C₁₋₃-alkyl)amino umfasst Amino-Gruppen, die gleiche oder zwei verschiedene Alkylgruppen aufweisen.

Die vorstehend und nachfolgend verwendete Schreibweise, bei der in einer Phenyl-gruppe eine Bindung eines Substituenten zur Mitte des Phenylrings hin dargestellt ist, bedeutet, sofern nicht anders angegeben, dass dieser Substituent an jede freie, ein H-Atom tragende Position des Phenylrings gebunden sein kann.

Die erfindungsgemäßen Verbindungen sind unter Anwendung im Prinzip bekannter Syntheseverfahren erhältlich. Bevorzugt werden die Verbindungen nach den nachfolgend näher erläuterten erfindungsgemäßen Herstellungsverfahren erhalten.

Die erfindungsgemäßen Glucose-Derivate der Formel II können aus D-Gluconolacton oder eines Derivats davon durch Addition der gewünschten Benzylbenzol-Verbindung in Form einer Organometallverbindung (Schema 1) aufgebaut werden.

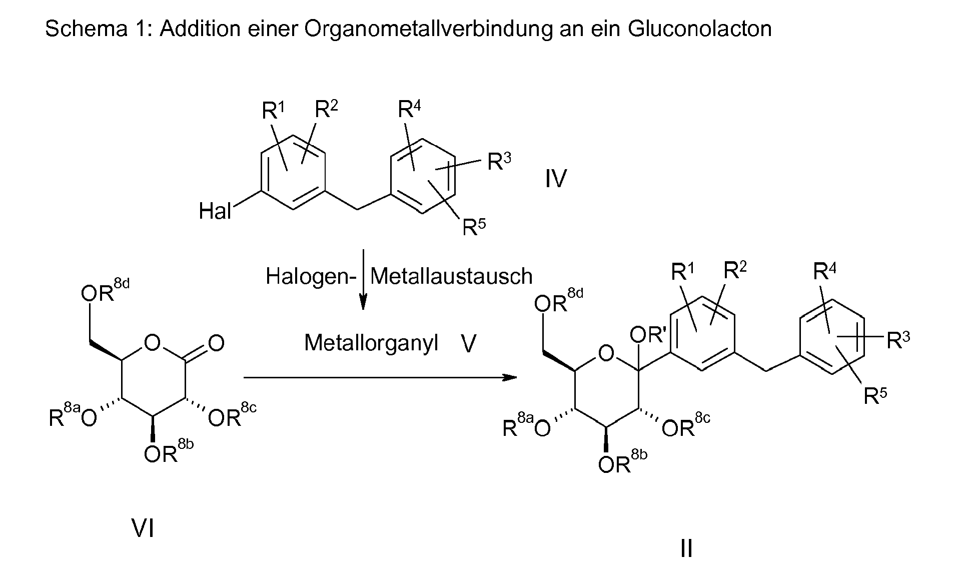

Die Reaktion gemäß Schema 1 wird vorzugsweise ausgehend von einer Halogen-Benzylbenzol-Verbindung der allgemeinen Formel IV, in der Hal Chlor-, Brom- oder Iod bedeutet, durchgeführt. Ausgehend vom Halogenaromaten IV kann die entsprechende Organometallverbindung (V) entweder über einen so genannten Halogen-Metall-Austausch oder über eine Insertion des Metalls in die Kohlenstoff-Halogen-Bindung hergestellt werden. Der Halogen-Metallaustausch mit Brom- oder Iod-substituierten Aromaten kann beispielweise mit einer Organolithiumverbindung wie z.B. n-, sec- oder tert-Butyllithium durchgeführt werden und liefert dabei den entsprechenden lithüerten Aromaten. Die analoge Magnesiumverbindung kann ebenfalls über einen Halogen-Metallaustausch mit einer geeigneten Grignard-Verbindung wie z.B. Isopropylmagnesiumbromid oder Düsopropylmagnesium generiert werden. Die Reaktionen werden vorzugsweise zwischen 0 und -100°C, besonders bevorzugt zwischen -10 und -80°C, in einem inerten Lösungsmittel oder Gemischen daraus, wie beispielsweise Diethylether, Tetrahydrofuran, Toluol, Hexan oder Methylenchlorid, durchgeführt. Die so erhaltenen Magnesium- bzw. Lithium-Verbindungen können gegebenenfalls mit Metallsalzen, wie z.B. Certrichlorid, zu weiteren zur Addition geeigneten Organometallverbindungen (V) ummetalliert werden. Alternativ kann die Organometallverbindung (V) auch durch Insertion eines Metalls in die Kohlenstoff-Halogen-Bindung des Halogenaromaten IV dargestellt werden. Hierzu eignen sich Metalle wie z.B. Lithium oder Magnesium. Die Addition der Organometallverbindung V an das Gluconolacton bzw. Derivaten davon der Formel VI erfolgt vorzugsweise bei Temperaturen zwischen 0 und -100°C, besonders bevorzugt bei -30 bis -80°C, in einem inerten Lösungsmittel oder Gemischen daraus unter Erhalt der Verbindung der Formel II. Die Lithiierungs- und/oder Kupplungsreaktion kann zur Vermeidung tiefer Temperaturen auch in Mikroreaktoren und/oder Mikromischern durchgeführt werden; beispielsweise analog zu den in der WO 2004/076470 beschriebenen Verfahren.

Als Lösungsmittel eignen sich z.B. Diethylether, Toluol, Methylenchlorid, Hexan, Tetrahydrofuran oder Gemische daraus. Die Reaktionen können ohne weitere Hilfsmittel oder im Fall von reaktionsträgen Kupplungspartnern in Gegenwart von Lewis-Säuren wie z.B. BF₃*OEt₂ oder Me₃SiCl durchgeführt werden (siehe M. Schlosser, Organometallics in Synthesis, John Wiley & Sons, Chichester/New York/Brisbane/Toronto/Singapore, 1994). Hierbei bevorzugte Bedeutungen der Gruppen R^{8a}, R^{8b}, R^{8e} und R^{8d} sind Benzyl, substituiertes Benzyl, Trialkylsilyl, besonders bevorzugt Trimethylsilyl, Triisopropylsilyl, 4-Methoxybenzyl und Benzyl. Wenn zwei benachbarte Reste der Gruppe bestehend aus R^{8a}, R^{8b}, R^{8e} und R^{8d} miteinander verknüpft sind, sind diese beiden Reste bevorzugt Bestandteil eines Benzylidenacetals, 4-Methoxybenzylidenacetals, Isopropylketals oder stellen eine 2,3-Dimethoxy-butylengruppe dar, die über die 2 und 3-Position des Butans mit den benachbarten Sauerstoffatomen des Pyranoserings verknüpft ist. Der Rest R' bedeutet vorzugsweise Wasserstoff oder C₁₋₄-Alkyl, besonders bevorzugt Wasserstoff, Methyl oder Ethyl. Der Rest R' wird nach der Addition der metallorganischen Verbindung V oder eines Derivats davon an das Gluconolacton VI eingeführt. Dazu wird die Reaktionslösung mit einem Alkohol wie z.B. Methanol oder Ethanol oder Wasser in Gegenwart einer Säure wie z.B. Methansulfonsäure, Toluolsulfonsäure, Schwefelsäure oder Salzsäure behandelt.

Die Synthese von Halogenaromaten der Formel IV kann unter Anwendung von Standardtransformationen in der Organischen Chemie oder zumindest von aus der Fachliteratur bekannten Methoden in der organischen Synthese durchgeführt werden (siehe u.a. J. March, Advanced Organic Reactions, Reactions, Mechanisms, and Structure, 4. Edition, John Wiley & Sons, Chichester/New York/Brisbane/Toronto/Singapore, 1992 und darin zitierte Literatur). Die nachfolgend dargestellten Synthesestrategien sollen das stellvertretend demonstrieren.

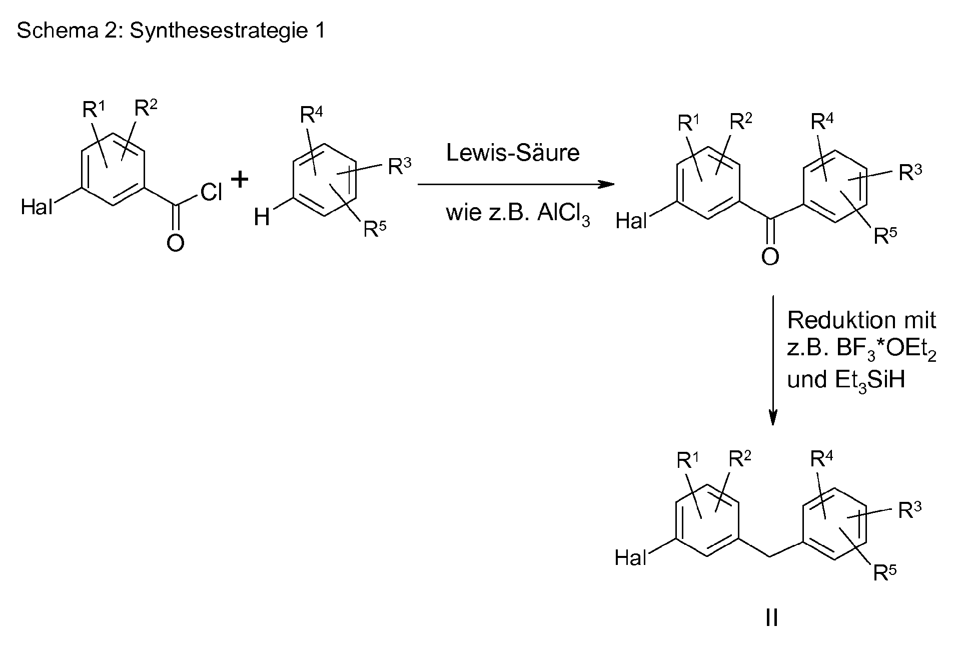

Synthesestrategie 1 (Schema 2) zeigt die Darstellung des Halogenaromaten der Formel II ausgehend von einem Benzoylchlorid und einem zweiten Aromaten, der mittels einer Friedel-Crafts-Acylierung in das Diphenylketonderivat überführt wird. Diese klassische Reaktion hat eine große Substratbreite und wird in Gegenwart eines Katalysators, der katalytisch oder stöchiometrisch eingesetzt wird, wie z.B. AlCl₃, FeCl₃, Iod, Eisen, ZnCl₂, Schwefelsäure oder Trifluormethansulfonsäure durchgeführt. An Stelle des Carbonsäurechlorids können auch die Carbonsäure, ein Anhydrid oder Ester davon oder auch das entsprechende Benzonitril eingesetzt werden. Die Reaktionen werden bevorzugt in chlorierten Kohlenwasserstoffen wie z.B. Dichlormethan und 1,2-Dichlorethan bei Temperaturen von -30 bis 120°C, bevorzugt bei 30 bis 100°C durchgeführt. Aber auch lösungsmittelfreie Umsetzungen oder Umsetzungen in einem Mikrowellenofen sind möglich. In einem zweiten Reaktionsschritt wird das Diphenylketon zum Diphenylmethan reduziert. Diese Reaktion kann zweistufig über das entsprechende Diphenylmethanol oder einstufig ausgeführt werden. In der zweistufigen Variante wird das Keton mit einem Reduktionsmittel wie beispielsweise einem Metallhydrid wie z.B. NaBH₄, LiAlH₄ oder iBu₂AlH zum Alkohol reduziert. Der resultierende Alkohol lässt sich in Gegenwart einer Lewis-Säure wie beispielsweise BF₃*OEt₂, Trifluoressigsäure, InCl₃ oder AlCl₃ mit einem Reduktionsmittel wie z.B. Et₃SiH, NaBH₄, oder Ph₂SiClH zum gewünschten Diphenylmethan umformen. Der einstufige Prozess ausgehend vom Keton zum Diphenylmethan ist z.B. mit einem Silan wie z.B. Et₃SiH, einem Borhydrid wie z.B. NaBH₄ oder einem Aluminiumhydrid wie LiAlH₄ in Gegenwart einer Lewis-Säure wie beispielsweise BF₃*OEt₂, Tris(pentafluorphenyl)boran, Trifluoressigsäure, Aluminiumchlorid oder InCl₃ möglich. Die Reaktionen werden bevorzugt in Lösungsmitteln wie z.B. halogenierten Kohlenwasserstoffen wie Dichlormethan, Toluol oder Acetonitril bei Temperaturen von -30 bis 150°C, bevorzugt bei 20 bis 100°C durchgeführt. Reduktionen mit Wasserstoff in Gegenwart eines Übergangsmetallkatalysators wie z.B. Pd auf Kohle sind ebenfalls ein möglicher Syntheseweg. Reduktionen nach Wolff-Kishner oder Varianten davon sind auch möglich. Dabei wird das Keton mit Hydrazin oder einem Derivat davon, wie z.B. 1,2-Bis(tert-butyldimethylsilyl)hydrazin, zuerst in das Hydrazon überführt, welches unter stark basischen Reaktionsbedingungen und Erwärmen zum Diphenylmethan und Stickstoff zerfällt. Die Reaktion kann in einem Reaktionsschritt oder nach Isolierung des Hydrazons oder eines Derivats davon in zwei separaten Reaktionsschritten durchgeführt werden. Als Basen finden z.B. KOH, NaOH oder KOtBu in Lösungsmitteln wie z.B. Ethylenglycol, Toluol, DMSO, 2-(2-Butoxyethoxy)ethanol oder t-Butanol Anwendung; lösungsmittelfreie Umsetzungen sind ebenfalls möglich. Die Reaktionen können bei Temperaturen zwischen 20 bis 250°C, bevorzugt zwischen 80 bis 200°C durchgeführt werden. Eine Alternative zu den basischen Bedingungen der Wolff-Kishner-Reduktion ist die unter sauren Bedingungen verlaufende Clemmensen-Reduktion, die hier ebenfalls Anwendung finden kann.

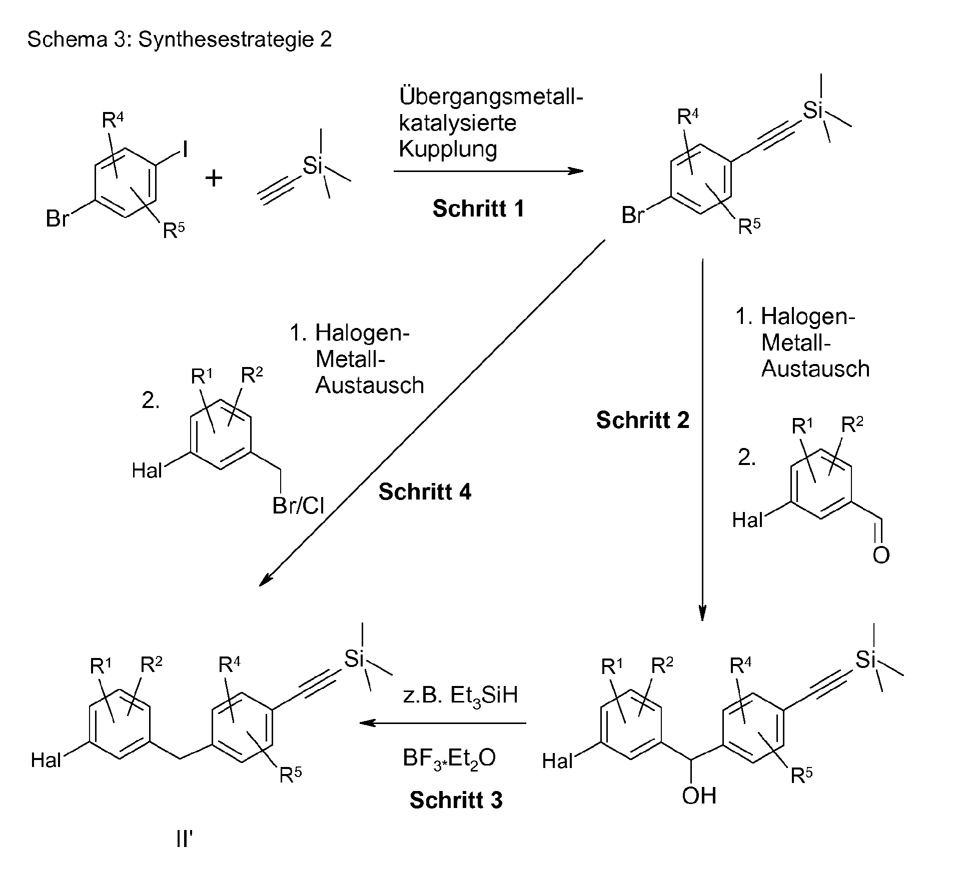

Die zweite Synthesestrategie (Schema 3) zeigt eine weitere Möglichkeit zum Aufbau des Halogen-Aromaten der Formel II' am Beispiel von einem Trimethylsilylacetylen-substituierten Diphenylmethan. Ausgehend von einem Aromaten, der zwei Reste aus der Gruppe Iod, Brom, Chlor oder Sulfonat wie z.B. Trifluormethylsulfonat, trägt, wird über eine Übergangsmetall-katalysierte Monokupplung am reaktiveren Ende des Dihalogenaromaten, der Iod-Kohlenstoff-Bindung, ein Alkinrest angehängt (Schritt 1). Als Katalysatoren werden beispielsweise elementares Palladium oder Nickel oder Salze oder Komplexe daraus eingesetzt. Die Reaktionen können mit dem Alkin selbst oder Metallacetyliden daraus durchgeführt werden. Wenn das Alkin selbst eingesetzt wird, kann die Kupplung in Gegenwart einer Base wie z.B. NEt₃ und eines Co-Katalysators wie z.B. eines Kupfersalzes wie Cul durchgeführt werden (Sonogashira-Kupplung). Die Reaktionen sind nicht auf Trimethylsilylacetylen beschränkt, sondern erlauben die Verwendung einer Vielzahl von terminalen Alkinen. Die Reaktion ist in allen ihren Variationen ausführlich in der Literatur dokumentiert (siehe P.J. Stang, F. Diederich, Metal-Catalyzed Cross-Coupling Reactions, Wiley-VCH, Weinheim, 1997 und Angew. Chem. Int. Ed. 2003, 42, 1566-1568 und dort zitierte Literatur). Die beiden weiteren Stufen zur Herstellung des Diphenylmethan-Derivats beinhalten die Umfunktionalisierung des Alkin-substituierten Aromaten zu einem metallierten (Mg-, Li-) Aromaten, der beispielsweise mittels eines Halogen-Metall-Austausches wie zuvor schon beschrieben, dargestellt werden kann (Schritt 2). Dieser metallierte Aromat, der direkt oder nach einer weiteren Ummetallierung eingesetzt werden kann, wird an ein Benzaldehyd-Derivat addiert. Dabei entsteht das im Schema abgebildete Diphenylmethanol. Alternativ kann auch ein Benzoesäurederivat wie z.B. ein Benzoesäureester, -anhydrid, -chlorid oder auch die Säure selbst oder das Benzonitril eingesetzt werden. Dabei entsteht statt dem Alkohol das entsprechende Keton, welches auch über die oben bereits dargestellte Friedel-Crafts-Acylierung zugänglich ist. Die weitere Umsetzung sowohl des Alkohols als auch des Ketons zum Diphenylmethan-Derivat wurde bereits oben beschrieben (Schritt 3). Der trimethylsilylethinylierte Halogenaromat kann aber auch direkt nach Ummetallierung in das gewünschte Produkt überführt werden (Schritt 4). Dazu setzt man den nach einem Halogen-Metallaustausch erhaltenen Lithium- oder Magnesium-Aromaten mit einem Benzylelektrophil wie z.B. einem Benzylbromid oder -chlorid um. Die Reaktion kann ohne oder besser in Gegenwart eines Übergangsmetallkatalysators, wie z.B. eines Kupfersalzes oder eines Palladiumkomplexes, durchgeführt werden (siehe z.B. Org. Lett. 2001, 3, 2871-2874 und dort zitierte Literatur). Der Lithium- oder Magnesiumaromat kann aber auch zuerst beispielsweise zu den entsprechenden Boronsäuren, Boronsäureestern, Stannanen, Silanen oder Zink-Verbindungen ummetalliert werden. Danach wird dann mittels eines Übergangsmetalles wie z.B. Palladium, Nickel, Rhodium, Kupfer oder Eisen der Benzylrest angeknüpft (siehe L. Brandsma, S.F. Vasilevsky, H.D. Verkruijsse, Application of Transition Metal Catalysts in Organic Synthesis, Springer-Verlag, Berlin/Heidelberg, 1998). Die Umsetzungen des Alkin-substituierten Aromaten zum Zwischenprodukt der Formel II' gemäß der Schritte 2 und 3 oder Schritt 4, die hier beispielhaft für R³ in der Bedeutung Ethinyl bzw. Trimethylsilylethinyl dargestellt sind, können in analoger Anwendung auch mit anderen R³-substituierten Aromaten durchgeführt werden.

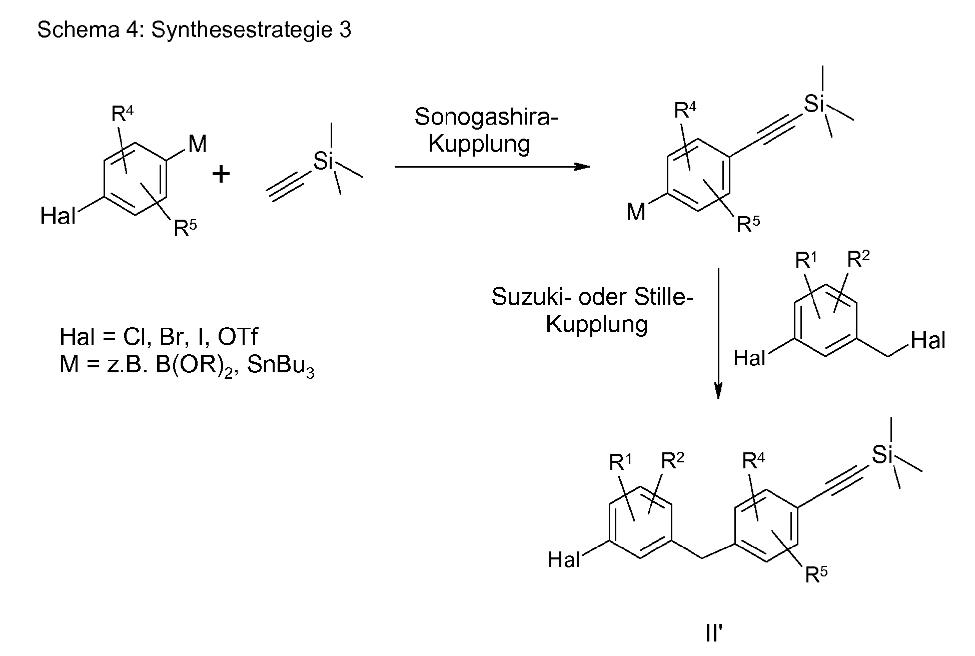

Synthesestrategie 3 (Schema 4) stellt eine Variante zu Synthesestrategie 2 vor, die ebenfalls anhand eines Trimethylsilylethinylaromaten II' verdeutlicht wird, jedoch nicht auf diesen beschränkt sein soll. Die Synthese startet mit einem Aromaten, der sowohl eine Gruppe Hal, die ein Halogenatom Chlor, Brom oder Iod, oder eine Pseudohalogengruppe, wie z.B. Trifluormethansulfonat, bedeutet, als auch ein metallisches Zentrum M, wie z.B. ein B(OH)₂-, Si(OAlk)₃ oder SnBu₃-Rest trägt. Die beiden so "aktivierten" Zentren können nacheinander chemoselektiv ausgetauscht werden. Synthesestrategie 3 verdeutlicht das an einem Beispiel, in dem zuerst das Halogenatom Hal in einer Übergangsmetall-katalysierten Reaktion, wie z.B. der sogenannten Sonogashira-Kupplung, gegen einen Alkin-Substituenten ausgetauscht wird. Im zweiten Schritt wird das metallische Zentrum M in einer weiteren Übergangsmetall-katalysierten Kupplung gegen einen Benzylrest, der z.B. als Benzylhalogenid aktiviert ist, zum gewünschten Produkt ausgetauscht (siehe z.B. Tetrahedron Lett. 2003, 44, 9255-9258 und dort zitierte Literatur). Beide Schritte sind unter Einsatz von Übergangsmetallen, wie z.B. Palladium, Rhodium, Nickel, Kupfer oder Eisen, oder Komplexen dieser durchführbar. Beide Reaktionstypen sind in der Literatur ausführlich beschrieben. Das Vorgehen ist nicht auf das hier Geschilderte beschränkt, sondern kann auch unter Umkehrung der Reihenfolge der beiden Reaktionsschritte durchgeführt werden. Hiernach wird zuerst das metallische Zentrum M mit dem Benzylrest verknüpft und dann die Halogen- oder Pseudohalogengruppe Hal gegen das Alkin ausgetauscht.

Zur Herstellung von Verbindungen der allgemeinen Formel I wird gemäß dem erfindungsgemäßen Verfahren a) eine Verbindung der allgemeinen Formel II 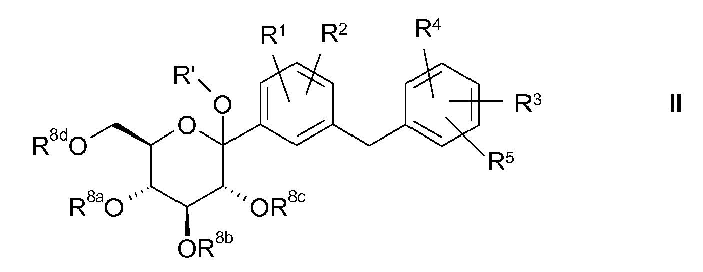 in der R', R¹ bis R⁵ wie zuvor definiert sind und

R^{8a}, R^{8b}, R^{8e}, R^{8d} wie zuvor definiert sind und beispielsweise unabhängig voneinander Acetyl, Pivaloyl, Benzoyl, tert-Butoxycarbonyl, Benzyloxycarbonyl, Trialkylsilyl, Benzyl oder substituiertes Benzyl bedeuten oder jeweils zwei benachbarte Reste R^{8a}, R^{8b}, R^{8e}, R^{8d} ein Benzylidenacetal oder Isopropylidenketal bilden oder eine 2,3-Dimethoxy-butylen-Gruppe, die über die Position 2 und 3 der Butylengruppe mit den Sauerstoffatomen des Pyranoserings verknüpft ist und mit diesen ein substituiertes Dioxan bildet,
die wie zuvor beschrieben erhältlich ist, mit einem Reduktionsmittel in Gegenwart einer Lewis- oder Bronsted-Säure umgesetzt.

Für die Umsetzung eignen sich als Reduktionsmittel beispielsweise Silane, wie Triethyl-, Tripropyl-, Triisopropyl- oder Diphenylsilan, Natriumborhydrid, Natriumcyanoborhydrid, Zinkborhydrid, Boran, Lithiumaluminiumhydrid, Diisobutylaluminiumhydrid oder Samariumiodid. Die Reduktionen finden ohne oder in Gegenwart einer geeigneten Brønsted-Säure, wie z.B. Salzsäure, Toluolsulfonsäure, Trifluoressigsäure oder Essigsäure, oder Lewis-Säure, wie z.B. Bortrifluoridetherat, Trimethylsilyltriflat, Titantetrachlorid, Zinntetrachlorid, Scandiumtriflat oder Zinkiodid statt. In Abhängigkeit vom Reduktionsmittel und der Säure kann die Reaktion in einem Lösungsmittel, wie beispielsweise Methylenchlorid, Chloroform, Acetonitril, Toluol, Hexan, Diethylether, Tetrahydrofuran, Dioxan, Ethanol, Wasser oder Gemischen daraus bei Temperaturen zwischen -60°C und 120°C durchgeführt werden. Ein besonders geeignete Reagenzienkombination besteht beispielsweise aus Triethylsilan und Bortrifluorid-Etherat, die zweckmäßigerweise in Acetonitril oder Dichlormethan bei Temperaturen von -60°C und 60°C zum Einsatz kommt. Des Weiteren kann Wasserstoff in Gegenwart eines Übergangsmetallkatalysators, wie z.B. Palldium auf Kohle oder Raney-Nickel, in Lösungsmitteln wie Tetrahydrofuran, Ethylacetat, Methanol, Ethanol, Wasser oder Essigsäure, für die dargestellte Transformation angewendet werden.

Alternativ werden zur Herstellung von Verbindungen der allgemeinen Formel I gemäß dem erfindungsgemäßen Verfahren b) in einer Verbindung der allgemeinen Formel III all 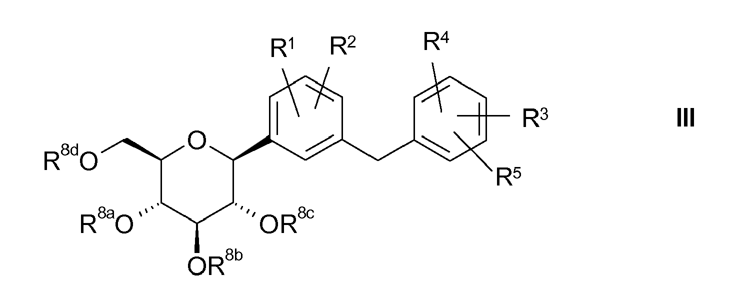
in der R¹ bis R⁵ wie zuvor definiert sind und
R^{8a} bis R^{8d} eine der zuvor definierten Schutzgruppen, wie z.B. eine Acyl-, Arylmethyl-, Acetal-, Ketal- oder Silylgruppe bedeuten, und die beispielsweise aus der Verbindung der Formel II wie zuvor beschrieben durch Reduktion erhältlich ist, die Schutzgruppen abgespalten.

Die Abspaltung eines verwendeten Acyl-Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C. Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung eines verwendeten Acetal- oder Ketal-Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Trimethylsilylrestes erfolgt beispielsweise in Wasser, einem wässrigen Lösemittelgemisch oder einem niederen Alkohol wie Methanol oder Ethanol in Gegenwart einer Base wie Lithiumhydroxid, Natriumhydroxid, Kaliumcarbonat oder Natriummethylat. In wässrigen oder alkoholischen Lösungsmitteln eignen sich ebenfalls Säuren, wie z.B. Salzsäure, Trifluoressigsäure oder Essigsäure. Zur Abspaltung in organischen Lösungsmitteln, wie beispielsweise Diethylether, Tetrahydrofuran oder Dichlormethan, eignen sich auch Fluoridreagenzien, wie z.B. Tetrabutylammoniumfluorid.

Die Abspaltung eines Benzyl-, Methoxybenzyl- oder Benzyloxycarbonylrestes erfolgt vorteilhaft hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig, gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Bei den vorstehend beschriebenen Umsetzungen können gegebenenfalls vorhandene reaktive Gruppen wie Ethinyl-, Hydroxy-, Amino-, Alkylamino- oder Iminogruppen während der Umsetzung durch übliche Schutzgruppen geschützt werden, welche nach der Umsetzung wieder wie u.a. oben beschrieben abgespalten werden.

Beispielsweise kommen als Schutzrest für eine Ethinylgruppe die Trimethylsilyl- oder Triisopropylgruppe in Betracht. Die 2-Hydroxisoprop-2-ylgruppe kann ebenfalls als Schutzgruppe Anwendung finden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Weiterhin können die erhaltenen Verbindungen in Gemische, beispielsweise in 1:1 oder 1:2 Gemische mit Aminosäuren, insbesondere mit alpha-Aminosäuren wie Prolin oder Phenylalanin, übergeführt werden, die besonders günstige Eigenschaften wie hohe Kristallinität aufweisen können.

Die erfindungsgemäßen Verbindungen sind vorteilhaft auch nach den in den nachfolgenden Beispielen beschriebenen Verfahren zugänglich, wobei diese hierzu auch mit dem Fachmann beispielsweise aus der Literatur bekannten Verfahren, insbesondere den in den WO 98/31697, WO 01/27128, WO 02/083066, WO 03/099836 und WO 2004/063209 beschriebenen Verfahren, kombiniert werden können.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf natriumabhängige Glucose-Cotransporter, vorzugsweise SGLT2.

Die biologischen Eigenschaften der neuen Verbindungen können wie folgt geprüft werden:
Die Fähigkeit der Substanzen die SGLT-2 Aktivität zu hemmen, kann in einem Versuchsaufbau gezeigt werden, in dem eine CHO-K1 Zelllinie (ATCC No. CCL 61) oder alternativ eine HEK293 Zelllinie (ATCC No. CRL-1573), die stabil mit einem Expressionsvektor pZeoSV (Invitrogen, EMBL accession number L36849) transfiziert ist, der die cDNA für die kodierende Sequenz des humanen Natrium Glucose Cotransporters 2 (Genbank Acc. No.NM_003041) enthält (CHO-hSGLT2 bzw. HEK-hSGLT2). Diese Zelllinien transportieren Natrium-abhängig ¹⁴C-markiertes alpha-Methyl-Glucopyranosid (¹⁴C-AMG, Amersham) in das Zellinnere.

Der SGLT-2 Assay wird wie folgt durchgeführt:
CHO-hSGLT2 Zellen werden in Ham's F12 Medium (BioWhittaker) mit 10% fötalem Kälberserum und 250 µg/ml Zeocin (Invitrogen), HEK293-hSGLT2 Zellen in DMEM Medium mit 10% fötalem Kälberserum und 250 µg/ml Zeocin (Invitrogen) kultiviert.
Die Zellen werden von den Kulturflaschen durch zweimaliges Waschen mit PBS und anschließende Behandlung mit Trypsin/EDTA abgelöst. Nach Zugabe von Zellkulturmedium werden die Zellen abzentrifugiert, in Kulturmedium resuspendiert und in einem Casy-cellcounter gezählt. Anschließend werden 40.000 Zellen pro Loch in eine weiße, Poly-D-Lysin beschichtete 96-Loch Platte ausgesät und über Nacht bei 37°C, 5% CO₂ inkubiert. Die Zellen werden zweimal mit 300 µl Assaypuffer (Hanks Balanced Salt Solution, 137 mM NaC1, 5,4 mM KCl, 2,8 mM CaCl₂, 1,2 mM MgSO₄ und 10 mM HEPES (pH7,4), 50µg/ml Gentamycin) gewaschen. In jedes Loch werden dann 250 µl Assaypuffer und 5 µl Testverbindung hinzugegeben und für weitere 15 Minuten im Brutschrank inkubiert. Als Negativkontrolle werden 5 µl 10% DMSO eingesetzt. Durch Zugabe von 5 µl ¹⁴C-AMG (0.05 pCi) in jedes Loch wird die Reaktion gestartet. Nach einer 2 stündigen Inkubation bei 37°C, 5% CO₂ werden die Zellen wiederum mit 300 µl PBS (20°C) gewaschen und anschließend durch Zugabe von 25 µl 0.1 N NaOH lysiert (5 min. bei 37°C). Pro Loch werden 200 µl MicroScint20 (Packard) hinzugefügt und für weitere 20 min bei 37°C inkubiert. Nach dieser Inkubation wird die Radioaktivität des aufgenommenen ¹⁴C-AMG in einem Topcount (Packard) mittels eines ¹⁴C-Szintillationsprogramms gemessen.

Zur Bestimmung der Selektivität gegenüber dem humanen SGLT1 wird ein analoger Test aufgebaut, in dem die cDNA für hSGLT1 (Genbank Acc. No. NM000343) statt der hSGLT2 cDNA in CHO-K1 bzw. HEK293 Zellen exprimiert wird.

Die erfindungsgemäßen Verbindungen der allgemeinen Formel I können beispielsweise EC50-Werte unter 1000 nM, insbesondere unter 200 nM, besonders bevorzugt unter 50 nM aufweisen.

Im Hinblick auf die Fähigkeit, die SGLT Aktivität zu hemmen, sind die erfindungsgemäßen Verbindungen der allgemeinen Formel I und ihre entsprechenden pharmazeutisch akzeptablen Salze prinzipiell geeignet, alle diejenigen Zustände oder Krankheiten zu behandeln und/oder vorbeugend zu behandeln, die durch eine Hemmung der SGLT Aktivität, insbesondere der SGLT-2 Aktivität beeinflusst werden können. Daher sind erfindungsgemäße Verbindungen insbesondere zur Prophylaxe oder Behandlung von Krankheiten, insbesondere Stoffwechselerkrankungen, oder Zuständen wie Diabetes mellitus Typ 1 und Typ 2, diabetische Komplikationen (wie z.B. Retinopathie, Nephropathie oder Neuropathien, diabetischer Fuß, Ulcus, Makroangiopathien), metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie geeignet. Darüber hinaus sind diese Substanzen geeignet, die beta-Zelldegeneration wie z.B. Apoptose oder Nekrose von pankreatischen beta-Zellen zu verhindern. Die Substanzen sind weiter geeignet, die Funktionalität von pankreatischen Zellen zu verbessern oder wiederherzustellen, daneben die Anzahl und Größe von pankreatischen beta-Zellen zu erhöhen. Die erfindungsgemäßen Verbindungen sind ebenfalls als Diuretika oder Antihypertensiva einsetzbar und zur Prophylaxe und Behandlung des akuten Nierenversagens geeignet.

Ganz besonders sind die erfindungsgemäßen Verbindungen, einschließlich deren physiologisch verträglichen Salze, zur Prophylaxe oder Behandlung von Diabetes, insbesondere Diabetes mellitus Typ 1 und Typ 2, und/oder diabetischen Komplikationen geeignet.

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei intravenöser Gabe im Bereich von 1 bis 100 mg, vorzugsweise 1 bis 30 mg, und bei oraler Gabe im Bereich von 1 bis 1000 mg, vorzugsweise 1 bis 100 mg, jeweils 1 bis 4 x täglich, liegen. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Lösungen, Suspensionen oder Zäpfchen einarbeiten.

Die erfindungsgemäßen Verbindungen können auch in Kombination mit anderen Wirkstoffen, insbesondere zur Behandlung und/oder Prophylaxe der zuvor angegebenen Krankheiten und Zustände verwendet werden. Für solche Kombinationen kommen als weitere Wirksubstanzen insbesondere solche in Betracht, die beispielsweise die therapeutische Wirksamkeit eines erfindungsgemäßen SGLT-Hemmers im Hinblick auf eine der genannten Indikationen verstärken und/oder die eine Reduzierung der Dosierung eines erfindungsgemäßen SGLT-Hemmers erlauben. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), alpha-Glucosidasehemmer (z.B. Acarbose, Voglibose), DPPIV Inhibitoren (z.B. LAF237, MK-431), alpha2-Antagonisten, Insulin und Insulinanaloga, GLP-1 und GLP-1 Analoga (z.B. Exendin-4) oder Amylin. Daneben sind weitere als Kombinationspartner geeignete Wirkstoffe Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannabinoid1 Rezeptors, MCH-1 Rezeptorantagonisten, MC4 Rezeptor Agonisten, NPY5 oder NPY2 Antagonisten oder β3-Agonisten wie SB-418790 oder AD-9677 ebenso wie Agonisten des 5HT2c Rezeptors.

Daneben ist eine Kombination mit Medikamenten zur Beeinflussung des Bluthochdrucks, des chronischen Herzversagens oder der Atherosklerose wie z.B. A-II Antagonisten oder ACE Inhibitoren, ECE-Inhibitoren, Diuretika, β-Blocker, Ca-Antagonisten, zentral wirksamen Antihypertensiva, Antagonisten des alpha-2-adrenergen Rezeptors, Inhibitoren der neutralen Endopeptidase, Thrombozytenaggregationshemmer und anderen oder Kombinationen daraus geeignet. Beispiele von Angiotensin II Rezeptor Antagonisten sind Candesartan Cilexetil, Kalium Losartan, Eprosartan Mesylat, Valsartan, Telmisartan, Irbesartan, EXP-3174, L-158809, EXP-3312, Olmesartan, Medoxomil, Tasosartan, KT-3-671, GA-0113, RU-64276, EMD-90423, BR-9701, etc.. Angiotensin II Rezeptor Antagonisten werden vorzugsweise zur Behandlung oder Prophylaxe von Bluthochdruck und diabetischen Komplikationen verwendet, oft in Kombination mit einem Diuretikum wie Hydrochlorothiazide.

Zur Behandlung oder Prophylaxe der Gicht ist eine Kombination mit Harnsäuresynthese Inhibitoren oder Urikosurika geeignet.

Zur Behandlung oder Prophylaxe diabetischer Komplikationen kann eine Kombination mit GABA-Rezeptor-Antagonisten, Na-Kanal-Blockern, Topiramat, Protein-Kinase C Inhibitoren, advanced glycation endproduct Inhibitoren oder Aldose Reduktase Inhibitoren erfolgen.

Die Dosis für die zuvor angeführten Kombinationspartner beträgt hierbei zweckmäßigerweise 1/5 der üblicherweise empfohlenen niedrigsten Dosierung bis zu 1/1 der normalerweise empfohlenen Dosierung.

Daher betrifft ein weiterer Gegenstand dieser Erfindung die Verwendung einer erfindungsgemäßen Verbindung oder eines physiologisch verträglichen Salzes solch einer Verbindung in Kombination mit mindestens einem der zuvor als Kombinationspartner beschriebenen Wirkstoffe zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind. Hierbei handelt es sich vorzugsweise um eine Stoffwechselerkrankung, insbesondere eine der zuvor angeführten Erkrankungen oder Zustände, ganz besonders Diabetes oder diabetischer Komplikationen.

Die Verwendung der erfindungsgemäßen Verbindung, oder eines physiologisch verträglichen Salzes hiervon, in Kombination mit einem weiteren Wirkstoff kann zeitgleich oder zeitlich versetzt, insbesondere aber zeitnah erfolgen. Bei einer zeitgleichen Verwendung werden beide Wirkstoffe dem Patienten zusammen verabreicht; bei einer zeitlich versetzten Verwendung werden beide Wirkstoffe dem Patienten in einem Zeitraum von kleiner gleich 12, insbesondere kleiner gleich 6 Stunden nacheinander verabreicht.

Folglich betrifft ein weiterer Gegenstand dieser Erfindung ein Arzneimittel, das eine erfindungsgemäße Verbindung oder ein physiologisch verträgliches Salz solch einer Verbindung sowie mindestens einen der zuvor als Kombinationspartner beschriebenen Wirkstoffe neben gegebenenfalls einem oder mehreren inerten Trägerstoffen und/oder Verdünnungsmitteln aufweist.

So weist beispielsweise ein erfindungsgemäßes Arzneimittel eine Kombination aus einer erfindungsgemäßen Verbindung der Formel I oder eines physiologisch verträglichen Salzes solch einer Verbindung sowie mindestens einem Angiotensin II Rezeptor Antagonisten neben gegebenenfalls einem oder mehreren inerten Trägerstoffen und/oder Verdünnungsmitteln auf.

Die erfindungsgemäße Verbindung, oder eines physiologisch verträglichen Salzes, und der damit zu kombinierende weitere Wirkstoff können zusammen in einer Darreichungsform, beispielsweise einer Tablette oder Kapsel, oder getrennt in zwei gleichen oder verschiedenen Darreichungsformen, beispielsweise als sogenanntes kit-of-parts, vorliegen.

Vorstehend und nachfolgend werden in Strukturformeln H-Atome von Hydroxylgruppen nicht in jedem Fall explizit dargestellt. Die nachfolgenden Beispiele sollen die vorliegende Erfindung näher erläutern ohne diese zu beschränken:

### Herstellung der Ausgangsverbindungen:

### Beispiel I

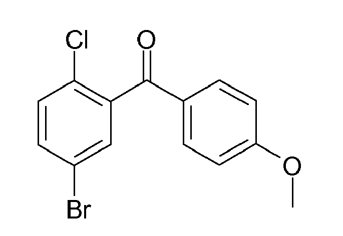

### (5-Brom-2-chlor-phenyl)-(4-methoxy-phenyl)-methanon

Zu einer Mischung von 100 g 5-Brom-2-chlor-benzoesäure in 500 ml Dichlormethan werden 38,3 ml Oxalylchlorid und 0,8 ml Dimethylformamid gegeben. Das Reaktionsgemisch wird 14 h gerührt, danach filtriert und von allen flüchtigen Bestandteilen im Rotationsverdampfer getrennt. Der Rückstand wird in 150 ml Dichlormethan gelöst, die Lösung auf -5°C abgekühlt, und es werden 46,5 g Anisol zugegeben. Danach werden 51,5 g Aluminiumtrichlorid portionsweise so zugegeben, dass die Temperatur nicht über 5°C steigt. Die Lösung wird noch 1 h bei 1-5°C gerührt und anschließend auf Eis gegossen. Die organische Phase wird abgetrennt und die wässrige noch drei Mal mit Dichlormethan extrahiert. Die vereinigten organischen Phasen werden mit wässriger 1 M Salzsäure, zwei Mal mit 1 M Natronlauge und mit gesättigter Natriumchlorid-Lösung gewaschen. Danach wird die organische Phase getrocknet, das Lösungsmittel entfernt und der Rückstand in Ethanol umkristallisiert.
Ausbeute: 86,3 g (64% der Theorie)
Massenspektrum (ESI⁺): m/z = 325/327/329 (Br+Cl) [M+H]⁺

Analog Beispiel I werden folgende Verbindungen erhalten:
(1) (5-Brom-2-iod-phenyl)-(4-ethoxy-phenyl)-methanon 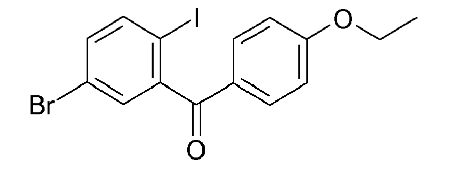 Massenspektrum (ESI⁺): m/z = 431/433 (Br) [M+H]⁺
(2) (5-Brom-2-chlor-phenyl)-(4-iod-phenyl)-methanon

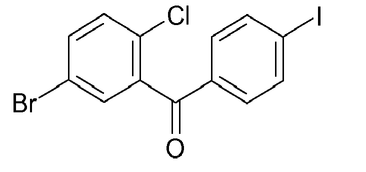

### Beispiel II

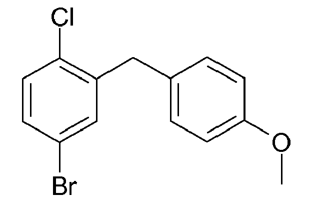

### 4-Brom-1-chlor-2-(4-methoxy-benzyl)-benzol

Eine Lösung von 86,2 g (5-Brom-2-chlor-phenyl)-(4-methoxy-phenyl)-methanon und 101,5 ml Triethylsilan in 75 ml Dichlormethan und 150 ml Acetonitril wird auf 10°C abgekühlt. Dann werden unter Rühren 50,8 ml Bortrifluoridetherat so zugegeben, dass die Temperatur nicht über 20°C steigt. Die Lösung wird 14 h bei Raumtemperatur gerührt, bevor noch einmal 9 ml Triethylsilan und 4,4 ml Bortrifluoridetherat zugegeben werden. Die Lösung wird weitere 3 h bei 45-50°C gerührt und dann auf Raumtemperatur abgekühlt. Es wird eine Lösung von 28 g Kaliumhydroxid in 70 ml Wasser zugesetzt und 2 h gerührt. Danach wird die organische Phase abgetrennt und die wässrige noch drei Mal mit Diisopropylether extrahiert. Die vereinten organischen Phasen werden zwei Mal mit 2 M Kalilauge und einmal mit wässriger Natriumchlorid-Lösung gewaschen und anschließend über Natriumsulfat getrocknet. Nach Entfernen des Lösungsmittels wird der Rückstand in Ethanol verrührt, wieder abgetrennt und bei 60°C getrocknet.
Ausbeute: 50,0 g (61 % der Theorie)
Massenspektrum (ESI⁺): m/z = 310/312/314 (Br+Cl) [M+H]⁺

Analog Beispiel II werden folgende Verbindungen erhalten:
(1) 4-Brom-1-iod-2-(4-ethoxy-benzyl)-benzol 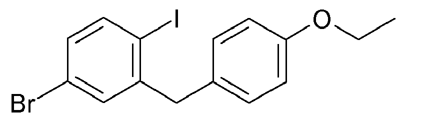 Massenspektrum (ESI⁺): m/z=434/436 [M+NH₄]⁺
(2) 4-Brom-1-chlor-2-(4-iod-benzyl)-benzol

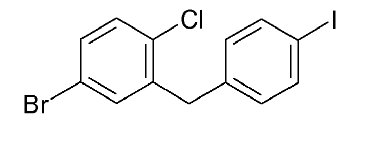

### Beispiel III

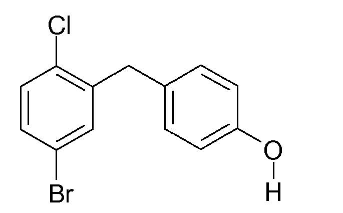

### 4-(5-Brom-2-chlor-benzyl)-phenol

Eine Lösung von 14,8 g 4-Brom-1-chlor-2-(4-methoxy-benzyl)-benzol in 150 ml Dichlormethan wird im Eisbad abgekühlt. Dann werden 50 ml einer 1 M Lösung von Bortribromid in Dichlormethan zugegeben, und die Lösung wird 2 h bei Raumtemperatur gerührt. Die Lösung wird danach wieder im Eisbad gekühlt, und es wird gesättigte Kaliumcarbonat-Lösung zugetropft. Bei Raumtemperatur wird mit wässriger 1 M Salzsäure auf einen pH-Wert von 1 eingestellt, die organische Phase abgetrennt und die wässrige noch drei Mal mit Ethylacetat extrahiert. Die vereinten organischen Phasen werden über Natriumsulfat getrocknet, und das Lösungsmittel wird vollständig entfernt.
Ausbeute: 13,9 g (98% der Theorie)
Massenspektrum (ESI⁻): m/z = 295/297/299 (Br+Cl) [M-H]⁻

### Beispiel IV

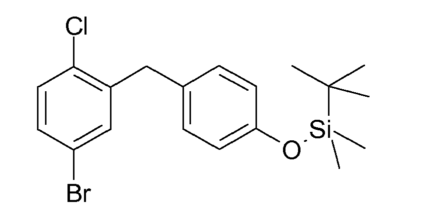

### [4-(5-Brom-2-chlor-benzyl)-phenoxy]-tert-butyl-dimethyl-silan

Eine Lösung von 13,9 g 4-(5-Brom-2-chlor-benzyl)-phenol in 140 ml Dichlormethan wird im Eisbad abgekühlt. Dann werden 7,54 g tert-Butyldimethylsilylchlorid in 20 ml Dichlormethan gefolgt von 9,8 ml Triethylamin und 0,5 g Dimethylaminopyridin zugegeben. Die Lösung wird 16 h bei Raumtemperatur gerührt und dann mit 100 ml Dichlormethan verdünnt. Die organische Phase wird zwei Mal mit wässriger 1 M Salzsäure und einmal mit wässriger Natriumhydrogencarbonatlösung gewaschen und anschließend über Natriumsulfat getrocknet. Nach Entfernen des Lösungsmittels wird der Rückstand über Kieselgel filtriert (Cyclohexan/Ethylacetat 100:1).
Ausbeute: 16,8 g (87% der Theorie)
Massenspektrum (EI): m/z = 410/412/414 (Br+Cl) [M]⁺

### Beispiel V

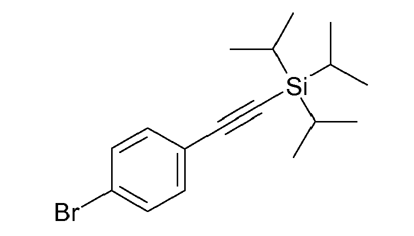

### 1-Brom-4-triisopropylsilylethinyl-benzol

Unter Argon werden zu einer sauerstofffreien Lösung von 15,0 g 1-Brom-4-iod-benzol in 150 ml trockenem Tetrahydrofuran 11,6 ml Triisopropylacetylen und 14,4 ml Triethylamin gefolgt von 0,2 g Kupferiodid und 0,73 g Bis-(triphenylphosphin)-palladiumdichlorid gegeben. Die Lösung wird 16 h bei Raumtemperatur gerührt und dann über Celite filtriert und eingeengt. Der Rückstand wird über Kieselgel chromatografiert (Cyclohexan).
Ausbeute: 17,4 g (100% der Theorie)
Massenspektrum (ESI⁺): m/z = 336/338 (Br) [M]⁺

Analog Beispiel V werden folgende Verbindungen erhalten:
(1) 4-Brom-1-(triisopropylsilylethinyl)-2-(4-ethoxy-benzyl)-benzol 4-Brom-1-iod-2-(4-ethoxy-benzyl)-benzol wird hierbei als Ausgangsmaterial für die zuvor beschriebene Kupplungsreaktion verwendet. 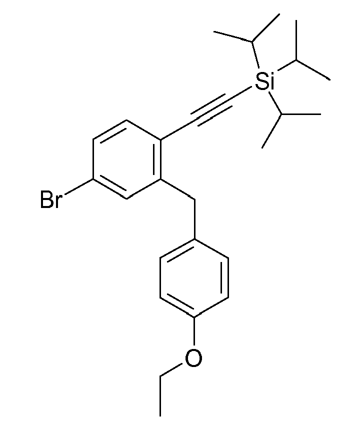 Massenspektrum (ESI⁺): m/z = 471/473 (Br) [M+H]⁺
(2) [4-(5-Brom-2-chlor-benzyl)-phenylethinyl]-triisopropyl-silan 4-Brom-1-chlor-2-(4-iod-benzyl)-benzol wird als Ausgangsmaterial verwendet.

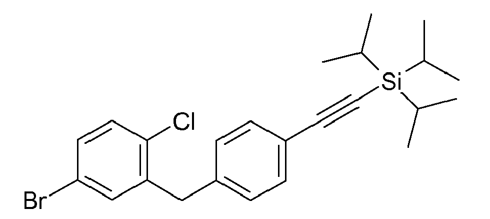

Diese Verbindung kann auch gemäß Beispiel X erhalten werden.

### Beispiel VI

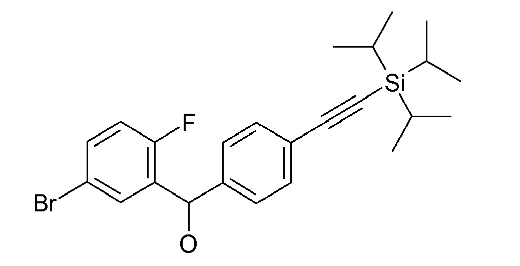

### (5-Brom-2-fluor-phenyl)-{4-[(triisopropylsilyl)-ethinyl]-phenyl}-methanol

Zu einer -78°C-kalten Lösung von 17,4 g 1-Brom-4-triisopropylsilylethinyl-benzol in 120 ml trockenem Tetrahydrofuran werden unter Argon 33,8 ml einer 1,6 M Lösung von n-Butyllithium in Hexan getropft. Die Lösung 1 h bei -70°C gerührt. Danach werden 10,8 g 5-Brom-2-fluor-benzaldehyd in 30 ml Tetrahydrofuran gelöst über 15 min zugetropft. Die resultierende Lösung wird über Nacht im Kühlbad auf Raumtemperatur erwärmen gelassen. Danach wird Wasser zugegeben und mit Ethylacetat extrahiert. Die vereinigten organischen Phase werden über Natriumsulfat getrocknet, und das Lösungsmittel wird entfernt. Der Rückstand wird über Kieselgel (Cyclohexan/Ethylacetat 4:1) gereinigt.
Ausbeute: 14,3 g (60% der Theorie)
Massenspektrum (ESI⁺): m/z = 461/463 (Br) [M+H]⁺

Analog Beispiel VI werden folgende Verbindungen erhalten:
(1) (3-Brom-phenyl)-{4-[(triisopropylsilyl)-ethinyl]-phenyl}-methanol 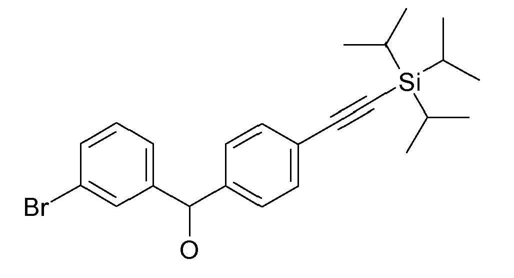 Massenspektrum (ESI⁻): m/z = 487/489 (Br) [M+HCOO]⁻
(2) (5-Brom-2-methoxy-phenyl)-{4-[(triisopropylsilyl)-ethinyl]-phenyl}-methanol 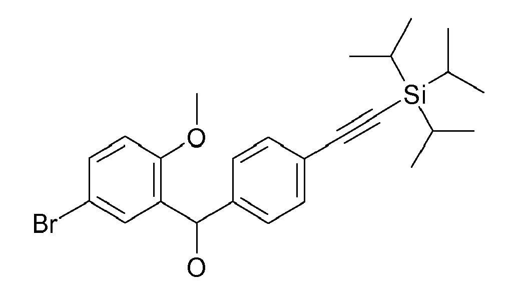 Massenspektrum (ESI⁺): m/z = 473/475 (Br) [M+H]⁺

### Beispiel VII

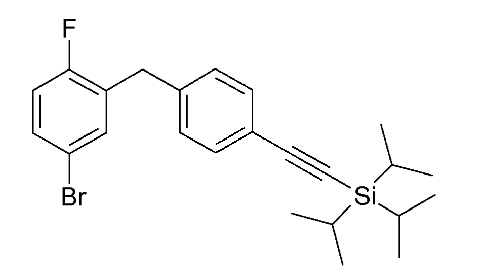

### [4-(5-Brom-2-fluor-benzyl)-phenylethinyl]-triisopropyl-silan

Eine Lösung von 5,6 g (5-Brom-2-fluor-phenyl)-{4-[(triisopropylsilyl)-ethinyl]-phenyl}-methanol und 4,1 ml Triethylsilan in 50 ml Dichlormethan wird im Eisbad abgekühlt. Dann werden 4,7 ml Trifluoressigsäure langsam zugetropft, und die Lösung wird 4 h bei Raumtemperatur gerührt. Die Lösung wird mit Dichlormethan verdünnt und mit wässriger Natriumhydrogencarbonatlösung gewaschen. Nach Trocknen über Natriumsulfat wird das Lösungsmittels entfernt und der Rückstand über Kieselgel gereinigt (Cyclohexan).
Ausbeute: 2,6 g (48% der Theorie)
Massenspektrum (EI): m/z = 445/447 (Br) [M]⁺

Analog Beispiel VII werden folgende Verbindungen erhalten:
(1) [4-(3-Brom-benzyl)-phenylethinyl]-triisopropyl-silan 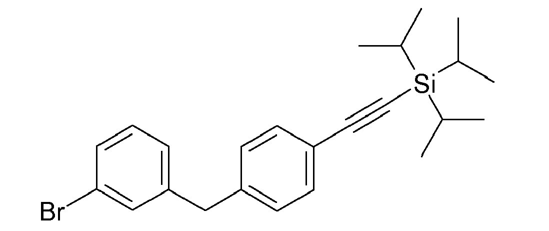 Massenspektrum (ESI⁺): m/z = 427/429 (Br) [M+H] ⁺
(2) [4-(5-Brom-2-methoxy-benzyl)-phenylethinyl]-triisopropyl-silan

Abweichend vom zuvor beschriebenen Verfahren wird hier die Reaktionslösung im Eisbad statt bei Raumtemperatur bis zur vollständigen Umsetzung gerührt. 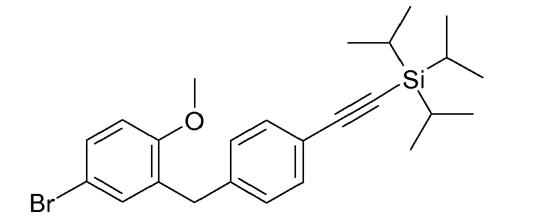 Massenspektrum (ESI⁺): m/z = 457/459 (Br) [M+H] ⁺

### Beispiel VIII

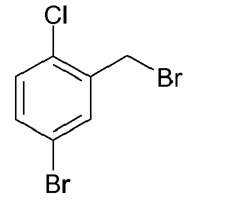

### 4-Brom-2-brommethyl-1-chlor-benzol

Zu einer 5°C-kalten Lösung von 5,0 g 4-Brom-1-chlor-2-hydroxymethyl-benzol und 5,9 g Triphenylphosphin in 50 ml Tetrahydrofuran werden langsam 4,0 g N-Bromsuccinimid gegeben. Nach 1 h Rühren bei Raumtemperatur wird der Niederschlag abfiltriert und das Lösungsmittel im Vakuum entfernt. Der Rückstand wird über Kieselgel gereinigt (Cyclohexan/Ethylacetat 50:1).
Ausbeute: 4,9 g (76% der Theorie)
Massenspektrum (EI): m/z = 282/284/286 (Br+Cl) [M]⁺

### Beispiel IX

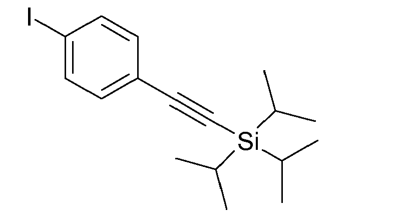

### (4-Iod-phenylethinyl)-triisopropyl-silan

Unter Argon werden zu einer Lösung von 20,0 g (4-Brom-phenylethinyl)-triisopropyl-silan 18,0 g Natriumiodid (trocken), 0,6 g Kupferiodid und 0,8 g N,N'-Dimethyl-cyclohexan-1,2-diamin gegeben. Die Lösung wird 24 h unter Rückfluss gerührt und dann auf Raumtemperatur abgekühlt. Es wird 1 %ige Ammoniaklösung (100 ml) zugegeben und mit Ethylacetat extrahiert. Nach Trocknen über Natriumsulfat wird das Lösungsmitttel entfernt und der Rückstand über Kieselgel gereinigt (Cyclohexan).
Ausbeute: 21,0 g (92% der Theorie)
Massenspektrum (EI): m/z = 384 [M]⁺

### Beispiel X

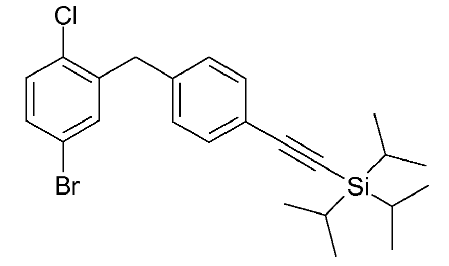

### [4-(5-Brom-2-chlor-benzyl)-phenylethinyl]-triisopropyl-silan

Unter Argon werden zu einer -25°C-kalten Lösung von 0,50 g (4-Iod-phenylethinyl)-triisopropyl-silan in 2,2 ml trockenem Tetrahydrofuran 0,66 ml einer 2 M Lösung von Isopropylmagnesiumchlorid in Tetrahydrofuran getropft. Die Lösung wird 30 min bei -25°C gerührt und dann mit 0,26 ml einer 1 M Lösung von CuCN*2 LiCl in Tetrahydrofuran (hergestellt durch Lösen von CuCN und LiCl im Verhältnis 1:2) versetzt. Kurz danach werden 0,35 g 4-Brom-2-brommethyl-1-chlorbenzol zugegeben und das Reaktionsgemisch im Kühlbad auf -5°C erwärmt. Nach 6 h Rühren bei -5°C wird die Lösung auf Raumtemperatur erwärmt und über Nacht gerührt. Danach wird eine Mischung aus gesättigter Ammoniumchloridlösung und 25%iger Ammoniaklösung (9:1) zugegeben und das resultierende Gemisch zu Wasser gegeben. Die organische Phase wird abgetrennt und die wässrige mit Ethylacetat extrahiert, die vereinten organischen Phasen werden über Natriumsulfat getrocknet, und das Lösungsmittel wird entfernt. Der Rückstand wird über Kieselgel gereinigt (Cyclohexan).
Ausbeute: 0,28 g (50% der Theorie)
Massenspektrum (EI): m/z = 461/463/465 (Br+Cl) [M+H]⁺

### Beispiel XI

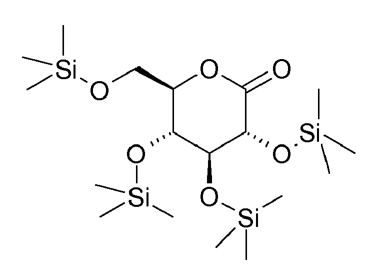

### 2,3,4,6-Tetrakis-O-(trimethylsilyl)-D-glucopyranon

Eine Lösung von 20 g D-Glucono-1,5-lacton und 98,5 ml N-Methylmorpholin in 200 ml Tetrahydrofuran wird auf -5°C abgekühlt. Dann werden 85 ml Trimethylsilylchlorid so zugetropft, dass die Temperatur nicht über 5°C steigt. Die Lösung wird danach 1 h bei Raumtemperatur, 5 h bei 35°C und noch einmal 14 h bei Raumtemperatur gerührt. Nach Zugabe von 300 ml Toluol wird die Lösung im Eisbad abgekühlt, und es werden 500 ml Wasser so zugegeben, dass die Temperatur nicht über 10°C steigt. Die organische Phase wird anschließend abgetrennt und jeweils einmal mit wässriger Natriumdihydrogenphosphatlösung, Wasser und gesättigter wässriger Natriumchloridlösung gewaschen. Das Lösungsmittel wird entfernt, der Rückstand in 250 ml Toluol aufgenommen und das Lösungsmittel erneut vollständig entfernt.
Ausbeute: 52,5 g (ca. 90% rein)
Massenspektrum (ESI⁺): m/z = 467 [M+H]⁺

### Beispiel XII

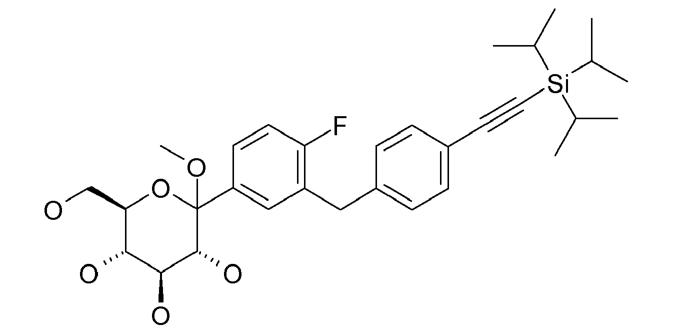

### 1-Fluor-4-(1-methoxy-D-glucopyranos-1-yl)-2-(4-triisopropylsilylethinyl-benzyl)-benzol

Eine Lösung von 4,46 g [4-(5-Brom-2-fluor-benzyl)-phenylethinyl]-triisopropyl-silan in 30 ml trockenem Diethylether wird unter Argon auf -80°C abgekühlt. Zu der gekühlten Lösung werden langsam 11,8 ml einer 1,7 M Lösung von tert-Butyllithium in Pentan getropft, und dann wird die Lösung 45 min bei -80°C gerührt. Zu dieser Lösung wird nun über eine Umdrücknadel eine -80°C-kalte Lösung von 5,19 g 2,3,4,6-Tetrakis-O-(trimethylsilyl)-D-glucopyranon in 50 ml Diethylether getropft. Die resultierende Lösung wird 3 h bei -78°C gerührt. Danach wird eine Lösung von 1,7 ml Methansulfonsäure in 50 ml Methanol zugegeben, das Kühlbad entfernt und die Lösung 16 h bei Raumtemperatur gerührt. Die Lösung wird anschließend mit Ethyldiisopropylamin neutralisiert und bis zur Trockene eingeengt. Der Rückstand wird über Kieselgel gereinigt (Dichlormethan/Methanol 50:1->4:1).
Ausbeute: 2,8 g (50% der Theorie)
Massenspektrum (ESI⁺): m/z = 576 [M+NH₄]⁺

Analog Beispiel XII werden folgende Verbindungen erhalten:
(1) 1-Methoxy-4-(1-methoxy-D-glucopyranos-1-yl)-2-(4-triisopropylsilylethinyl-benzyl)-benzol Vorteilhaft wird hierbei das Reaktionsgemisch nur mit einem geringen Überschuss an Methansulfonsäure versetzt. 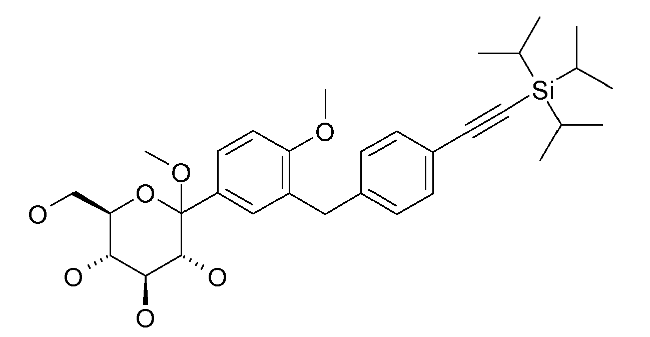 Massenspektrum (ESI⁺): m/z = 588 [M+NH₄]⁺
(2) 1-Chlor-4-(1-methoxy-D-glucopyranos-1-yl)-2-(4-triisopropylsilylethinyl-benzyl)-benzol 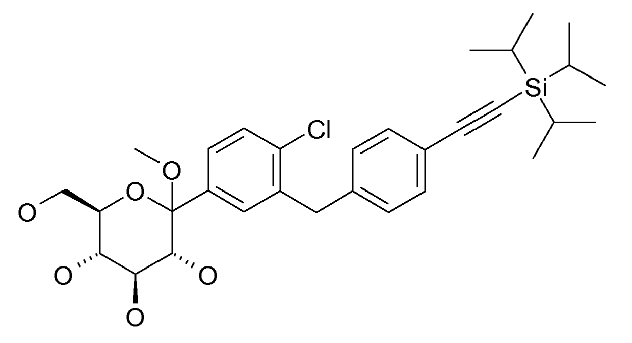 Massenspektrum (ESI⁺): m/z = 592/594 (CI) [M+NH₄]⁺

### Beispiel XIII

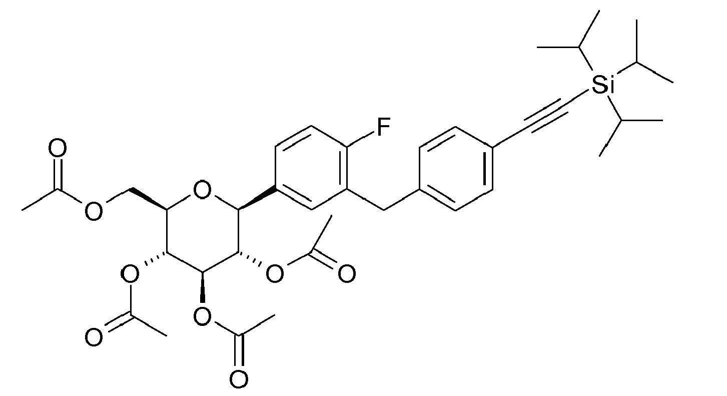

### 1-Fluor-4-(2,3,4,6-tetra-O-acetyl-β-D-glucopyranos-1-yl)-2-(4-triisopropylsilylethinyl-benzyl)-benzol

Eine Lösung von 0,8 g 1-Fluor-4-(1-methoxy-D-glucopyranos-1-yl)-2-(4-triisopropylsilylethinyl-benzyl)-benzol und 0,5 ml Triethylsilan in 6 ml Dichlormethan und 10 ml Acetonitril wird auf -10°C abgekühlt. Zu der gekühlten Lösung werden 0,27 ml Bortrifluoridetherat getropft. Die Lösung wird dann 3 h im Eisbad gerührt. Zu der Lösung wird wässrige Natriumhydrogencarbonatlösung gegeben und dann mit Ethylacetat extrahiert. Die organische Phase wird über Natriumsulfat getrocknet, das Lösungsmittel entfernt und der Rückstand in 6 ml Dichlormethan aufgenommen. Dann werden 1,2 ml Pyridin, 1,3 ml Essigsäureanhydrid und 8 mg 4-Dimethylaminopyridin zugegeben. Die Lösung wird 1 h bei Raumtemperatur gerührt und dann mit Wasser versetzt. Das Gemisch wird mit Dichlormethan extrahiert, die organische Phase mit 1 M Salzsäure gewaschen und über Natriumsulfat getrocknet. Nach Entfernen des Lösungsmittels wird der Rückstand über Kieselgel chromatografiert (Cyclohexan/Ethylacetat 4:1->1:1).
Ausbeute: 0,23 g (23% der Theorie)
Massenspektrum (ESI⁺): m/z = 714 [M+NH₄]⁺

Analog Beispiel XIII werden folgende Verbindungen erhalten:
(1) 1-Methoxy-4-(2,3,4,6-tetra-O-acetyl-β-D-glucopyranos-1-yl)-2-(4-triisopropylsilylethinylbenzyl)-benzol 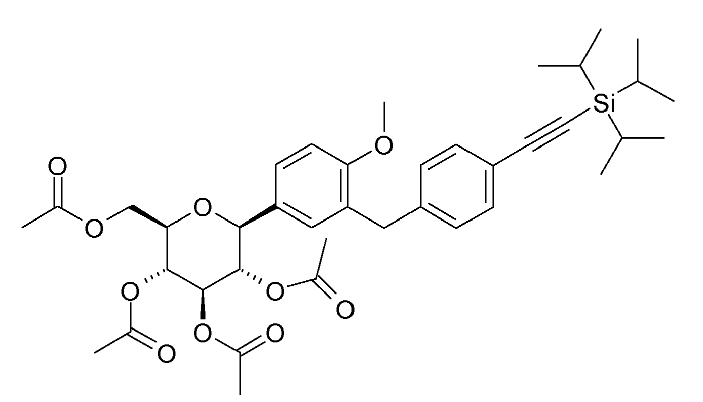 Massenspektrum (ESI⁺): m/z = 726 [M+NH₄]⁺
(2) 1-Chlor-4-(2,3,4,6-tetra-O-acetyl-β-D-glucopyranos-1-yl)-2-(4-triisopropylsilylethinylbenzyl)-benzol 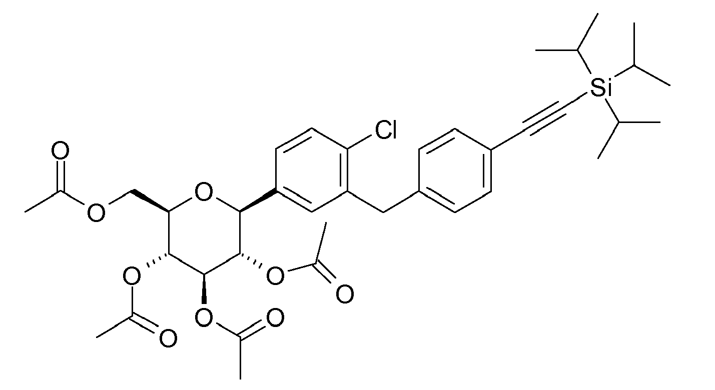 Massenspektrum (ESI⁺): m/z = 730/732 (CI) [M+NH₄]⁺

### Beispiel XIV

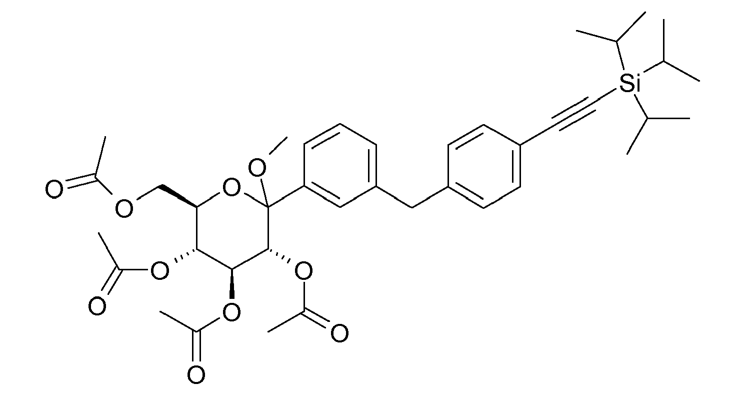

### 1-(2,3,4,6-Tetra-O-acetyl-1-methoxy-D-glucopyranos-1-yl)-3-(4-triisopropylsilylethinylbenzyl)-benzol

Eine Lösung von 2,6 g [4-(3-Brom-benzyl)-phenylethinyl]-triisopropyl-silan in 20 ml trockenem Diethylether wird unter Argon auf -80°C abgekühlt. Zu der gekühlten Lösung werden 7,9 ml einer 1,7 M Lösung von tert-Butyllithium in Pentan langsam getropft, und dann wird die Lösung 30 min bei -80°C gerührt. Zu dieser Lösung wird nun über eine Umdrücknadel eine -80°C-kalte Lösung von 3,2 g 2,3,4,6-Tetrakis-O-(trimethylsilyl)-D-glucopyranon in 30 ml Diethylether getropft. Die resultierende Lösung wird 2 h bei -78°C gerührt und danach wird noch einmal eine -80°C-kalte Lösung von 1,0 g g 2,3,4,6-Tetrakis-O-(trimethylsilyl)-D-glucopyranon in 10 ml Diethylether zugetropft. Nach einer weiteren Stunde Rührens bei -78°C wird eine Lösung von 2 ml Methansulfonsäure in 20 ml Methanol zugegeben, das Kühlbad entfernt und die Lösung 16 h bei Raumtemperatur gerührt. Die Lösung wird anschließend mit Ethyldiisopropylamin neutralisiert, das Lösungsmittel vollständig entfernt und der Rückstand in 50 ml Toluol aufgenommen. Es werden 8,5 ml Ethyldiisopropylamin zugesetzt, und die Lösung wird im Eisbad abgekühlt. Danach werden 4,3 ml Essigsäureanhydrid und 0,15 g 4-Dimethylaminopyridin zugegeben. Die Lösung wird 2 h bei Raumtemperatur gerührt und dann mit wässriger Natriumhydrogencarbonatlösung versetzt. Es wird mit Ethylacetat extrahiert, die organischen Phasen werden über Natriumsulfat getrocknet, und das Lösungsmittel wird entfernt. Der Rückstand wird über Kieselgel chromatografiert (Cyclohexan/Ethylacetat 4:1->1:3).
Ausbeute: 2,0 g (46% der Theorie)
Massenspektrum (ESI⁺): m/z = 726 [M+NH₄]⁺

Analog Beispiel XIV wird folgende Verbindung erhalten:
(1) 1-(Triisopropylsilylethinyl)-4-(2,3,4,6-tetra-O-acetyl-1-methoxy-D-glucopyranos-1-yl)-2-(4-ethoxy-benzyl)-benzol 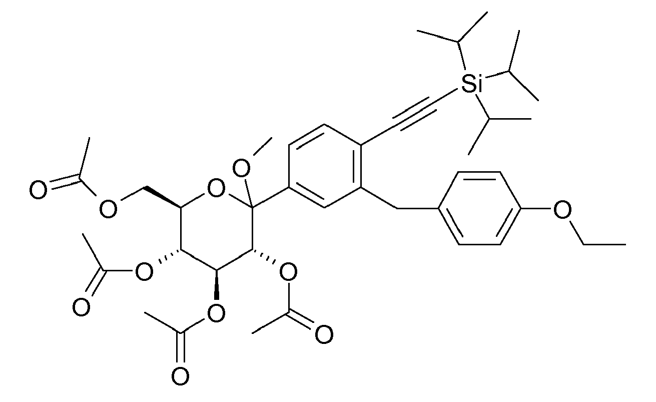 Massenspektrum (ESI⁺): m/z = 770 [M+NH₄]⁺

### Beispiel XV

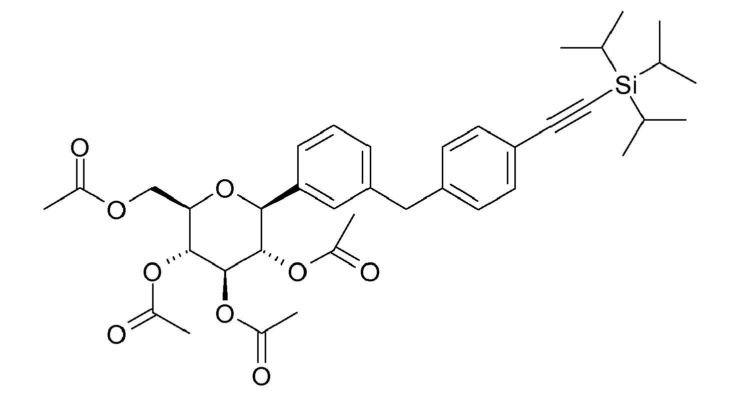

### 1-(2,3,4,6-Tetra-O-acetyl-β-D-glucopyranos-1-yl)-3-(4-triisopropylsilylethinyl-benzyl)-benzol

Zu einer eisgekühlten Lösung von 1,0 g 1-(2,3,4,6-tetra-O-acetyl-1-methoxy-D-glucopyranos-1-yl)-3-(4-triisopropylsilylethinyl-benzyl)-benzol und 25 µl Wasser in 10 ml Acetonitril werden 1,2 ml Triethylsilan und 0,36 ml Bortrifluoridetherat getropft. Die Lösung wird dann 3 h im Eisbad und 1 h bei Raumtemperatur gerührt. Danach wird die Lösung erneut im Eisbad abgekühlt, und es werden noch einmal 1,2 ml Triethylsilan und 0,36 ml Bortrifluoridetherat zugegeben. Die Lösung wird weitere 0,5 h im Eisbad und 2 h bei Raumtemperatur gerührt. Zu der Lösung wird dann wässrige Natriumhydrogencarbonatlösung gegeben, und die resultierende Lösung wird mit Ethylacetat extrahiert. Die organische Phase wird über Natriumsulfat getrocknet und das Lösungsmittel entfernt.
Ausbeute: 0,78 g (81 % der Theorie)
Massenspektrum (ESI⁺): m/z = 696 [M+NH₄]⁺

Analog Beispiel XV wird folgende Verbindung erhalten:
(1) 1-(Triisopropylsilylethinyl)-4-(2,3,4,6-tetra-O-acetyl-β-D-glucopyranos-1-yl)-2-(4-ethoxybenzyl)-benzol

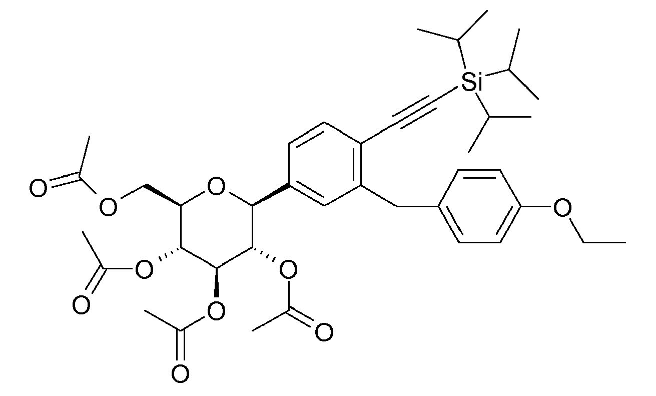

### Beispiel XVI

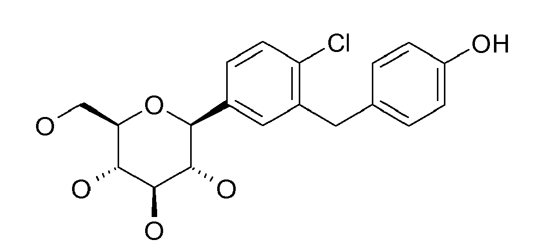

### 1-Chlor-4-(β-D-glucopyranos-1-yl)-2-(4-hydroxybenzyl)-benzol

Eine Lösung von 4,0 g [4-(5-Brom-2-chlor-benzyl)-phenoxy]-tert-butyl-dimethyl-silan in 42 ml trockenem Diethylether wird unter Argon auf -80°C abgekühlt. Zu der gekühlten Lösung werden 11,6 ml einer 1,7 M Lösung von tert-Butyllithium in Pentan langsam getropft, und dann wird die Lösung 30 min bei -80°C gerührt. Diese Lösung wird nun über eine Umdrücknadel, die mit Trockeneis gekühlt wird, zu einer -80°C-kalten Lösung von 4,78 g 2,3,4,6-Tetrakis-O-(trimethylsilyl)-D-glucopyranon in 38 ml Diethylether getropft. Die resultierende Lösung wird 3 h bei -78°C gerührt. Danach wird eine Lösung von 1,1 ml Methansulfonsäure in 35 ml Methanol zugegeben und die Lösung 16 h bei Raumtemperatur gerührt. Die Lösung wird anschließend mit festem Natriumhydrogencarbonat neutralisiert, es wird Ethylacetat zugegeben und das Methanol zusammen mit dem Ether entfernt. Zur verbleibenden Lösung wird wässrige Natriumhydrogencarbonatlösung gegeben und vier Mal mit Ethylacetat extrahiert. Die organischen Phasen werden über Natriumsulfat getrocknet und eingeengt. Der Rückstand wird in 30 ml Acetonitril und 30 ml Dichlormethan gelöst und die Lösung auf -10°C abgekühlt. Nach Zugabe von 4,4 ml Triethylsilan werden 2,6 ml Bortrifluoridetherat so zugetropft, dass die Temperatur nicht über -5°C steigt. Nach vollständiger Zugabe wird die Lösung noch 5 h bei -5 bis -10°C gerührt und anschließend durch Zugabe von wässriger Natriumhydrogencarbonatlösung gequencht. Die organische Phase wird abgetrennt und die wässrige vier Mal mit Ethylacetat extrahiert. Die vereinigten organischen Phase werden über Natriumsulfat getrocknet, das Lösungsmittel wird entfernt und der Rückstand über Kieselgel gereinigt. Das danach erhaltene Produkt ist ein ca. 6:1-β/a-Gemisch, welches durch vollständige Acetylierung der Hydroxygruppen mit Acetanhydrid und Pyridin in Dichlormethan und Umkristallisieren des Produkts in Ethanol in das reine β-Anomer überführt werden kann. Das so erhaltene Produkt wird durch Umsetzung in Methanol mit 4 M Kalilauge in die Titelverbindung überführt.
Ausbeute: 1,6 g (46% der Theorie)
Massenspektrum (ESI⁺): m/z = 398/400 (CI) [M+H]⁺

### Beispiel XVII

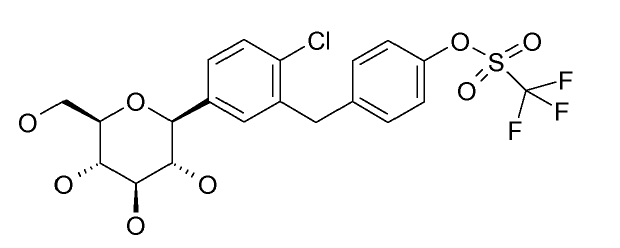

### 1-Chlor-4-(β-D-glucopvranos-1-yl)-2-[4-(trifluormethylulfonyloxy)-benzyl]-benzol

Zu einer Lösung von 0,38 g 1-Chlor-4-(β-D-glucopyranos-1-yl)-2-(4-hydroxybenzyl)-benzol, 0, 21 ml Triethylamin und 0,39 g N,N-Bis-(trifluormethansulfonyl)-anilin in 10 ml trockenem Dichlormethan werden 10 mg 4-Dimethylaminopyridin gegeben. Die Lösung wird 4 h bei Raumtemperatur gerührt und dann mit wässriger Natriumchloridlösung versetzt. Es wird mit Ethylacetat extrahiert, die organischen Extrakte werden über Natriumsulfat getrocknet, und das Lösungsmittel wird entfernt. Der Rückstand wird über Kieselgel chromatografiert (Dichlormethan/Methanol 1:0->4:1).
Ausbeute: 0,33 g (64% der Theorie)
Massenspektrum (ESI⁺): m/z = 530/532 (CI) [M+NH₄]⁺

### Beispiel XVIII

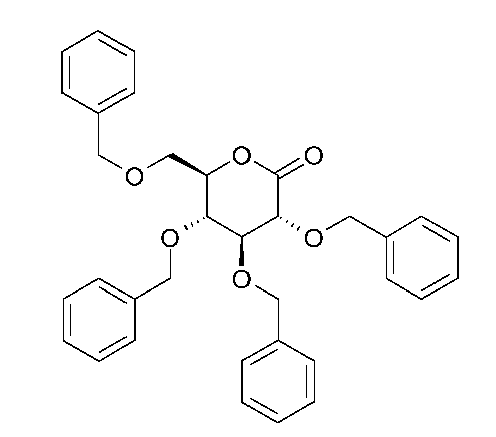

### 2,3,4,6-Tetra-O-benzyl-D-glucopyranon

Zu einer Lösung von 10,0 g 2,3,4,6-Tetra-O-benzyl-α-D-glucopyranose in 140 ml Dichlormethan werden 4 g frisch aktiviertes Molekularsieb 4Å und 3,3 g N-Methylmorpholin-N-oxid gegeben. Die Lösung wird 20 min bei Raumtemperatur gerührt, bevor 0,3 g Tetrapropylammoniumperrhutenat zugesetzt werden. Nach 2 h Rühren bei Raumtemperatur wird die Lösung mit Dichlormethan verdünnt und über Celite filtriert. Das Filtrat wird mit wässriger Natriumthiosulfatlösung und Wasser gewaschen und dann über Natriumsulfat getrocknet. Nach Entfernen des Lösungsmittels wird der Rückstand über Kieselgel chromatografiert (Cyclohexan/Ethylacetat 4:1).
Ausbeute: 8,2 g (82% der Theorie)
Massenspektrum (ESI⁺): m/z = 539 [M+H]⁺

### Beispiel XIX

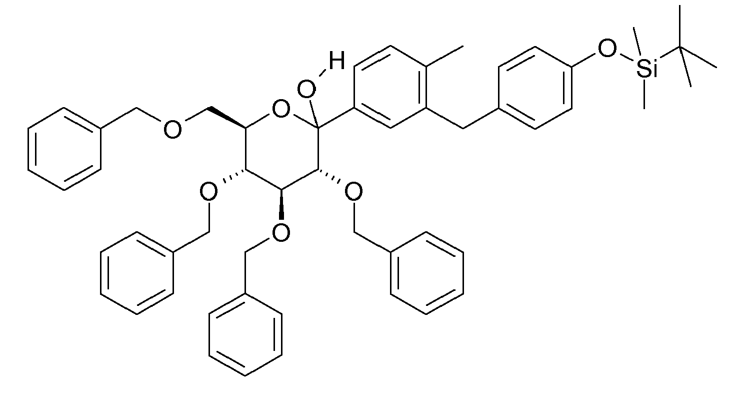

### 1-(2,3,4,6-Tetra-O-benzyl-1-hydroxy-D-glucopyranos-1-yl)-3-[4-(tert-butyl-dimethyl-silyloxy)-benzyl]-4-methyl-benzol

Eine Lösung von 0,34 g [4-(5-Brom-2-methyl-benzyl)-phenoxy]-tert-butyl-dimethyl-silan in 3 ml trockenem Tetrahydrofuran wird unter Argon auf -80°C abgekühlt. Zu der gekühlten Lösung werden 0,54 ml einer 1,6 M Lösung von n-Butyllithium in Hexan getropft, und die Lösung wird 1,5 h bei -78°C gerührt. Zu dieser Lösung wird mittels Umdrücknadel eine - 80°C-kalte Lösung von 0,43 g 2,3,4,6-Tetra-O-benzyl-D-glucopyranon in 2,5 ml Tetrahydrofuran getropft. Die resultierende Lösung wird 5 h bei -78°C gerührt. Die Reaktion wird mit einer Lösung von 0,1 ml Essigsäure in 1 ml Tetrahydrofuran gequencht und auf Raumtemperatur erwärmt. Nun wird wässrige Natriumhydrogencarbonatlösung zugegeben und vier Mal mit Ethylacetat extrahiert. Die organischen Phasen werden über Natriumsulfat getrocknet und eingeengt. Der Rückstand wird durch Chromatographie auf Kieselgel gereinigt (Cyclohexan/Ethylacetat 15:1->4:1).
Ausbeute: 0,48 g (ca. 88% rein)
Massenspektrum (ESI⁺): m/z = 868 [M+H]⁺

### Beispiel XX

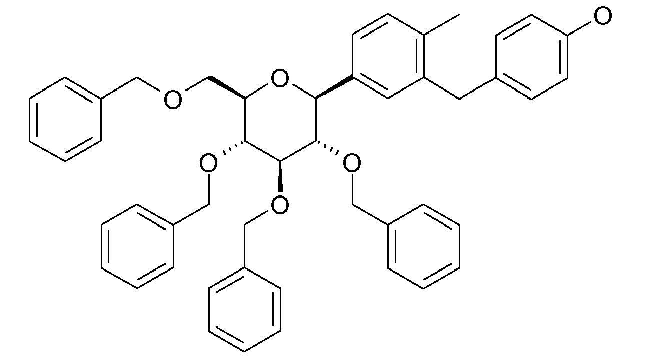

### 1-(2,3,4,6-Tetra-O-benzyl-β-D-glucopyranos-1-yl)-3-(4-hydroxy-benzyl)-4-methyl-benzol

Eine Lösung von 0,48 g (ca. 88% rein) 1-(2,3,4,6-Tetra-O-benzyl-1-hydroxy-D-glucopyranosyl)-3-[4-(tert-butyl-dimethyl-silyloxy)-benzyl]-4-methyl-benzol in 3,5 ml trockenem Acetonitril wird unter Argon auf -40°C abgekühlt. Zur gekühlten Lösung werden 0,13 ml Triisopropylsilan und 0,08 ml Bortrifluoridetherat getropft. Die Lösung wird 3 h bei - 35°C gerührt, bevor noch einmal 0,02 ml Triisopropylsilan und 0,01 ml Bortrifluoridetherat zugegeben werden. Nach weiteren 2 h bei -40°C wird wässriges Kaliumcarbonat zugesetzt und die Lösung 1 h bei Raumtemperatur gerührt. Danach wird mit Wasser verdünnt und vier Mal mit Ethylacetat extrahiert. Die organische Phase wird über Natriumsulfat getrocknet, konzentriert und über Kieselgel chromatografiert (Cyclohexan/Ethylacetat 10:1->4:1). Ausbeute: 0,24 g (68% der Theorie ). Massenspektrum (ESI⁺): m/z = 738 [M+NH₄]⁺

### Beispiel XXI

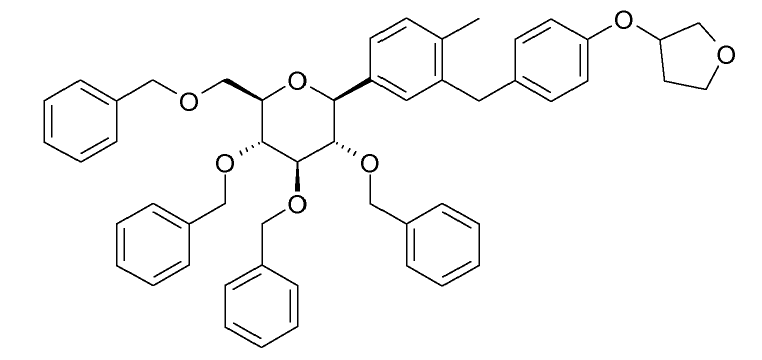

### 1-(2,3,4,6-Tetra-O-benzyl-β-D-glucopyranos-1-yl)-3-[4-(tetrahydrofuran-3-yloxy)-benzyl]-4-methyl-benzol

Zu einem Gemisch von 0,24 g 1-(2,3,4,6-Tetra-O-benzyl-β-D-glucopyranos-1-yl)-3-(4-hydroxy-benzyl)-4-methyl-benzol und 0,13 g Cesiumcarbonat in 2,5 ml Dimethylformamid werden 0,10 g Toluol-4-sulfonsäure-tetrahydrofuran-3-ylester gegeben. Das Gemisch wird 4 h bei 65°C gerührt, bevor Wasser zugesetzt wird. Es wird drei Mal mit Ethylacetat extrahiert, die organische Phase über Natriumsulfat getrocknet und das Lösungsmittel entfernt. Der Rückstand wird über Kieselgel gereinigt (Cyclohexan/Ethylacetat 10:1->4:1).
Ausbeute: 0,23 g (78% der Theorie). Massenspektrum (ESI⁺): m/z = 808 [M+H]⁺

### Beispiel XXII

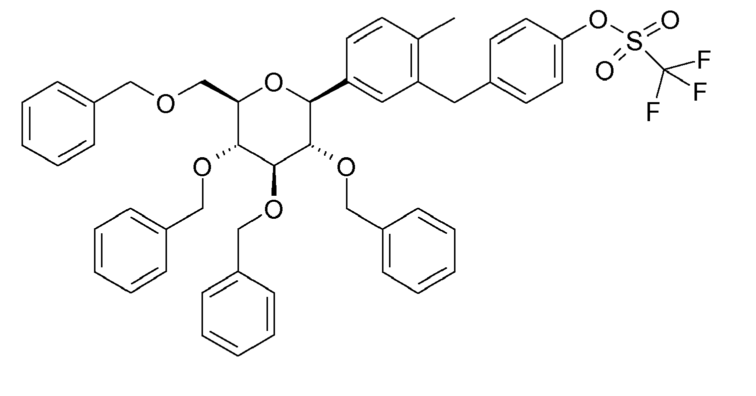

### 1-(2,3,4,6-Tetra-O-benzyl-β-D-glucopyranos-1-yl)-3-[4-(trifluormethylsulfonyloxy)-benzyl]-4-methyl-benzol

Eine Lösung von 0,62 g 1-(2,3,4,6-Tetra-O-benzyl-β-D-glucopyranos-1-yl)-3-(4-hydroxybenzyl)-4-methyl-benzol in 4,5 ml trockenem Dichlormethan wird unter Argon auf -10°C abgekühlt. Zur gekühlten Lösung werden 0,14 ml Pyridin und eine Lösung von 0,3 g Trifluormethansulfonsäureanhydrid in 0,5 ml Dichlormethan gegeben. Die Lösung wird 0,5 h bei -5 bis -10°C gerührt, bevor wässrige Natriumhydrogencarbonatlösung zugegeben wird. Es wird drei Mal mit Dichlormethan extrahiert, die gesammelten organischen Phasen werden mit wässriger 1 M Salzsäure gewaschen und über Natriumsulfat getrocknet. Nach Entfernen des Lösungsmittels wird der Rückstand über Kieselgel chromatografiert (Cyclohexan/Ethylacetat 15:1->7:1).
Ausbeute: 0,62 g (84% der Theorie)
Massenspektrum (ESI⁺): m/z = 853 [M+H]⁺

### Beispiel XXIII

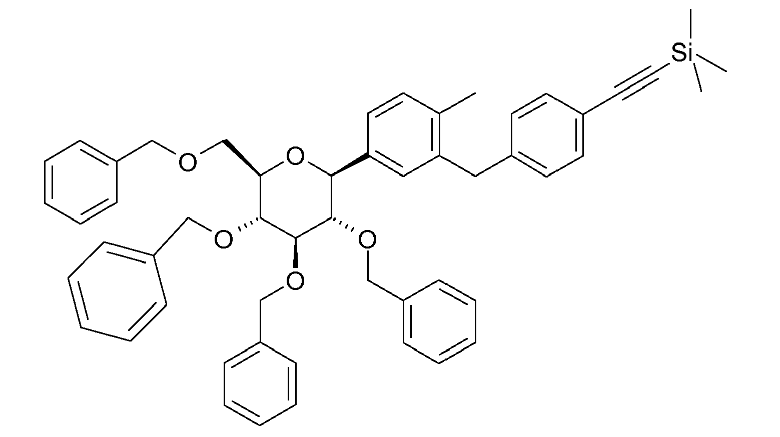

### 1-(2,3,4,6-Tetra-O-benzyl-β-D-glucopyranos-1-yl)-3-[4-(trimethylsiylethinyl)-benzyl]-4-methyl-benzol

Unter Argon werden zu einer Lösung von 0,60 g 1-(2,3,4,6-Tetra-O-benzyl-β-D-glucopyranos-1-yl)-3-[4-(trifluormethylsulfonyloxy)-benzyl]-4-methyl-benzol in 3 ml Dimethylformamid 27 mg Kupferiodid, 49 mg Bis-(triphenylphosphin)-palladiumdichlorid, 0,30 ml Triethylamin und zuletzt 0,14 ml Trimethylsilylacetylen gegeben. Der Kolben wird dicht verschlossen und 4 h bei 90°C gerührt. Danach werden noch einmal 20 mg Bis-(triphenylphosphin)-palladiumdichlorid und 0,6 ml Trimethylsilylacetylen zugegeben, und die Lösung wird weitere 4 h bei 90°C gerührt. Anschließend wird wässrige Natriumhydrogencarbonatlösung zugesetzt, drei Mal mit Ethylacetat extrahiert, und die gesammelten organischen Phasen werden über Natriumsulfat getrocknet. Nach Entfernen des Lösungsmittels wird der Rückstand über Kieselgel chromatografiert (Cyclohexan/Ethylacetat 40:1->10:1).
Ausbeute: 0,45 g (80% der Theorie )
Massenspektrum (ESI⁺): m/z = 818 [M+NH₄]⁺

### Herstellung der Endverbindungen:

### Beispiel 1

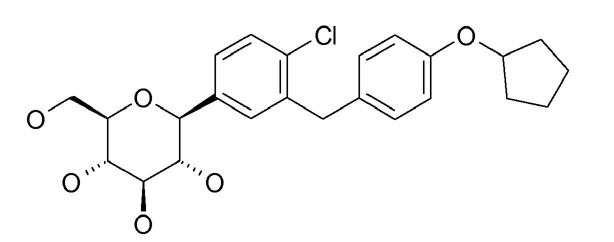

### 1-Chlor-2-(4-cyclopentyloxybenzyl)-4-(β-D-glucopyranos-1-yl)-benzol

Zu einem Gemisch von 0,25 g 1-Chlor-4-(β-D-glucopyranos-1-yl)-2-(4-hydroxybenzyl)-benzol und 0,4 g Cesiumcarbonat in 2,5 ml Dimethylformamid werden 0,16 ml Iodcyclopentan gegeben. Das Gemisch wird 4 h bei 45°C gerührt, bevor noch einmal 0,1 g Cesiumcarbonat und 0,05 ml Iodcyclopentan zugesetzt werden. Nach weiteren 14 h Rühren bei 45°C wird wässrige Natriumchloridlösung zugegeben und mit Ethylacetat extrahiert. Die organische Phase wird über Natriumsulfat getrocknet, das Lösungsmittel entfernt und der Rückstand über Kieselgel gereinigt (Dichlormethan/Methanol 1:0->5:1).
Ausbeute: 0,23 g (78% der Theorie)
Massenspektrum (ESI⁺): m/z = 466/468 (CI) [M+NH₄]⁺

Analog Beispiel 1 werden folgende Verbindungen erhalten:
(2) 1-Chlor-4-(β-D-glucopyranos-1-yl)-2-[4-((*R*)-tetrahydrofuran-3-yloxy)-benzyl]-benzol Die Reaktion wird mit (*S*)-Toluol-4-sulfonsäure-tetrahydrofuran-3-ylester als Kupplungspartner durchgeführt. 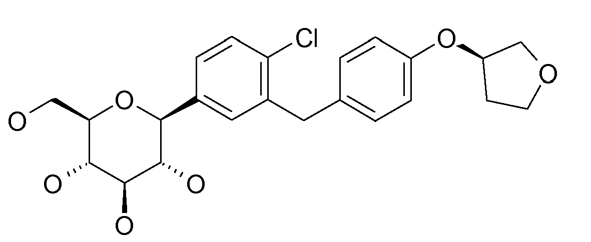 Massenspektrum (ESI⁺): m/z = 451/453 (CI) [M+H] ⁺
(3) 1-Chlor-4-(β-D-glucopyranos-1-yl)-2-[4-((*S*)-tetrahydrofuran-3-yloxy)-benzyl]-benzol Die Reaktion wird mit (*R*)-Toluol-4-sulfonsäure-tetrahydrofuran-3-ylester als Kupplungspartner durchgeführt. 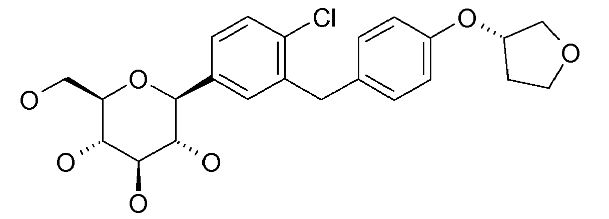 Massenspektrum (ESI⁺): m/z = 451/453 (CI) [M+H] ⁺
(4) 1-Chlor-4-(β-D-glucopyranos-1-yl)-2-[4-(tetrahydrofuran-2-on-3-yloxy)-benzyl]-benzol Die Reaktion wird mit 3-Brombutyrolacton als Kupplungspartner durchgeführt. 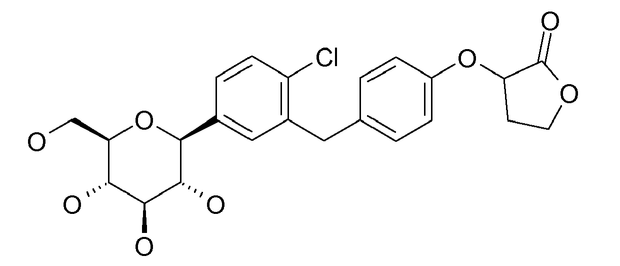 Massenspektrum (ESI⁺): m/z = 465/467 (CI) [M+H] ⁺
(5) 1-Chlor-4-(β-D-glucopyranos-1-yl)-2-(4-cyclobutyloxy-benzyl)-benzol 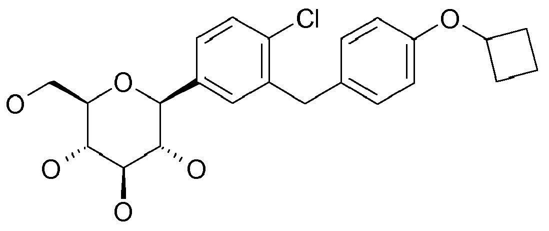 Massenspektrum (ESI⁺): m/z = 452/454 (CI) [M+NH₄]⁺
(6) 1-Chlor-4-(β-D-glucopyranos-1-yl)-2-(4-cyclohexyloxy-benzyl)-benzol 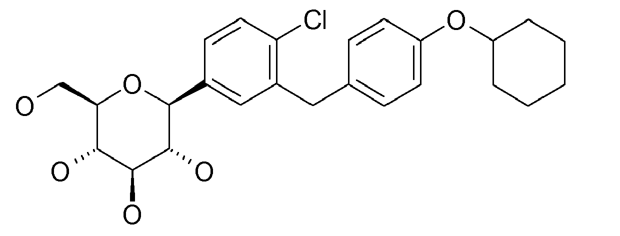 Massenspektrum (ESI⁺): m/z = 480/482 (CI) [M+NH₄]⁺
(7) 1-Chlor-4-(β-D-glucopyranos-1-yl)-2-[4-(tetrahydropyran-4-yloxy)-benzyl]-benzol 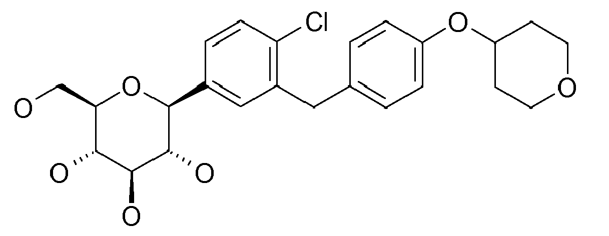 Massenspektrum (ESI⁺): m/z = 487/489 (CI) [M+Na]⁺
(8) 1-Chlor-4-(β-D-glucopyranos-1-yl)-2-[4-(1-acetyl-piperidin-4-yloxy)-benzyl]-benzol Die Umsetzung wird mit 1-Acetyl-4-methylsulfonyloxy-piperidin als Elektrophil durchgeführt. 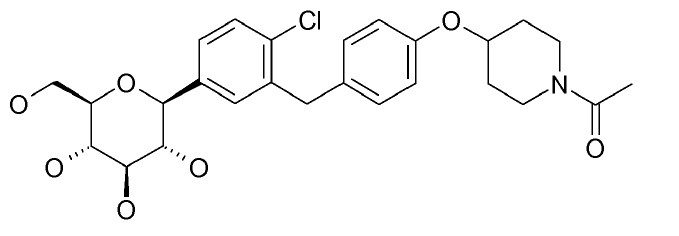 Massenspektrum (ESI⁺): m/z = 506/508 (CI) [M+H] ⁺
(9) 1-Chlor-4-(β-D-glucopyranos-1-yl)-2-[4-(1-tert-butyloxycarbonylpiperidin-4-yloxy)-benzyl]-benzol
   Die Umsetzung wird mit 1-tert-Butyloxycarbonyl-4-methylsulfonyloxy-piperidin als Elektrophil durchgeführt. 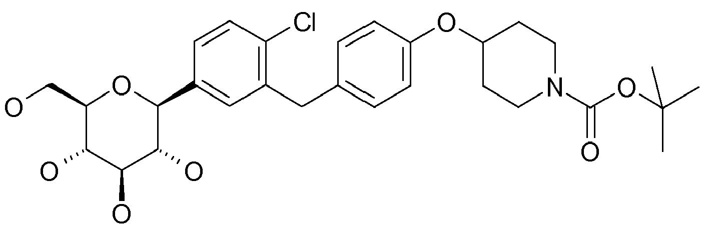 Massenspektrum (ESI⁺): m/z = 586/588 (CI) [M+Na]⁺

### Beispiel 10

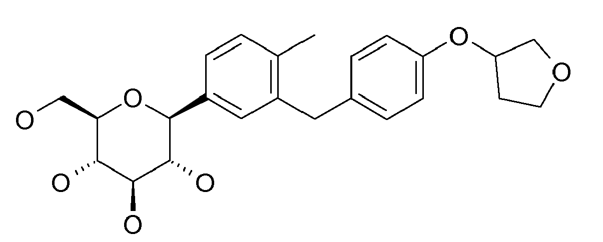

### 1-(β-D-Glucopyranos-1-yl)-4-methyl-3-[4-(tetrahydrofuran-3-yloxy)-benzyl]-benzol

Ein Gemisch von 0,21 g 1-(2,3,4,6-Tetra-O-benzyl-β-D-glucopyranos-1-yl)-3-[4-(tetrahydrofuran-3-ylox)-benzyl]-4-methyl-benzol und 0,1 g 10% Palladiumhydroxid auf Kohle in 3 ml Ethylacetat wird 24 h unter einem Wasserstoffdruck von 1 atm bei Raumtemperatur geschüttelt. Danach wird noch einmal die gleiche Menge Katalysator zugegeben und für weitere 24 h unter Wasserstoffatmosphäre geschüttelt. Dann wird der Katalysator abfiltriert, das Filtrat eingeengt und der Rückstand über Kieselgel chromatografiert (Dichlormethan/Methanol 1:0->5:1).
Ausbeute: 0,06 g (49% der Theorie)
Massenspektrum (ESI⁺): m/z = 448 [M+NH₄]⁺

### Beispiel 11

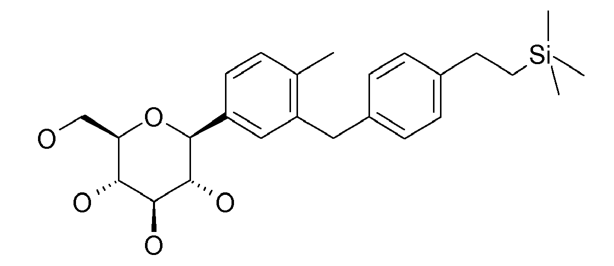

### 1-(β-D-Glucopyranos-1-yl)-4-methyl-3-[4-(2-trimethylsilyl-ethyl)-benzyl]-benzol

Ein Gemisch von 0,29 g 1-(2,3,4,6-tetra-O-benzyl-β-D-glucopyranos-1-yl)-4-methyl-3-[4-(trimethylsilylethinyl)-benzyl]-benzol und 0,25 g 10% Palladiumhydroxid auf Kohle in 3 ml Ethylacetat wird 24 h unter einem Wasserstoffdruck von 1 atm bei Raumtemperatur geschüttelt. Danach werden noch einmal 0,2 g Katalysator zugegeben, und die Lösung wird für weitere 20 h unter Wasserstoffatmosphäre geschüttelt. Dann wird der Katalysator abfiltriert, das Filtrat eingeengt und der Rückstand über Kieselgel chromatografiert (Dichlormethan/Methanol 1:0->5:1).
Ausbeute: 0,08 g (51% der Theorie)
Massenspektrum (ESI⁺): m/z = 462 [M+NH₄]⁺

### Beispiel 12

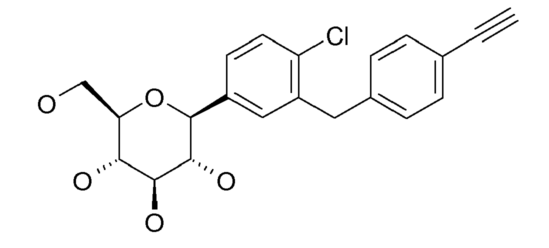

### 1-Chlor-4-(β-D-glucopyranos-1-yl)-2-(4-ethinyl-benzyl)-benzol

Unter Argon werden zu einer Lösung von 0,32 g 1-Chlor-4-(β-D-glucopyranos-1-yl)-2-[4-(trifluormethylsulfonyloxy)-benzyl]-benzol in 3 ml Dimethylformamid 25 mg Kupferiodid, 44 mg Bis-(triphenylphosphin)-palladiumdichlorid, 0,30 ml Triethylamin und zuletzt 0,14 ml Trimethylsilylacetylen gegeben. Der Kolben wird dicht verschlossen und 8 h bei 90°C gerührt. Danach werden noch einmal 25 mg Bis-(triphenylphosphin)-palladiumdichlorid und 0,1 ml Trimethylsilylacetylen zugegeben, und die Lösung wird weitere 10 h bei 90°C gerührt. Anschließend wird wässrige Natriumhydrogencarbonatlösung zugesetzt, drei Mal mit Ethylacetat extrahiert, und die gesammelten organischen Phasen werden über Natriumsulfat getrocknet. Nach Entfernen des Lösungsmittels wird der Rückstand in 5 ml Methanol gelöst und mit 0,12 g Kaliumcarbonat versetzt. Das Gemisch wird 1 h bei Raumtemperatur gerührt und dann mit 1 M Salzsäure neutralisiert. Danach wird das Methanol abgedampft, der Rückstand mit wässriger Natriumchloridlösung versetzt und mit Ethylacetat extrahiert. Die gesammelten organischen Extrakte werden über Natriumsulfat getrocknet, und das Lösungsmittel wird entfernt. Der Rückstand wird über Kieselgel chromatografiert (Dichlormethan/Methanol 1:0->5:1).
Ausbeute: 0,095 g (40% der Theorie)
Massenspektrum (ESI⁺): m/z = 406/408 (CI) [M+NH₄]⁺

Diese Verbindung kann auch gemäß Beispiel 14 erhalten werden.

### Beispiel 13

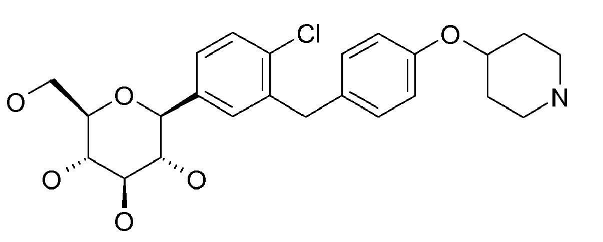

### 1-Chlor-4-(β-D-glucopyranos-1-yl)-2-[4-(piperdin-4-yloxy)-benzyl]-benzol

Zu einer Lösung von 0,19 g 1-Chlor-4-(β-D-glucopyranos-1-yl)-2-[4-(1-tert-butlyoxycarbonylpiperidin-4-yloxy)-benzyl]-benzol in 4 ml Dichlormethan werden 2 ml Trifluoressigsäure gegeben. Die Lösung wird 1,5 h bei Raumtemperatur gerührt und dann mit Ethylacetat verdünnt und mit wässriger Kaliumcarbonatlösung basisch gestellt. Die organische Phase wird abgetrennt und die wässrige mit Ethylacetat extrahiert. Die vereinten organischen Phasen werden über Natriumsulfat getrocknet und das Lösungsmittels vollständig entfernt.
Ausbeute: 0,060 g (38% der Theorie)
Massenspektrum (ESI⁺): m/z = 464/466 (CI) [M+H]⁺

### Beispiel 14

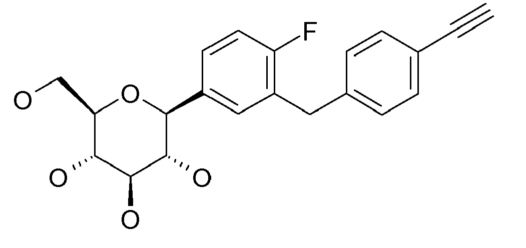

### 1-Fluor-4-(β-D-glucopyranos-1-yl)-2-(4-ethinyl-benzyl)-benzol

Zu einer Lösung von 0,23 g 1-Fluor-4-(2,3,4,6-tetra-O-acetyl-β-D-glucopyranos-1-yl)-2-(triisopropylsilylethinyl-benzyl)-benzol in 1,5 ml Tetrahydrofuran werden 0,33 ml einer 1 M Lösung von Tetrabutylammoniumfluorid in Tetrahydrofuran gegeben. Die Lösung wird 1 h bei Raumtemperatur gerührt. Dann werden 1 ml Methanol und 1,5 ml 4 M Kalilauge zugegeben und die Lösung eine weitere Stunde bei Raumtemperatur gerührt. Die Lösung wird mit 1 M Salzsäure neutralisiert und danach das Methanol abgedampft. Der Rückstand wird mit wässriger Natriumchloridlösung versetzt und mit Ethylacetat extrahiert. Die gesammelten organischen Extrakte werden über Natriumsulfat getrocknet, und das Lösungsmittel wird entfernt. Der Rückstand wird über Kieselgel chromatografiert (Dichlormethan/Methanol 19:1->2:1).
Ausbeute: 0,060 g (49% der Theorie)
Massenspektrum (ESI⁺): m/z = 390 [M+NH₄]⁺

Analog Beispiel 14 werden folgende Verbindungen erhalten:
(15) 1-(β-D-Glucopyranos-1-yl)-3-(4-ethinyl-benzyl)-benzol 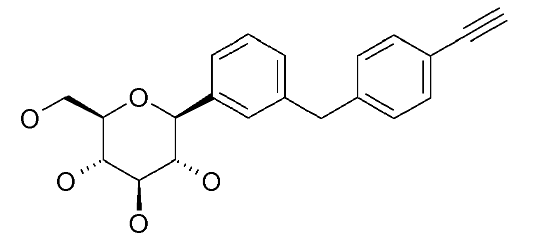 Massenspektrum (ESI⁺): m/z = 372 [M+NH₄]⁺
(16) 1-Ethinyl-4-(β-D-glucopyranos-1-yl)-2-(4-ethoxy-benzyl)-benzol 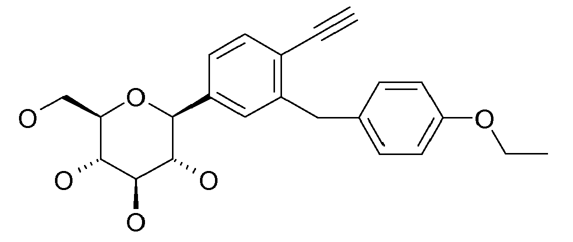 Massenspektrum (ESI⁺): m/z = 416 [M+NH₄]⁺
(17) 1-Methoxy-4-(β-D-glucopyranos-1-yl)-2-(4-ethinyl-benzyl)-benzol 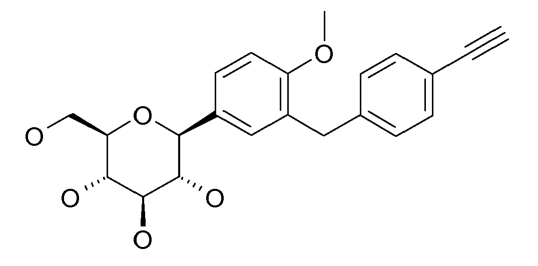 Massenspektrum (ESI⁺): m/z = 402 [M+NH₄]⁺

Die Verbindung gemäß Beispiel (12) (1-Chlor-4-(β-D-glucopyranos-1-yl)-2-(4-ethinyl-benzyl)-benzol) kann auch analog Beispiel 14 synthetisiert werden. Optional kann hierbei die Zwischenstufe, 1-Chlor-4-(2,3,4,6-tetra-O-acteyl-β-D-glucopyranos-1-yl)-2-(4-ethinyl-benzyl)-benzol, die nach der Desilylierung mit Tetrabutylammoniumfluorid erhalten wird, durch Umkristallisieren in Ethanol gereinigt werden.

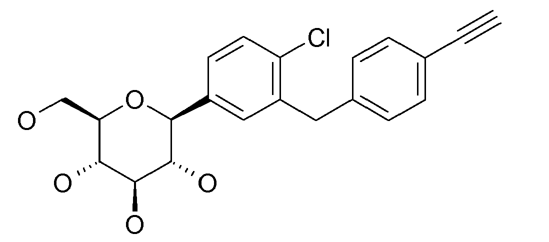

Massenspektrum (ESI⁺): m/z = 406/408 (CI) [M+NH₄]⁺

Analog den vorstehend genannten Beispielen und anderen literaturbekannten Verfahren werden auch folgende Verbindungen hergestellt:

| Bsp. | Struktur | Bsp. | Struktur |
|---|---|---|---|
| (18) | 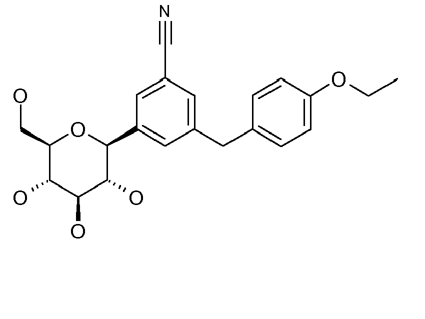 | (19) | 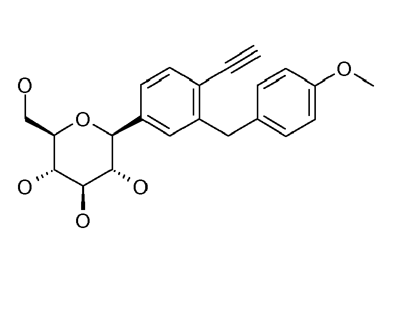 |
| (20) | 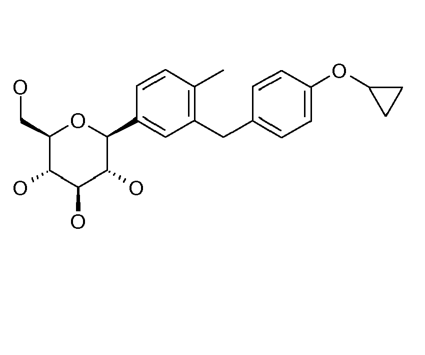 | (21) | 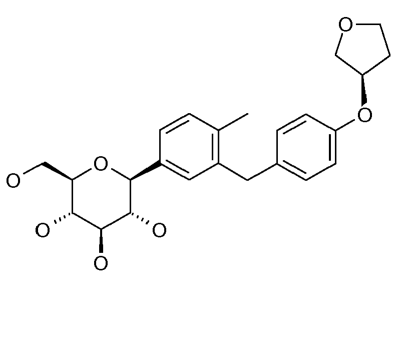 |
| (22) | 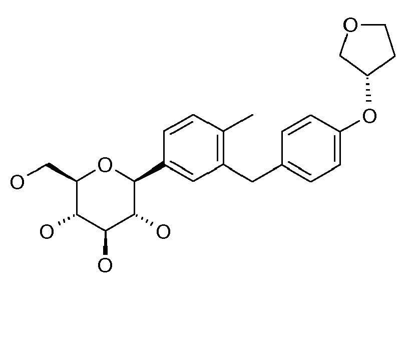 | (23) | 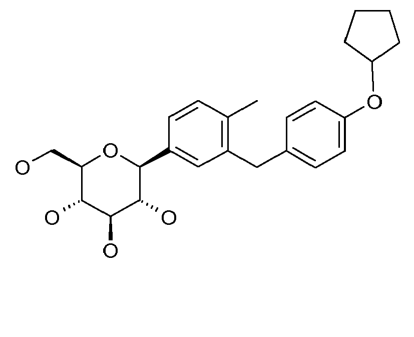 |
| (24) | 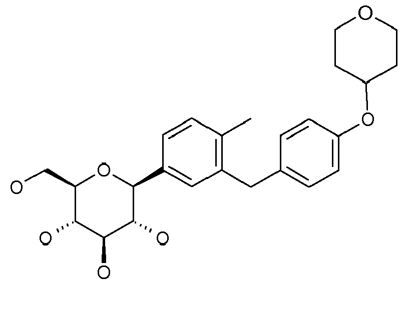 | (25) | 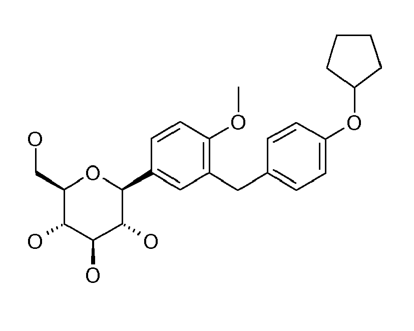 |
| (26) | 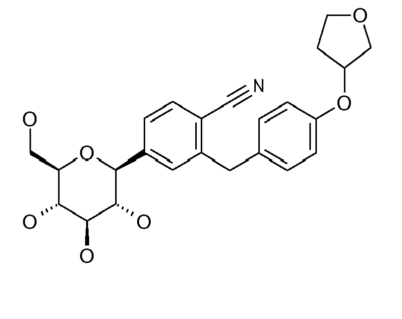 | (27) | 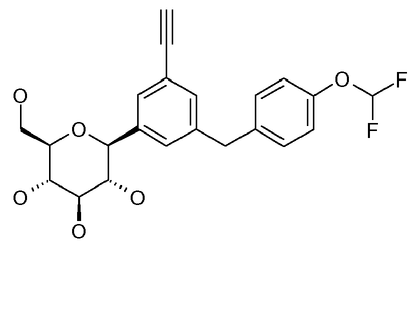 |
| (28) | 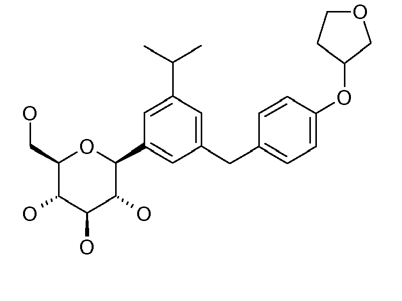 | (29) | 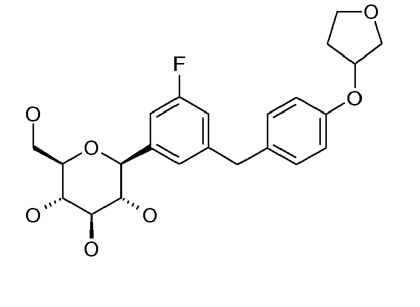 |
| (30) | 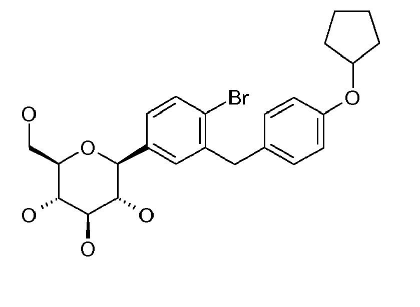 | (31) | 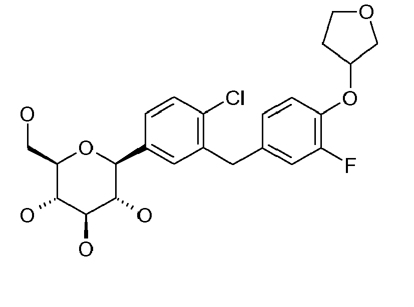 |
| (32) | 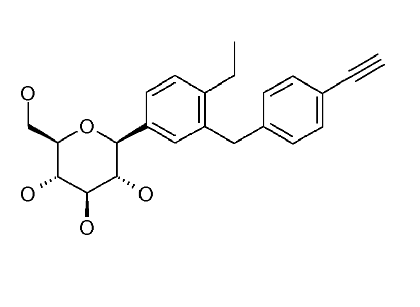 | (33) | 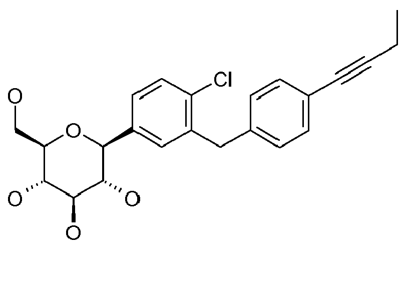 |
| (34) | 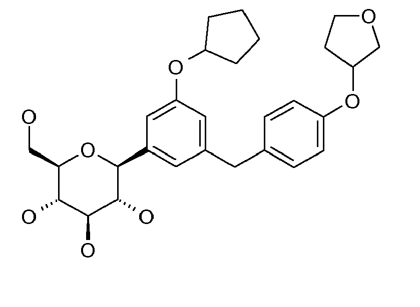 | (35) | 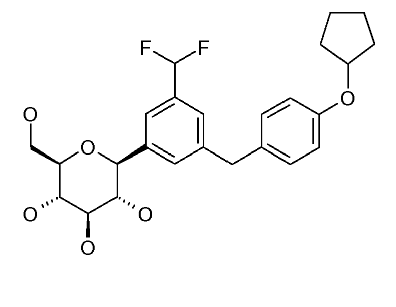 |
| (36) | 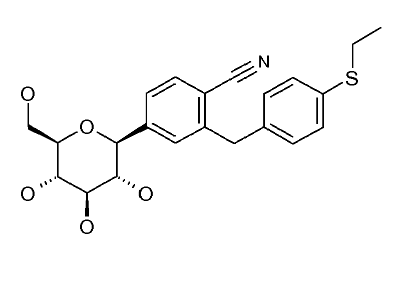 | (37) | 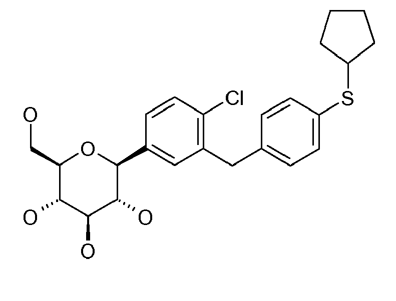 |
| (38) | 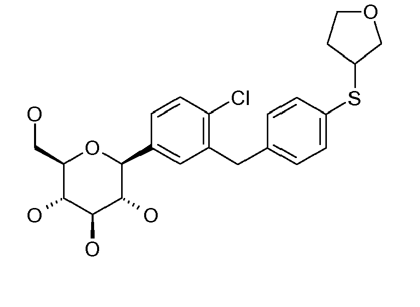 | (39) | 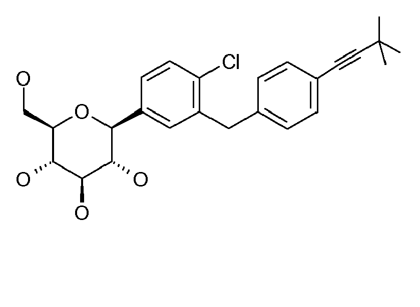 |
| (40) | 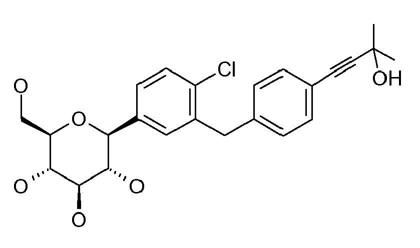 | (41) | 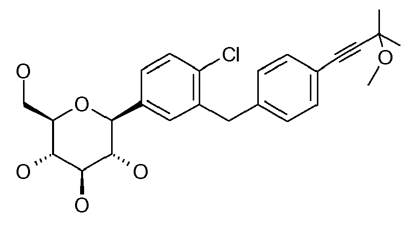 |
| (42) | 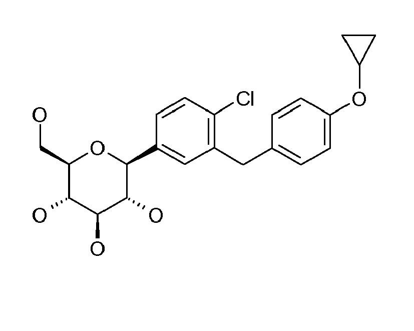 | (43) | 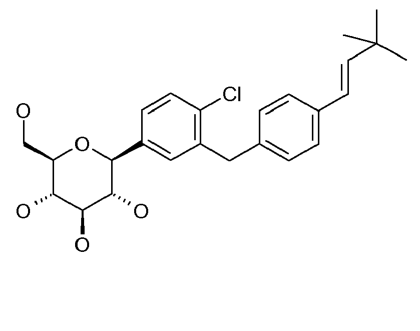 |
| (44) | 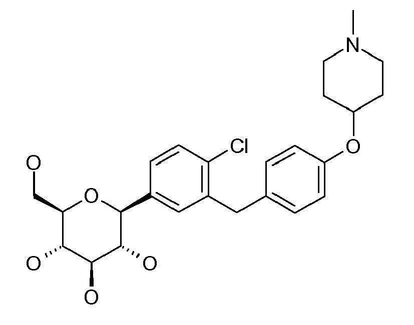 | (45) | 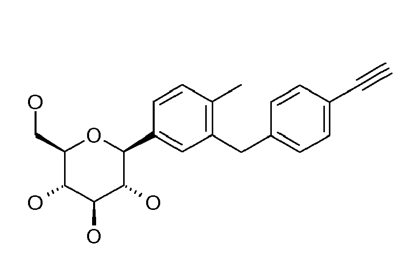 |
| (46) | 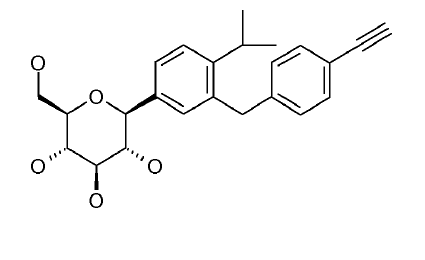 | (47) | 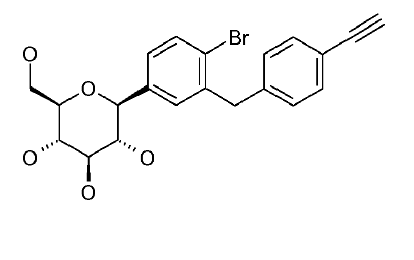 |
| (48) | 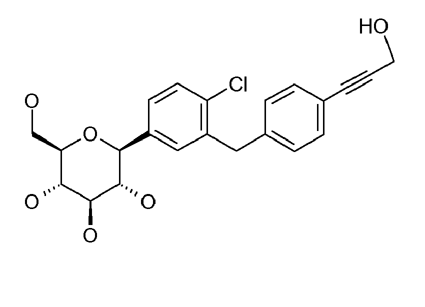 | (49) | 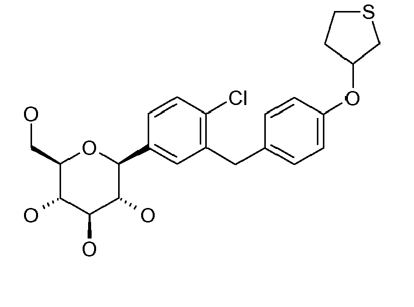 |
| (50) | 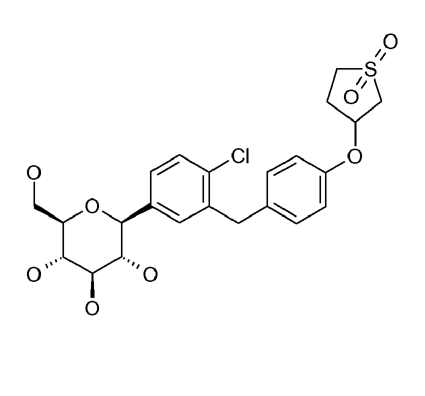 | (51) | 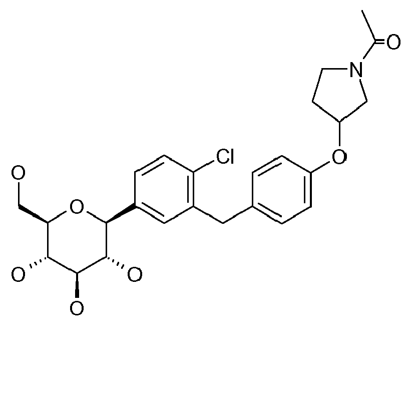 |
| (52) | 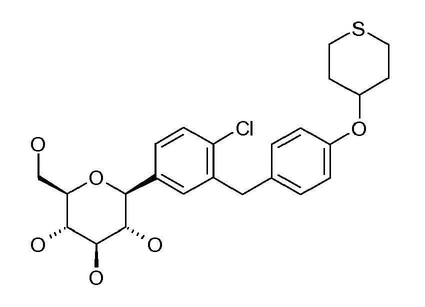 | (53) | 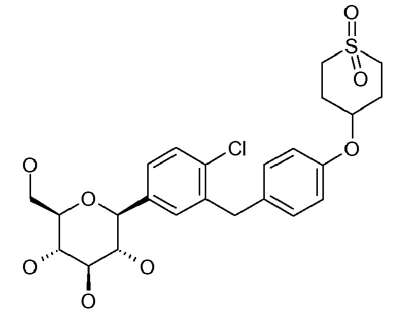 |
| (54) | 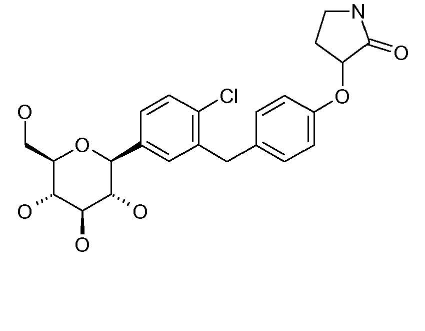 | (55) | 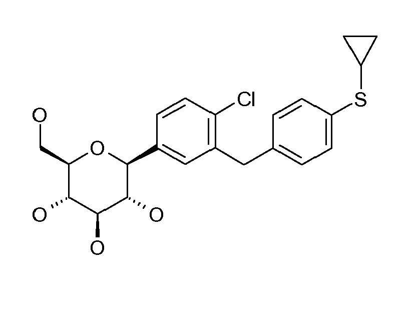 |
| (56) | 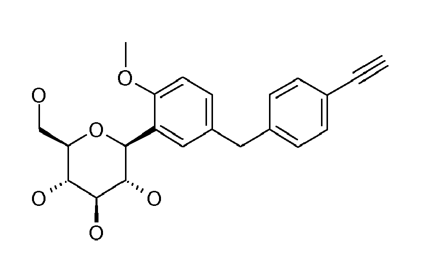 | (57) | 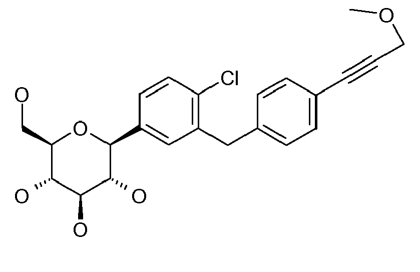 |

Nachfolgend werden Beispiele zu Darreichungsformen beschrieben, worin die Angabe "Wirkstoff" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

### Beispiel A

| Tabletten mit 100 mg Wirksubstanz | | |
|---|---|---|
| Zusammensetzung: | | |
| 1 | Tablette enthält: | |
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die pressfertige Mischung wird zu Tabletten verarbeitet.

| | |
|---|---|
| Tablettengewicht: | 220 mg |
| Durchmesser: 10 mm, | biplan mit beidseitiger Facette und einseitiger Teilkerbe. |

### Beispiel B

| Tabletten mit 150 mg Wirksubstanz | | |
|---|---|---|
| Zusammensetzung: | | |
| 1 | Tablette enthält: | |
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | | |
|---|---|---|
| Tablettengewicht: | 300 | mg |
| Stempel: | 10 | mm, flach |

### Beispiel C

| Hartgelatine-Kapseln mit 150 mg Wirksubstanz | | | |
|---|---|---|---|
| Zusammensetzung: | | | |
| 1 | Kapsel enthält: | | |
| | Wirkstoff | | 150.0 mg |
| | Maisstärke getr. | ca. | 180.0 mg |
| | Milchzucker pulv. | ca. | 87.0 mg |
| | Magnesiumstearat | | 3.0 mg |
| | ca. | | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel D

| Suppositorien mit 150 mg Wirksubstanz | | |
|---|---|---|
| Zusammensetzung: | | |
| 1 | Zäpfchen enthält: | |
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel E

| Ampullen mit 10 mg Wirksubstanz | | |
|---|---|---|
| Zusammensetzung: | | |
| Wirkstoff | 10.0 | mg |
| 0.01 n Salzsäure s.q. | | |
| Aqua bidest ad | 2.0 | ml |

### Herstellung:

Die Wirksubstanz wird in der erforderlichen Menge 0.01 n HCl gelöst, mit Kochsalzgelöst, mit Kochsalz isotonisch gestellt, sterilfiltriert und in 2 ml Ampullen abgefüllt.

### Beispiel F

| Ampullen mit 50 mg Wirksubstanz | | |
|---|---|---|
| Zusammensetzung: | | |
| | Wirkstoff | 50.0 mg |
| | 0.01 n Salzsäure s.q. | |
| | Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

## Patentansprüche

1. Glucopyranosyl-substituierte Benzol-Derivate der allgemeinen Formel I 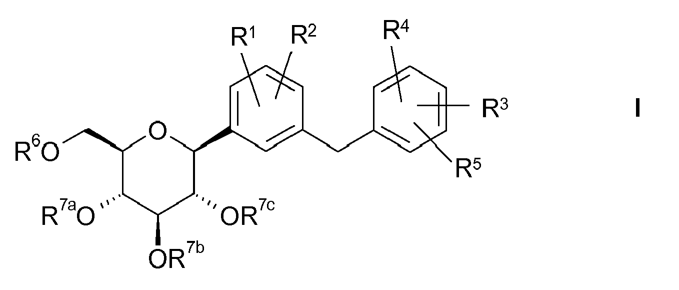 in der
R¹ ausgewählt ist aus den Bedeutungen der Gruppe A und
falls R³ ausgewählt ist aus den Bedeutungen der Gruppe B, kann R¹ zusätzlich auch ausgewählt sein aus den Bedeutungen Wasserstoff, Fluor, Chlor, Brom, Iod, C₁₋₄-Alkyl, C₂₋₄-Alkenyl-C₁₋₄-alkyl, C₂₋₄-Alkinyl-C₁₋₄-alkyl, C₂₋₄-Alkenyl-C₁₋₄-alkoxy, C₂₋₄-Alkinyl-C₁₋₄-alkoxy, C₃₋₇-Cycloalkyl-C₁₋₄-alkyl, C₅₋₇-Cycloalkenyl-C₁₋₄-alkyl, eine durch 1 bis 3 Fluoratome substituierte Methylgruppe, eine durch 1 bis 5 Fluoratome substituierte Ethylgruppe, C₁₋₄-Alkoxy, eine durch 1 bis 3 Fluoratome substituierte Methoxygruppe, eine durch 1 bis 5 Fluoratome substituierte Ethoxygruppe, eine durch eine Hydroxy- oder C₁₋₃-Alkoxygruppe substituierte C₁₋₄-Alkylgruppe, eine durch eine Hydroxy- oder C₁₋₃-Alkoxygruppe substituierte C₂₋₄-Alkoxygruppe, C₃₋₆-Cycloalkyl-C₁₋₃-alkoxy oder Hydroxy,
wobei in den vorstehend genannten Cycloalkyl- und Cycloalkenyl-Ringen ein oder zwei Methylengruppen unabhängig voneinander durch O oder CO ersetzt sein können, und
R² Wasserstoff, Fluor, Chlor, Brom, Hydroxy, C₁₋₄-Alkyl, C₁₋₄-Alkoxy, Cyan oder Nitro, wobei die Alkyl- oder Alkoxygruppe ein oder mehrfach mit Fluor substituiert sein kann, und
R³ ausgewählt ist aus den Bedeutungen der Gruppe B und
falls R¹ ausgewählt ist aus den Bedeutungen der Gruppe A, kann R³ zusätzlich auch ausgewählt sein aus den Bedeutungen Wasserstoff, Fluor, Chlor, Brom, Iod, C₁₋₆-Alkyl, C₂₋₄-Alkenyl-C₁₋₄-alkyl, C₂₋₄-Alkinyl-C₁-₄-alkyl, C₂₋₄-Alkenyl-C₁₋₄-alkoxy, C₂₋₄-Alkinyl-C₁₋₄-alkoxy, C₃₋₇-Cycloalkyl, C₅₋₇-Cycloalkenyl, C₃₋₇-Cycloalkyl-C₁₋₄-alkyl, C₅₋₇-Cycloalkenyl-C₁₋₄-alkyl, C₃₋₆-Cycloalkyliden-methyl, Hydroxy, C₁₋₆-Alkoxy, C₃₋₆-Cycloalkyl-C₁₋₃-alkoxy, Aryl, Aryl-C₁₋₃-alkyl, Heteroaryl, Heteroaryl-C₁₋₃-alkyl, Aryloxy, Aryl-C₁₋₃-alkyl-oxy, eine durch 1 bis 3 Fluoratome substituierte Methyl- oder Methoxygruppe, eine durch 1 bis 5 Fluoratome substituierte C₂₋₄-Alkyl- oder C₂₋₄-Alkoxygruppe, eine durch eine Cyangruppe substituierte C₁₋₄-Alkylgruppe, eine durch eine Hydroxy- oder C₁₋₃-Alkyloxygruppe substituierte C₁₋₄-Alkylgruppe, Cyan, Carboxy, C₁₋₃-Alkoxy-carbonyl, Aminocarbonyl, (C₁₋₃-Alkylamino)carbonyl, Di-(C₁₋₃-alkyl)amino-carbonyl, Pyrrolidin-1-ylcarbonyl, Piperidin-1-ylcarbonyl, Morpholin-4-ylcarbonyl, Piperazin-1-yl-carbonyl, 4-(C₁₋₃-Alkyl)-piperazin-1-ylcarbonyl, (C₁₋₄-Alkyl)carbonylamino-, C₁₋₄-Alkylsulfonylamino, C₁₋₄-Alkylsulfanyl-, C₁₋₄-Alkylsulfinyl-, C₁₋₄-Alkylsulfonyl-, Arylsulfonylamino, Aryl-C₁₋₃-alkylsulfonyl-amino oder Arylsulfonyl,
R⁴, R⁵ unabhängig voneinander Wasserstoff, Fluor, Chlor, Brom, Iod, Cyan, Nitro, C₁₋₃-Alkyl, C₁₋₃-Alkoxy, durch 1 bis 3 Fluoratome substituiertes Methyl- oder Methoxy,
A C₂₋₆-Alkin-1-yl, C₂₋₆-Alken-1-yl, C₃₋₇-Cycloalkyl, C₅₋₇-Cycloalkenyl, Aryl, Heteroaryl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Arylamino-carbonyl, Heteroarylaminocarbonyl, C₁₋₄-Alkoxycarbonyl, Aryl-C₁₋₃-alkoxycarbonyl, Heteroaryl-C₁₋₃-alkoxycarbonyl, Amino, C₁₋₄-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, C₁₋₄-Alkylcarbonylamino, Arylcarbonylamino, Heteroaryl-carbonylamino, C₃₋₇-Cycloalkyloxy, C₅₋₇-Cycloalkenyloxy, Aryloxy, Heteroaryloxy, C₁₋₄-Alkylsulfinyl, C₁₋₄-Alkylsulfonyl, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇- Cycloalkenylsulfinyl, C₅₋₇-Cycloalkenylsulfonyl, Arylsulfanyl, Arylsulfinyl, Arylsulfonyl, Heteroarylsulfanyl, Heteroarylsulfinyl, Heteroarylsulfonyl, Cyan oder Nitro bedeutet,
wobei die vorstehend genannten Alkinyl- und Alkenylgruppen ein- oder mehrfach mit Fluor oder Chlor substituiert sein können, und
wobei die vorstehend genannten Alkinyl- und Alkenylgruppen ein- oder zweifach mit gleichen oder verschiedenen Resten L1 substituiert sein können, und
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
B Tri-(C₁₋₄-alkyl)silyl-C₁₋₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Arylcarbonylamino, Heteroarylcarbonylamino, Nitro, C₃₋₁₀-Cycloalkyloxy, C₅₋₁₀-Cycloalkenyloxy, C₃₋₁₀-Cycloalkylsulfanyl, C₃₋₁₀Cyclo-alkylsulfinyl, C₃₋₁₀-Cycloalkylsulfonyl, C₅₋₁₀-Cycloalkenylsulfanyl, C₅₋₁₀-Cyclo-alkenylsulfinyl, C₅₋₁₀-Cycloalkenylsulfonyl, Arylsulfanyl, Arylsulfinyl, Heteroarylsulfanyl oder Heteroarylsulfinyl,
wobei die vorstehend genannten Alkinyl- und Alkenylgruppen ein- oder mehrfach mit Fluor oder Chlor substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N} ersetzt sein können,
R^{N} H, C₁₋₄-Alkyl, C₁₋₄-Alkylcarbonyl oder C₁₋₄-Alkylsulfonyl,
L1 unabhängig voneinander ausgewählt aus der Gruppe bestehend aus Hydroxy-, Cyan-, Nitro-, C₃₋₇-Cycloalkyl, Aryl-, Heteroaryl-, C₁₋₄-Alkylcarbonyl-, Arylcarbonyl-, Heteroarylcarbonyl-, Aminocarbonyl-, C₁₋₄-Alkylaminocarbonyl-, Di-(C₁₋₃-alkyl)aminocarbonyl-, Pyrrolidin-1-ylcarbonyl-, Piperidin-1-ylcarbonyl-, Morpholin-4-ylcarbonyl, Arylaminocarbonyl-, Heteroarylaminocarbonyl-, C₁₋₄-Alkoxycarbonyl-, Aryl-C₁₋₃-alkoxycarbonyl-, Heteroaryl-C₁₋₃-alkoxycarbonyl-, C₁₋₄-Alkyloxy-, Aryloxy-, Heteroaryloxy-, C₁₋₄-Alkylsulfanyl-, Arylsulfanyl-, Heteroarylsulfanyl-, C₁₋₄-Alkylsulfinyl-, Arylsulfinyl-, Heteroarylsulfinyl-, C₁₋₄-Alkylsulfonyl-, Arylsulfonyl- und Heteroarylsulfonyl-; und
L2 unabhängig voneinander ausgewählt aus der Gruppe bestehend aus Fluor, Chlor, Brom, Iod, C₁₋₃-Alkyl, Difluormethyl, Trifluormethyl, C₁₋₃-Alkoxy, Difluormethoxy, Trifluormethoxy und Cyan; und
R⁶, R^{7a}, R^{7b}, R^{7c} unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl besitzen,
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl-, Isochinolinyl- oder Tetrazolylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L2 substituiert sein können;
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

2. Glucopyranosyl-substituierte Benzol-Derivate der allgemeinen Formel I.2c 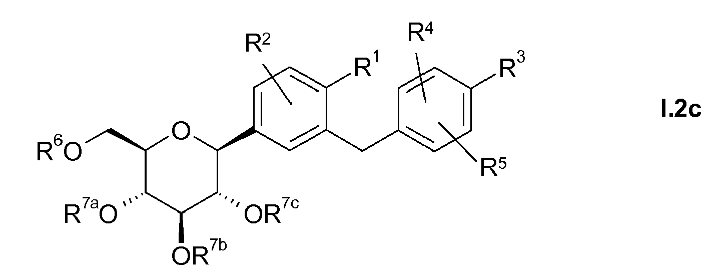 worin die Reste R¹ bis R⁶ und R^{7a}, R^{7b} und R^{7c} wie in Anspruch 1 definiert sind.

3. Glucopyranosyl-substituierte Benzol-Derivate gemäß Anspruch 1 oder 2, **dadurch gekennzeichnet, dass** die Gruppe R¹ ausgewählt ist aus den Bedeutungen Methyl und Chlor.

4. Glucopyranosyl-substituierte Benzol-Derivate gemäß einem oder mehreren der Ansprüche 1 bis 3, **dadurch gekennzeichnet, dass** die Gruppe R³ ausgewählt ist aus den Bedeutungen Ethinyl, Trimethylsilylethyl, Cyclobutyloxy, Cyclopentyloxy, Cyclohexyloxy, Tetrahydrofuran-3-yloxy und Tetrahydropyran-4-yloxy.

5. Glucopyranosyl-substituierte Benzol-Derivate gemäß Anspruch 1 oder 2, **dadurch gekennzeichnet, dass** die Gruppe R¹ ausgewählt ist aus den Bedeutungen C₂₋₆-Alkin-1-yl, C₂₋₆-Alken-1-yl, C₃₋₇-Cycloalkyl, C₃₋₇-Cycloalkyloxy und Cyan, wobei in C₅₋₆-Cycloalkyl-Gruppen eine Methyleneinheit durch O ersetzt sein kann.

6. Glucopyranosyl-substituierte Benzol-Derivate gemäß einem oder mehreren der Ansprüche 1, 2 oder 5, **dadurch gekennzeichnet, dass** der Rest R³ ausgewählt ist aus der Gruppe bestehend aus Wasserstoff, Fluor, Chlor, Methyl, Ethyl, Isopropyl, tert.-Butyl, Ethinyl, 1-Propinyl, Trimethylsilylethyl, Difluormethyl, Trifluormethyl, Cyclopropyl, Cyclobutyl, Cyclopentyl, Methoxy, Ethoxy, Isopropoxy, Cyclopentyloxy, Difluormethoxy, Trifluormethoxy, Pentafluorethoxy, Tetrahydrofuran-3-yloxy, Tetrahydrofuran-2-on-3-yloxy, Methylsulfanyl, Ethylsulfanyl, Isopropylsulfanyl und Cyclopropylidenmethyl.

7. Glucopyranosyl-substituierte Benzol-Derivate gemäß einem oder mehreren der Ansprüche 1 bis 6, **dadurch gekennzeichnet, dass** der Rest R² Wasserstoff oder Methyl bedeutet.

8. Glucopyranosyl-substituierte Benzol-Derivate gemäß einem oder mehreren der Ansprüche 1 bis 7, **dadurch gekennzeichnet, dass** die Reste R⁴ und R⁵ Wasserstoff bedeuten.

9. Glucopyranosyl-substituierte Benzol-Derivate gemäß einem oder mehreren der Ansprüche 1 bis 8, **dadurch gekennzeichnet, dass** der Rest R⁶ und die Reste R^{7a}, R^{7b}, R^{7c} Wasserstoff bedeuten.

10. Glucopyranosyl-substituierte Benzol-Derivate gemäß einem oder mehreren der Ansprüche 1 bis 9 ausgewählt aus der Gruppe der Verbindungen (1) bis (17):
(1) 1-Chlor-2-(4-cyclopentyloxybenzyl)-4-(β-D-glucopyranos-1-yl)-benzol
(2) 1-Chlor-4-(β-D-glucopyranos-1-yl)-2-[4-((*R*)-tetrahydrofuran-3-yloxy)-benzyl]-benzol
(3) 1-Chlor-4-(β-D-glucopyranos-1-yl)-2-[4-((*S*)-tetrahydrofuran-3-yloxy)-benzyl]-benzol
(4) 1-Chlor-4-(β-D-glucopyranos-1-yl)-2-[4-(tetrahydrofuran-2-on-3-yloxy)-benzyl]-benzol
(5) 1-Chlor-4-(β-D-glucopyranos-1-yl)-2-(4-cyclobutyloxy-benzyl)-benzol
(6) 1-Chlor-4-(β-D-glucopyranos-1-yl)-2-(4-cyclohexyloxy-benzyl)-benzol
(7) 1-Chlor-4-(β-D-glucopyranos-1-yl)-2-[4-(tetrahydropyran-4-yloxy)-benzyl]-benzol
(8) 1-Chlor-4-(β-D-glucopyranos-1-yl)-2-[4-(1-acetyl-piperidin-4-yloxy)-benzyl]-benzol
(10) 1-(β-D-Glucopyranos-1-yl)-4-methyl-3-[4-(tetrahydrofuran-3-yloxy)-benzyl]-benzol
(11) 1-(β-D-Glucopyranos-1-yl)-4-methyl-3-[4-(2-trimethylsilyl-ethyl)-benzyl]-benzol
(12) 1-Chlor-4-(β-D-glucopyranos-1-yl)-2-(4-ethinyl-benzyl)-benzol
(13) 1-Chlor-4-(β-D-glucopyranos-1-yl)-2-[4-(piperidin-4-yloxy)-benzyl]-benzol
(14) 1-Fluor-4-(β-D-glucopyranos-1-yl)-2-(4-ethinyl-benzyl)-benzol
(15) 1-(β-D-Glucopyranos-1-yl)-3-(4-ethinyl-benzyl)-benzol
(16) 1-Ethinyl-4-(β-D-glucopyranos-1-yl)-2-(4-ethoxy-benzyl)-benzol
(17) 1-Methoxy-4-(β-D-glucopyranos-1-yl)-2-(4-ethinyl-benzyl)-benzol

11. Physiologisch verträgliche Salze der Verbindungen nach mindestens einem der Ansprüche 1 bis 10 mit anorganischen oder organischen Säuren.

12. Arzneimittel, enthaltend eine Verbindung nach mindestens einem der Ansprüche 1 bis 10 oder ein physiologisch verträgliches Salz gemäß Anspruch 11 neben gegebenenfalls einem oder mehreren inerten Trägerstoffen und/oder Verdünnungsmitteln.

13. Verwendung mindestens einer Verbindung nach einem oder mehreren der Ansprüche 1 bis 10 oder eines physiologisch verträglichen Salzes gemäß Anspruch 11 zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind.

14. Verfahren zur Herstellung der Verbindungen der allgemeinen Formel I gemäß den Ansprüchen 1 bis 10, **dadurch gekennzeichnet, dass** eine Verbindung der allgemeinen Formel II 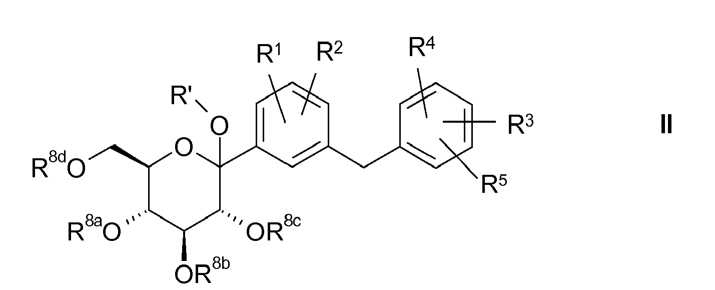 in der
R' H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
R^{8a}, R^{8b}, R^{8c}, R^{8d} unabhängig voneinander eine für die Reste R⁶, R^{7a}, R^{7b}, R^{7c} angegebenen Bedeutungen besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal-oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵, R⁶, R^{7a}, R^{7b}, R^{7c} die in den Ansprüchen 1 bis 10 angegebenen Bedeutungen besitzen,
mit einem Reduktionsmittel in Gegenwart einer Lewis- oder Brønsted-Säure umgesetzt wird, wobei die eventuell vorhandenen Schutzgruppen gleichzeitig oder nachträglich abgespalten werden;
gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
gewünschtenfalls eine so erhaltene Verbindung der allgemeinen Formel I in ihre physiologisch verträglichen Salze überführt wird.

15. Verfahren gemäß Anspruch 14, **dadurch gekennzeichnet, dass** die Verbindung der allgemeinen Formel II Gemäß dem in Anspruch 16 oder 17 beschriebenen Verfahren erhalten wird.

16. Verfahren zur Herstellung von Verbindungen der allgemeinen Formel II 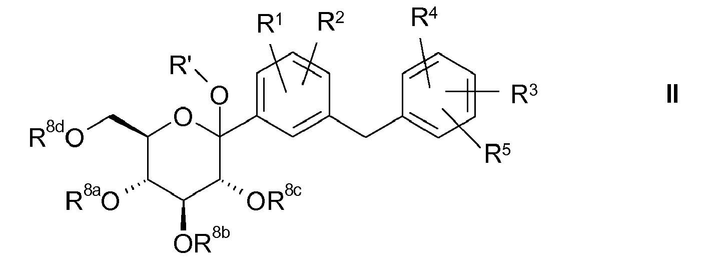 in der
R' H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
R^{8a}, R^{8b}, R^{8c}, R^{8d} unabhängig voneinander eine für die Reste R⁶, R^{7a}, R^{7b}, R^{7c} angegebenen Bedeutungen besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal-oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵, R⁶, R^{7a}, R^{7b}, R^{7c} die in den Ansprüchen 1 bis 10 angegebenen Bedeutungen besitzen,
bei dem eine Organometall-Verbindung (V), die durch Halogen-Metall-Austausch oder durch Insertion eines Metalls in die Kohlenstoff-Halogen-Bindung einer Halogen-Benzylbenzol-Verbindung der allgemeinen Formel IV 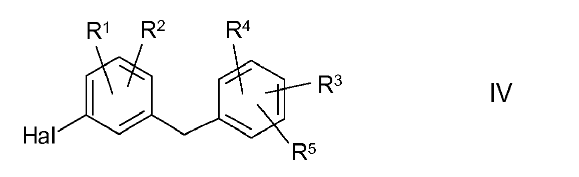 in der Hal Cl, Br und I bedeutet und R¹ bis R⁵ wie zuvor definiert sind, und gegebenenfalls anschließender Ummetallierung erhältlich ist, an ein Gluconolacton der allgemeinen Formel VI 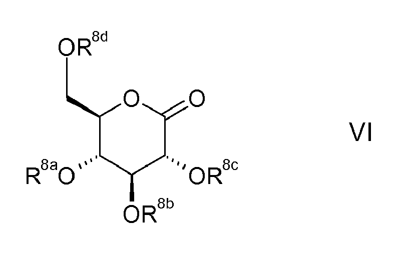 in der R^{8a}, R^{8b}, R^{8c}, R^{8d} wie zuvor definiert sind,
addiert wird, und
anschließend das erhaltene Addukt mit Wasser oder einem Alkohol R'-OH, wobei R' gegebenenfalls substituiertes C₁₋₄-Alkyl bedeutet, in Gegenwart einer Säure umgesetzt wird und optional das in der Umsetzung mit Wasser erhaltene Produkt, in dem R' H bedeutet, in einer nachfolgenden Reaktion mit einem Acylierungsmittel in das Produkt der Formel II überführt wird, worin R' (C₁₋₁₈-Alkyl)carbonyl, (C₁₋₁₈-Alkyl)oxycarbonyl, Arylcarbonyl oder Aryl-(C₁₋₃-alkyl)-carbonyl, das wie angegeben substituiert sein kann, bedeutet.

17. Verfahren gemäß Anspruch 16, **dadurch gekennzeichnet, dass** die Organometall-Verbindung (V) eine Lithium- oder Magnesium-Verbindung ist.

18. Verfahren zur Herstellung der Verbindungen der allgemeinen Formel I gemäß den Ansprüchen 1 bis 10, in der R⁶, R^{7a}, R^{7b} und R^{7c} Wasserstoff bedeuten, **dadurch gekennzeichnet, dass** eine Verbindung der allgemeinen Formel III 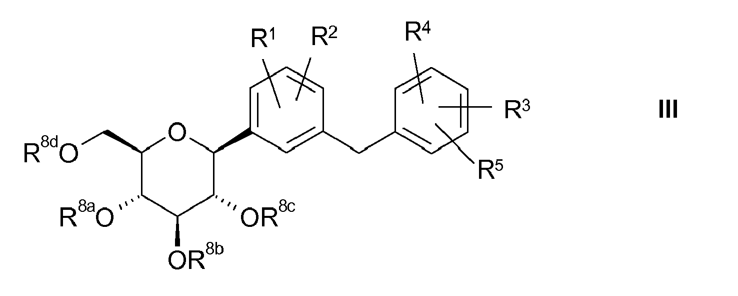 in der
R^{8a}, R^{8b}, R^{8c}, R^{8d} unabhängig voneinander eine für die Reste R⁶, R^{7a}, R^{7b}, R^{7c} angegebenen Bedeutungen, jedoch mindestens einer der Reste R^{8a}, R^{8b}, R^{8c}, R^{8d} nicht Wasserstoff bedeutet, besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal- oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein-oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵, R⁶, R^{7a}, R^{7b}, R^{7c} die in den Ansprüchen 1 bis 10 angegebenen Bedeutungen besitzen,
hydrolysiert wird, und
gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, überführt wird.

19. Verfahren gemäß Anspruch 18, **dadurch gekennzeichnet, dass** die Verbindung der Formel III mittels eines Verfahrens gemäß Anspruch 14 oder 15 erhalten wird.

20. Verbindung der allgemeinen Formel IV 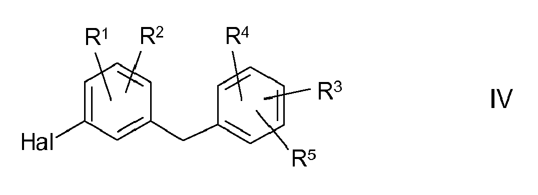 in der Hal Chlor, Brom oder Iod bedeutet und die Reste R¹, R², R³, R⁴ und R⁵ wie in einem oder mehreren der Ansprüche 1 und 3 bis 8 definiert sind.

21. Verbindung der Formel IV gemäß Anspruch 20 **gekennzeichnet durch** die Formel 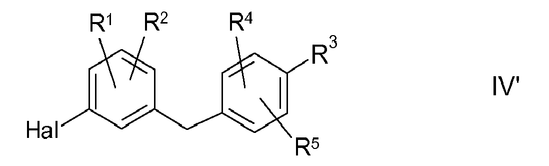 in der Hal Chlor, Brom oder Iod bedeutet und die Reste R¹, R², R⁴ und R⁵ wie in einem oder mehreren der Ansprüche 1, 3, 5, 7 und 8 definiert sind und der Rest R³ aus der Gruppe B gemäß Anspruch 1 oder aus den Bedeutungen gemäß Anspruch 4 ausgewählt ist.

22. Verbindung der allgemeinen Formel II 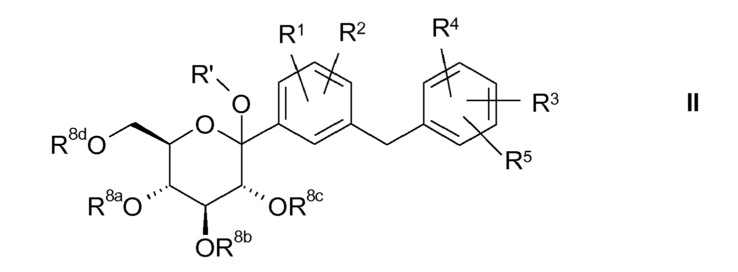 in der
R' H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
R^{8a}, R^{8b}, R^{8c}, R^{8d} unabhängig voneinander eine für die Reste R⁶, R^{7a}, R^{7b}, R^{7c} angegebenen Bedeutungen besitzt, eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal-oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵ wie in einem oder mehreren der Ansprüche 1 und 3 bis 8 definiert sind.

23. Eine Verbindung ausgewählt aus der Gruppe bestehend aus
4-Brom-1-iod-2-(4-ethoxy-benzyl)-benzol,
4-Brom-1-chlor-2-(4-iod-benzyl)-benzol,
4-(5-Brom-2-chlor-benzyl)-phenol,
1-Chlor-4-(β-D-glucopyranos-1-yl)-2-(4-hydroxybenzyl)-benzol,
1-Chlor-4-(β-D-glucopyranos-1-yl)-2-[4-(trifluormethylsulphonyloxy)-benzyl]-benzol.

24. Darreichungsform **dadurch gekennzeichnet, daß** in der Darreichungsform eine Verbindung gemäß einem oder mehreren der Ansprüche 1 bis 10 und ein weiterer Wirkstoff vorliegen.

25. Darreichungsform gemäß Anspruch 24 **dadurch gekennzeichnet, daß** der weitere Wirkstoff ausgewählt ist aus der Gruppe der Antidiabetika oder aus der Gruppe der Medikamente zur Beeinflussung des Bluthochdrucks, des chronischen Herzversagens oder der Atherosklerose.

26. Verwendung einer Verbindung gemäß einem oder mehreren der Ansprüche 1 bis 10 oder eines physiologisch verträglichen Salzes solch einer Verbindung in Kombination mit mindestens einem weiteren Wirkstoff zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind.

27. Verwendung gemäß Anspruch 26 **dadurch gekennzeichnet, daß** der weitere Wirkstoff ausgewählt ist aus der Gruppe der Antidiabetika oder aus der Gruppe der Medikamente zur Beeinflussung des Bluthochdrucks, des chronischen Herzversagens oder der Atherosklerose.
